# EUROPEAN PATENT SPECIFICATION

(11) **EP 0 767 786 B1**
(45) Date of publication and mention of the grant of the patent: **16.09.1998**
(21) Application number: 95923666.2
(22) Date of filing: 07.06.1995
(51) Int. Cl.: C07D 305/14, C07D 409/12, C07D 407/12, A61K 31/335

(54) **7-ETHER-TAXOL ANALOGS, ANTINEOPLASTIC USE AND PHARMACEUTICAL COMPOSITIONS CONTAINING THEM**
7-ÄTHER-TAXOL ÄHNLICHE VERBINDUNGEN ANTINEOPLASTISCHE VERWENDUNG UND DIESE ENTHALTENDE PHARMAZEUTISCHE ZUSAMMENSTELLUNGEN
ANALOGUES DE 7-ETHER-TAXOL, UTILISATION ANTINEOPLASIQUE ET COMPOSITIONS PHARMACEUTIQUES LES CONTENANT

(30) Priority: 28.06.1994 US 268179
(43) Date of publication of application: 16.04.1997
(73) Proprietor: PHARMACIA & UPJOHN COMPANY, Kalamazoo, Michigan 49001 (US)
(72) Inventor: KELLY, Robert, C., Augusta, MI 49012 (US); GEBHARD, Ilse, Kalamazoo, MI 49009 (US)
(74) Representative: Perry, Robert Edward
(86) International application number: US9506595
(87) International publication number: WO9600724

(56) References cited:
- WO-A-95/20582
- US-A- 4 960 790

## Description

### BACKGROUND OF THE INVENTION

Taxol is a member of the taxane family of diterpenes, having the structure shown below: 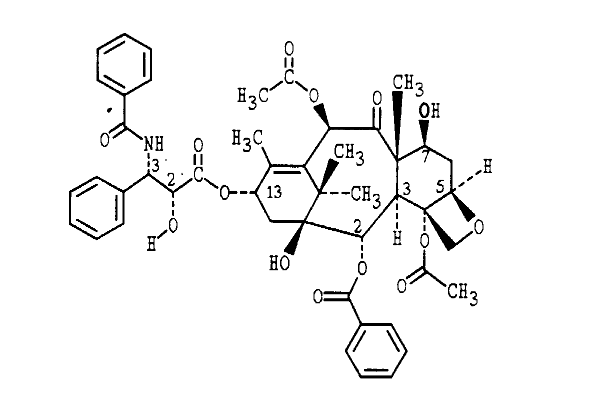 The numbering system shown for taxol is that recommended by IUPAC (IUPAC, Commission on the Nomenclature of Organic Chemistry, 1978).

The chemistry of the potent anticancer diterpenoid taxol and analogs thereof is reviewed, with an emphasis on isolation and analysis, structural modifications, partial synthesis, and structure-activity relationships by David G.I. Kingston, The Chemistry of Taxol, Pharmac. Ther., Vol 52, pp 1-34, 1991.

The clinical pharmacology of taxol is reviewed by Eric K Rowinsky and Ross C. Donehower, The Clinical Pharmacology and Use of Antimicrotubule Agents in Cancer Chemotherapeutics, Pharmac. Ther., Vol 52, pp 35-84, 1991. Clinical and preclinical studies with taxol are reviewed by William J. Slichenmyer and Daniel D. Von Hoff, Taxol: A New and Effective Anti-cancer Drug, Anti-Cancer Drugs, Vol. 2, pp 519-530, 1991.

Taxol and analogs thereof are the subject of various patents including, for example, U.S. Patent Nos. 4,814,470; 4,857,653; 4,942,184; 4,924,011; 4,924,012; 4,960,790; 5,015,744; 5,157,049; 5,059,699; 5,136,060; 4,876,399; 5,227,400; 5,248,796 as well as PCT Publication No. WO 92/09589, European Patent Application 90305845.1 (Publication No. A2 0 400 971), 90312366.9 (Publication No. A1 0 428 376), 89400935.6 (Publication No. A1 0 366 841) and 90402333.0 (Publication No. 0 414 610 A1), 87401669.4 (A1 0 253 739), 92308608.6 (A1 0 534 708), 92308609.4 (A1 534 709) and PCT Publication Nos. WO 91/17977, WO 91/17976, WO 91/13066, WO 91/13053.

Various processes for the preparation of taxol (and intermediates and analogs thereon are described in Tetrahedron Letters, 1992, 33 5185; J. Org. Chem., 1991, 56 1681 and J. Org. Chem., 1991, 56 5114.

Chen et al., Serendipitous Synthesis of a Cyclopropane-Containing Taxol Analog via Anchimeric Participation of an Unactivated Angular Methyl Group, Advance ACS Abstracts, Vol 1, No. 2., July 15, 1993 reported the treatment of a 7-*epi* taxol derivative with DAST in dichloromethane led to an unexpected reaction involving participation of the C-19 methyl group and clean formation of a cyclopropane ring. See also J. Org. Chem., 1993, 58, 4520 (August 13, 1993) and U.S. Patent 5,254,580 (granted 19 October 1993).

U.S. Patent 5,248,796 (granted 28 September 1993) relates to 10-desacetoxy-11,12-dihydrotaxol-10,12(18)-diene derivatives and the preparation of 10-desacetoxytaxol.

EP Application 0 558 959 Al discloses various phosphonooxy and carbonate 2' taxol derivatives of taxol with increased water solubility.

Water-soluble pro-taxol analogs are disclosed in Nicolaou, KC.; Riemer, C.; Kerr, M.A.; Rideout, D.; Wrasidlo, W., Nature 364:464-66 (1993).

J. Am. Chem. Soc., Vol. 116, No. 4, 1599-1600 (1994) decribes the production of 7-BOM baccatin III. The 7-BOM baccatin III was treated with lithium hexamethyl disilazide and the resulting alkoxide reacted with (3R,4S)-N-benzoyl-3-O-TES-4-phenyl-2-azetedinone to give 7-BOM-2'-TES-taxol. This was reacted with HF-pyridine to give 7-BOM-taxol.

At the 207 Annual Meeting of the American Chemical Society, L. Klein described the "surprisingly good activity" of 7-ether analogs of 9-OH-taxotere, in particular the 7-OMe and the 7-allyl analogs. No method of synthesis was described.

U.S. Patent 5,229,526 (Holton) describes the use of 7-O-protecting groups (namely T¹, including triethylsilyl and ethoxyethyl) in the the preparation of various biologically active derivatives of baccatin III and 10-deacetyl baccatin III wherein the C-7 and C-2' hydroxyl protecting groups are hydrolyzed under mild conditions so as not to disturb the ester linkage or the taxane substituents.

### SUMMARY OF THE INVENTION

This invention provides taxol analogs of Formula I:

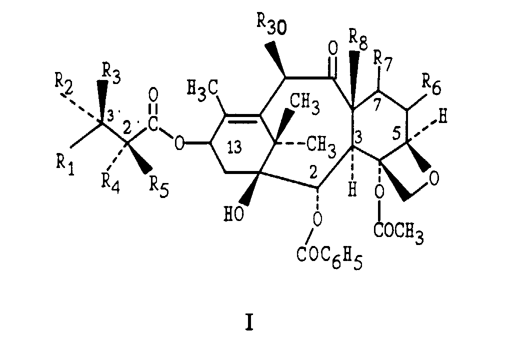

The compounds of Formula I are useful for the treatment of the same cancers for which taxol has been shown active, including human ovarian cancer, breast cancer, and malignant melanoma as well as lung cancer, gastric cancer, colon cancer, head and neck cancer, and leukemia.

### CONVENTIONS FOR FORMULAS AND DEFINITIONS OF VARIABLES

The chemical formulas representing various compounds or molecular fragments in the specification and claims may contain variable substituents in addition to expressly defined structural features. These variable substituents are identified by a letter or a letter followed by a numerical subscript, for example, "Z₁" or "Rᵢ" where "i" is an integer. These variable substituents are either monovalent or bivalent, that is, they represent a group attached to the formula by one or two chemical bonds. For example, a group Z₁ would represent a bivalent variable if attached to the formula CH₃-C(=Z₁)H. Groups Rᵢ and Rⱼ would represent monovalent variable substituents if attached to the formula CH₃-CH₂-C(Rᵢ)(Rⱼ)-H. When chemical formulas are drawn in a linear fashion, such as those above, variable substituents contained in parentheses are bonded to the atom immediately to the left of the variable substituent enclosed in parenthesis. When two or more consecutive variable substituents are enclosed in parentheses, each of the consecutive variable substituents is bonded to the immediately preceding atom to the left which is not enclosed in parentheses. Thus, in the formula above, both Rᵢ and Rⱼ are bonded to the preceding carbon atom. Also, for any molecule with an established system of carbon atom numbering, such as taxol, these carbon atoms are designated as Cᵢ, where "i" is the integer corresponding to the carbon atom number. For example, C₆ represents the 6 position or carbon atom number in the nucleus as traditionally designated by those skilled in the art.

Chemical formulas or portions thereof drawn in a linear fashion represent atoms in a linear chain. The symbol "-" in general represents a bond between two atoms in the chain. Thus CH₃-O-CH₂-CH(Rᵢ)-CH₃ represents a 2-substituted-1-methoxypropane compound. In a similar fashion, the symbol "=" represents a double bond, e.g., CH₂=C(Rᵢ)-O-CH₃, and the symbol "≡" represents a triple bond, e.g., HC≡C-CH(Rᵢ)-CH₂-CH₃. Carbonyl groups are represented in either one of two ways: -CO- or -C(=O)-, with the former being preferred for simplicity.

Chemical formulas of cyclic (ring) compounds or molecular fragments can be represented in a linear fashion. Thus, the compound 4-chloro-2-methylpyridine can be represented in linear fashion by N*=C(CH₃)-CH=CCl-CH=C*H with the convention that the atoms marked with an asterisk (*) are bonded to each other resulting in the formation of a ring. Likewise, the cyclic molecular fragment, 4-(ethyl)-1-piperazinyl can be represented by -N*-(CH₂)₂-N(C₂H₅-CH₂-C*H₂. Similarly, 2-furyl can be represented by -C*-O-CH=CH-C*H= and 2-thienyl represented by -C*-S-CH=CH-C*H=.

A rigid cyclic (ring) structure for any compounds herein defines an orientation with respect to the plane of the ring for substituents attached to each carbon atom of the rigid cyclic compound. For saturated compounds which have two substituents attached to a carbon atom which is part of a cyclic system, -C(X₁)(X₂)- the two substituents may be in either an axial or equatorial position relative to the ring and may change between axial/equatorial. However, the position of the two substituents relative to the ring and each other remains fixed. While either substituent at times may lie in the plane of the ring (equatorial) rather than above or below the plane (axial), one substituent is always above the other. In chemical structural formulas depicting such compounds, a substituent (X₁) which is "below" another substituent (X₂) will be identified as being in the alpha (α) configuration and is identified by a broken, dashed or dotted line attachment to the carbon atom, i.e., by the symbol "- - -" or "...". The corresponding substituent attached "above" (X₂) the other (X₁) is identified as being in the beta (β) configuration and is indicated by an unbroken line attachment to the carbon atom.

When a variable substituent is bivalent, the valences may be taken together or separately or both in the definition of the variable. For example, a variable Rᵢ attached to a carbon atom as -C(=Rᵢ)- might be bivalent and be defined as oxo or keto (thus forming a carbonyl group (-CO-) or as two separately attached monovalent variable substituents α-Rᵢ₋ⱼ and β-Rᵢ₋ₖ. When a bivalent variable, Rᵢ, is defined to consist of two monovalent variable substituents, the convention used to define the bivalent variable is of the form "α-Rᵢ₋ⱼ:β-Rᵢ₋ₖ" or some variant thereof. In such a case both α-Rᵢ₋ⱼ and β-Rᵢ₋ₖ are attached to the carbon atom to give -C(α-Rᵢ₋ⱼ)(β-Rᵢ₋ₖ). For example, when the bivalent variable R₆, -C(=R₆)- is defined to consist of two monovalent variable substituents, the two monovalent variable substituents are α-R₆₋₁:β-R₆₋₂, .... α-R₆₋₉:β-R₆₋₁₀, etc, giving -C(α-R₆₋₁)(β-R₆₋₂), ... -C(α-R₆₋₉)(β-R₆₋₁₀)-, etc. Likewise, for the bivalent variable R₁₁, -C(=R₁₁)-, two monovalent variable substituents are α-R₁₁₋₁:β-R₁₁₋₂. For a ring substituent for which separate α and β orientations do not exist (e.g. due to the presence of a carbon double bond in the ring), and for a substituent bonded to a carbon atom which is not part of a ring the above convention is still used, but the α and β designations are omitted.

Just as a bivalent variable may be defined as two separate monovalent variable substituents, two separate monovalent variable substituents may be defined to be taken together to form a bivalent variable. For example, in the formula -C₁(Rᵢ)H-C₂(Rⱼ)H- (C₁ and C₂ define arbitrarily a first and second carbon atom, respectively) Rᵢ and Rⱼ may be defined to be taken together to form (1) a second bond between C₁ and C₂ or (2) a bivalent group such as oxa (-O-) and the formula thereby describes an epoxide. When Rᵢ and Rⱼ are taken together to form a more complex entity, such as the group -X-Y-, then the orientation of the entity is such that C₁ in the above formula is bonded to X and C₂ is bonded to Y. Thus, by convention the designation "... Rⱼ and Rⱼ are taken together to form -CH₂-CH₂-O-CO- ..." means a lactone in which the carbonyl is bonded to C₂. However, when designated"... Rⱼ and Rᵢ are taken together to form -CO-O-CH₂-CH₂-the convention means a lactone in which the carbonyl is bonded to C₁.

The carbon atom content of variable substituents is indicated in one of two ways. The first method uses a prefix to the entire name of the variable such as "C₁-C₄", where both "1" and "4" are integers representing the minimum and maximum number of carbon atoms in the variable. The prefix is separated from the variable by a space. For example, "C₁-C₄ alkyl" represents alkyl of 1 through 4 carbon atoms, (including isomeric forms thereof unless an express indication to the contrary is given). Whenever this single prefix is given, the prefix indicates the entire carbon atom content of the variable being defined. Thus C₂-C₄ alkoxycarbonyl describes a group CH₃-(CH₂)ₙ-O-CO- where n is zero, one or two. By the second method the carbon atom content of only each portion of the definition is indicated separately by enclosing the "Cᵢ-Cⱼ" designation in parentheses and placing it immediately (no intervening space) before the portion of the definition being defined. By this optional convention (C₁-C₃)alkoxycarbonyl has the same meaning as C₂-C₄ alkoxycarbonyl because the "C₁-C₃" refers only to the carbon atom content of the alkoxy group. Similarly while both C₂-C₆ alkoxyalkyl and (C₁-C₃)alkoxy(C₁-C₃)alkyl define alkoxyalkyl groups containing from 2 to 6 carbon atoms, the two definitions differ since the former definition allows either the alkoxy or alkyl portion alone to contain 4 or 5 carbon atoms while the latter definition limits either of these groups to 3 carbon atoms.

When the claims contain a fairly complex (cyclic) substituent, at the end of the phrase naming/designating that particular substituent will be a notation in (parentheses) which will correspond to the same name/designation in one of the CHARTS/FIGURES which will also set forth the chemical structural formula of that particular substituent.

The term "Boc" refers to C(O)O-t-butyl, "Troc" refers to C(O))CH₂CCl₃, TES refers to Si(Et)₃, Ph refers to phenyl, Ac refers to C(O)CH₃, Bz refers to C(O)Ph, and Cbz refers to C(O)OCH₂C₆H₅.

### DETAILED DESCRIPTION OF THE INVENTION

More specifically, this invention provides 7-ether-taxol analogs of general Formula I 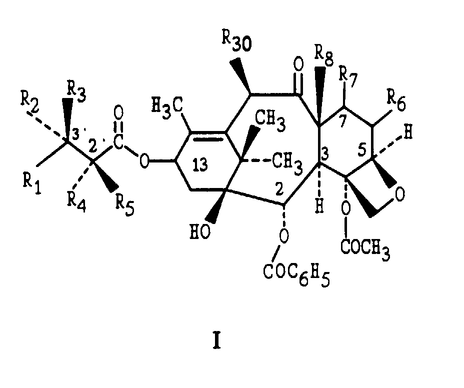 wherein:
R₁ is selected from the group consisting of
   -CH₃,
   -C₆H₅ or phenyl substituted with one, 2 or 3 C₁-C₄ alkyl, C₁-C₃ alkoxy, halo, C₁-C₃ alkylthio, trifluoromethyl, C₂-C₆ dialkylamino,
   hydroxy or nitro,
   -2-furyl, 2-thienyl, 1-naphthyl, 2-naphthyl or
   3,4-methylenedioxyphenyl;
R₂ is selected from the group consisting of -H, -NHC(O)H,-NHC(O)C₁-C₁₀alkyl (preferably -NHC(O)C₄-C₆alkyl), -NHC(O)phenyl, -NHC(O)phenyl substituted with one, 2 or 3 C₁-C₄ alkyl, C₁-C₃ alkoxy, halo, C₁-C₃ alkylthio, trifluoromethyl, C₂-C₆ dialkylamino, hydroxy or nitro, -NHC(O)C(CH₃)=CHCH₃, -NHC(O)OC(CH₃)₃, -NHC(O)OCH₂phenyl, -NH₂, -NHSO₂-4-methylphenyl,-NHC(O)(CH₂)₃COOH, -NHC(O)-4-(SO₃H)phenyl, -OH, -NHC(O)-1-adamantyl, -NHC(O)O-3-tetrahydrofuranyl, -NHC(O)O-4-tetrahydropyranyl, -NHC(O)CH₂C(CH₃)₃, -NHC(O)C(CH₃)₃, -NHC(O)OC₁-C₁₀alkyl, -NHC(O)NHC₁-C₁₀alkyl, -NHC(O)NHPh, -NHC(O)NHPh substituted with one, 2 or 3 C₁-C₄ alkyl, C₁-C₃ alkoxy, halo, C₁-C₃ alkylthio, trifluoromethyl, C₂-C₆ dialkylamino, or nitro, -NHC(O)C₃-C₈cycloalkyl, -NHC(O)OC(CH₂CH₃)₂CH₃, -NHC(O)OC(CH₃)₂CH₂Cl, -NHC(O)OC(CH₃)₂CH₂CH₃, -NHC(O)-1-phenyl-1-cyclopentyl, -NHC(O)-1-methyl-1-cyclohexyl, -NHC(S)NHC(CH₃)₃ or -NHC(O)NHC(CH₃)₃;
R₃ is selected from the group consisting of -H, -NHC(O)phenyl or -NHC(O)OC(CH₃)₃, with the overall proviso that one of R₂ and R₃ is -H but R₂ and R₃ are not both -H;
R₄ is -H or selected from the group consisting of -OH, -OAc (-OC(O)CH₃), -OC(O)OCH₂C(Cl)₃, -OCOCH₂CH₂NH₃⁺ HCOO⁻, -NHC(O)phenyl, -NHC(O)OC(CH₃)₃, -OCOCH₂CH₂COOH and pharmaceutically acceptable salts thereof, -OCO(CH₂)₃COOH and pharmaceutically acceptable salts thereof, and -OC(O)-Z-C(O)R' [where Z is ethylene (-CH₂CH₂-), propylene (-CH₂CH₂CH₂-), -CH=CH-, 1,2-cyclohexane or 1,2-phenylene, R' is -OH, -OH base, -NR'₂R'₃, -OR'₃, -SR'₃, -OCH₂C(O)NR'₄R'₅ where R'₂ is -H or -CH₃, R'₃ is -(CH₂)ₙNR'₆R'₇ or (CH₂)ₙN+R'₆R'₇R'₈ X⁻ where n is 1-3, R'₄ is -H or -C₁-C₄alkyl, R'₅ is -H, -C₁-C₄alkyl, benzyl, hydroxyethyl, -CH₂CO₂H or dimethylaminoethyl, R'₆ and R'₇ are -CH₃, -CH₂CH₃, benzyl or R'₆ and R'₇ together with the nitrogen of NR'₆R'₇ form a pyrrolidino, piperidino, morpholino, or N-methylpiperizino group; R'₈ is -CH₃, -CH₂CH₃ or benzyl, X⁻ is halide, and base is NH₃, (HOC₂H₄)₃N, N(CH₃)_{3,} CH₃N(C₂H₄)₂NH, NH₂(CH₂)₆NH₂, N-methylglucamine, NaOH or KOH], -OC(O)(CH₂)ₙNR²R³ [where n is 1-3, R² is -H or -C₁-C₃alkyl and R³ -H or -C₁-C₃alkyl], -OC(O)CH(R")NH₂ [where R" is selected from the group consisting of -H, -CH₃, -CH₂CH(CH₃)₂, -CH(CH₃)CH₂CH₃, -CH(CH₃)₂, -CH₂phenyl, -(CH₂)₄NH₂, -CH₂CH₂COOH, -(CH₂)₃NHC(=NH)NH₂], the residue of the amino acid proline, -OC(O)CH=CH₂, -C(O)CH₂CH₂C(O)NHCH₂CH₂SO₃⁻ Y⁺, -OC(O)CH₂CH₂C(O)NHCH₂CH₂CH₂SO₃⁻Y⁺ wherein Y⁺ is Na⁺ or N⁺(Bu)₄, -OC(O)CH₂CH₂C(O)OCH₂ CH₂OH;
R₅ is -H or -OH, with the overall proviso that when R₅ is -OH, R₄ is -H and with the further proviso that when R₅ is -H, R₄ is other than -H;
R₆ is -H:-H;
R₇ is α-R₉₁:β-R₉₂ where one of R₉₁ and R₉₂ is -H and the other of R₉₁ and R₉₂ is -W where W is selected from the group consisting of
   -O-C₁-C₁₀alkyl,
   -O-C₃-C₁₀ unsaturated alkyl (preferably allyl and crotyl),
   -O-C₅-C₁₅ heteroalkyl [e.g. -OCH₂(2- or 3-furyl), -OCH₂(2- or 3-pyrrolyl), -OCH₂(2-, 3- or 4-pyridyl), -OCH₂(2-, 3-, 4-, 5-, 6, 7- or 8-quinolinyl), -OCH₂(1-, 3-, 4-, 5-, 6, 7- or 8-isoquinolinyl), -OCH₂(2-, 4- or 5-imidazoyl), -OCH₂(3-, 4- or 5-pyrazolyl), -OCH₂(2-pyrazinyl), -OCH₂(2-, 4-, 5- or 6-pyrimidinyl), -OCH₂(2-, 3-, 4-, 5-, 6- or 7-indolyl), -OCH₂(3-, 4- or 5-isoxazolyl); preferably -OCH₂(2- or 3-furyl), -OCH₂(2- or 3-pyrrolyl), -OCH₂(2-, 3- or 4-pyridyl), -OCH₂(2-, 4- or 5-imidazoyl) or -OCH₂(3-, 4- or 5-isoxazolyl)],
   -O-CH(R²¹)OR²² where
      R²¹ is -H or -C₁-C₆ alkyl, and
      R²² is -C₁₋C₁₀alkyl, -C₃-C₁₀ unsaturated alkyl (preferably allyl and crotyl ), -C₅-C₁₅ heteroalkyl [e.g. CH₂-(2- or 3-furyl), CH₂(2- or 3-pyrrolyl), CH₂(2-, 3, or 4-pyridyl), CH₂(2-, 3-, 4-, 5-, 6, 7- or 8-quinolinyl), CH₂(1-, 3-, 4-, 5-, 6, 7- or 8-isoquinolinyl), CH₂(2-, 4- or 5-imidazoyl), CH₂(3-, 4- or 5-pyrazolyl), CH₂(2-pyrazinyl), CH₂(2-, 4-, 5-or 6-pyrimidinyl), CH₂(2-, 3-, 4-, 5-, 6- or 7-indolyl), CH₂(3-, 4- or 5-isoxazolyl); preferably CH₂-(2- or 3-furyl), CH₂(2- or 3-pyrrolyl), CH₂(2-, 3, or 4-pyridyl), CH₂(2-, 4- or 5-imidazoyl), CH₂(3-, 4- or 5-isoxazolyl); preferably CH₂-(2- or 3-furyl), CH₂(2- or 3-pyrrolyl), CH₂(2-, 3, or 4-pyridyl), CH₂(2-, 4- or 5-imidazoyl) or CH₂(3-, 4- or 5-isoxazolyl)],
         or when R²¹ and R²² are taken together to form a ring with 4 to 6 carbon atoms (preferably a ring with 5 or 6 carbon atoms),
      -CH(R²⁸)S(O)ₘAr
   where Ar is phenyl or phenyl substituted with one, 2 or 3 C₁-C₄ alkyl, C₁-C₃ alkoxy, halo, C₁-C₃ alkylthio, trifluoromethyl, C₂-C₆ dialkylamino, or nitro,
-CH(R²⁸)S(O)ₘCH₂R²⁸ where R²⁸ is
   C₁-C₆ alkyl,
   -C₃-C₁₀ unsaturated alkyl (preferably allyl and crotyl),
   -(CH₂)_{q}phenyl where q is 0-6,
   -(CH₂)_{q}phenyl where q is 0-6 and substituted with one, 2 or 3 C₁-C₄ alkyl, C₁-C₃ alkoxy, halo, C₁-C₃ alkylthio, trifluoromethyl, C₂-C₆ dialkylamino, or nitro,
   -naphthyl,
   -naphthyl substituted with one, 2 or 3 C₁-C₄ alkyl, C₁-C₃ alkoxy, halo,
      C₁-C₃ alkylthio, trifluoromethyl, C₂-C₆ dialkylamino, or nitro, -C₅-C₁₅ heteroalkyl [e.g. -(2- or 3-furyl), (2- or 3-pyrrolyl), (2-, 3, or 4-pyridyl), (2-, 3-, 4-, 5-, 6, 7- or 8-quinolinyl), (1-, 3-, 4-, 5-, 6, 7- or 8-isoquinolinyl), (2-, 4- or 5-imidazoyl), (3-, 4- or 5-pyrazolyl), (2-pyrazinyl), (2-, 4-, 5- or 6-pyrimidinyl), (2-, 3-, 4-, 5-, 6- or 7-indolyl), (3-, 4- or 5-isoxazolyl); preferably -(2- or 3-furyl), (2- or 3-pyrrolyl), (2-, 3, or 4-pyridyl), (2-, 4- or 5-imidazoyl), (3-, 4- or 5-isoxazolyl)],
      or when R²⁸ and R²⁸ are taken together to form a ring with 4 to 6 carbon atoms (preferably a ring with 5 or 6 carbon atoms); m is 0 to 2;
R₈ is -CH₃;
R₃₀ is -H, OH, or -OC(O)CH₃; and
pharmaceutically acceptable salts thereof when the compound contains either an acidic or basic functional group.

An embodiment of the subject invention are compounds of Formula I where position 2 is -OR₄₀ (where R₄₀ is -C(O)phenyl substituted with one, 2 or 3 azido, cyano, methoxy, or halo; preferably -C(O)-3-azidophenyl) rather than -O-C(O)phenyl.

A preferred embodiment of the subject invention is compounds of Formula I where R₁ is phenyl or phenyl substituted with halo, R₂ is -NHC(O)C₆H₅, R₃ and R₅ are -H, R₄ is -OH, and R₃₀ is -OH or -OC(O)CH₃. Another preferred embodiment of the subject invention is compounds of Formula I where R₁ is preferably phenyl or phenyl substituted with halo, R₂ is -NHC(O)OC(CH₃)₃, R₃ and R₅ are -H, R₄ is OH, and R₃₀ is -H or -COCH₃. A preferred embodiment of the subject invention is compounds of Formula I where R₁ is preferably phenyl or phenyl substituted with halo, R₂ is -NHC(O)NHC(CH₃)₃, R₃ and R₅ are -H, R₄ is -OH, and R₃₀ is -OH or -OCOCH₃.

W is preferably selected from the group consisting of:
-O-C₁-C₁₀alkyl (more preferably -O-C₁-C₁₀alkyl);
-O-C₃-C₁₀ unsaturated alkyl (more preferably -O-C₃-C₄ unsaturated alkyl);
-O-CH(R²¹)OR²² where
   R²¹ is H or C₁-C₆ alkyl, and
   R²² is preferably -C₁-C₆alkyl or -C₃-C₁₀ unsaturated alkyl (more preferably-O-C₃-C₄ unsaturated alkyl);
-CH(R²⁸)S(O)ₘAr where Ar is phenyl or phenyl substituted with one, 2 or 3 C₁-C₄ alkyl, C₁-C₃ alkoxy, halo, C₁-C₃ alkylthio,
   trifluoromethyl, C₂-C₆ dialkylamino, or nitro;
-CH(R²⁸)S(O)ₘCH₂R²⁸
   where R²⁸ is
   C₁-C₆ alkyl,
   -C₃-C₁₀ unsaturated alkyl (more preferably -O-C₃-C₄ unsaturated alkyl), or
   -(CH₂)_{q}phenyl where q is 0-3; and
m is 0.

An embodiment of the subject invention are compounds of Formula I where R₂ is -NHC(O)C₆H₅, R₄ is hydroxy, R₃ and R₅ are -H, R₁ is phenyl or substituted phenyl, and -W is selected from the group consisting of:
-O-C₁-C₁₀alkyl (more preferably -O-C₁-C₁₀alkyl);
-O-C₃-C₁₀ unsaturated alkyl (more preferably -O-C₃-C₄ unsaturated alkyl);
-O-CH(R²¹)OR²² where
   R²¹ is H or C₁-C₆ alkyl, and
   R²² is preferably -C₁-C₆alkyl or -C₃-C₁₀ unsaturated alkyl (more preferably -O-C₃-C₄ unsaturated alkyl);
-CH(R²⁸)S(O)ₘAr where Ar is phenyl or phenyl substituted with one, 2 or 3 C₁-C₄ alkyl, C₁-C₃ alkoxy, halo, C₁-C₃ alkylthio, trifluoromethyl, C₂-C₆ dialkylamino, or nitro;
-CH(R²⁸)S(O)ₘCH₂R²⁸
   where R²⁸ is
   C₁-C₆ alkyl,
   -C₃-C₁₀ unsaturated alkyl (more preferably -O-C₃-C₄ unsaturated alkyl), or
   -(CH₂)_{q}Phenyl where q is 0-3; and
m is 0.

Another embodiment of the subject invention are compounds of Formula I where R₂ is -NHC(O)OC(CH₃)₃, R₁ is phenyl or substituted phenyl, R₄ is hydroxy, R₃ and R₅ are -H, and -W is selected from the group consisting of:
-O-C₁-C₁₀alkyl (more preferably -O-C₁-C₁₀alkyl);
-O-C₃-C₁₀ unsaturated alkyl (more preferably -O-C₃-C₄ unsaturated alkyl);
-O-CH(R²¹)OR²² where
   R²¹ is H or C₁-C₆ alkyl, and
   R²² is preferably -C₁-C₆alkyl or -C₃-C₁₀ unsaturated alkyl (more preferably -O-C₃-C₄ unsaturated alkyl);
-CH(R²⁸)S(O)ₘAr where Ar is phenyl or phenyl substituted with one, 2 or 3 C₁-C₄ alkyl, C₁-C₃ alkoxy, halo, C₁-C₃ alkylthio,
trifluoromethyl, C₂-C₆ dialkylamino, or nitro;
-CH(R²⁸)S(O)ₘCH₂R²⁸
   where R²⁸ is
   C₁-C₆ alkyl,
   -C₃-C₁₀ unsaturated alkyl (more preferably -O-C₃-C₄ unsaturated alkyl), or
   -(CH₂)_{q}phenyl where q is 0-3; and
m is 0.

An embodiment of the subject invention are compounds of Formula I where R₁ is selected from the group consisting of -CH₃, -C₆H₅ or phenyl substituted with one, 2 or 3 C₁-C₄ alkyl, C₁-C₃ alkoxy, halo, C₁-C₃ alkylthio, trifluoromethyl, C₂-C₆ dialkylamino, hydroxy or nitro and R₂ is selected from the group consisting of -H, -NHC(O)H,-NHC(O)C₁-C₁₀alkyl (preferably -NHC(O)C₄-C₆alkyl), -NHC(O)phenyl, -NHC(O)phenyl substituted with one, 2 or 3 C₁-C₄ alkyl, C₁-C₃ alkoxy, halo, C₁-C₃ alkylthio, trifluoromethyl, C₂-C₆ dialkylamino, hydroxy or nitro, -NHC(O)C(CH₃)=CHCH₃, -NHC(O)OC(CH₃)₃, -NHC(O)OCH₂phenyl, -NH₂, -NHSO₂-4-methylphenyl, -NHC(O)(CH₂)₃COOH, -NHC(O)-4-(SO₃H)phenyl, -OH, -NHC(O)-1-adamantyl, -NHC(O)O-3-tetrahydrofuranyl, -NHC(O)O-4-tetrahydro-pyranyl, -NHC(O)CH₂C(CH₃)₃, -NHC(O)C(CH₃)₃, -NHC(O)OC₁-C₁₀alkyl, -NHC(O)NHC₁-C₁₀alkyl, -NHC(O)NHPh substituted with one, 2 or 3 C₁-C₄ alkyl, C₁-C₃ alkoxy, halo, C₁-C₃ alkylthio, trifluoromethyl, C₂-C₆ dialkylamino, or nitro, -NHC(O)C₃-C₈cycloalkyl, -NHC(O)OC(CH₂CH₃)₂CH₃, -NHC(O)OC(CH₃)₂CH₂Cl, -NHC(O)OC(CH₃)₂CH₂CH₃, -NHC(O)-1-phenyl-1-cyclopentyl, -NHC(O)-1-methyl-1-cyclohexyl, -NHC(S)NHC(CH₃)₃ or -NHC(O)NHC(CH₃)₃.

Additional preferred embodiments of Formula I include:
7-(O-ethoxymethyl)-13-(N-Boc-β-phenyl isoserinyl)-baccatin III (4),
7-(O-methoxyethoxymethyl)-13-(N-Boc-β-phenyl isoserinyl)-baccatin III (6),
7-(O-methoxymethyl)-13-(N-Boc-2'-β-phenyl isoserinyl)-baccatin III (8),
7-(O-benzyloxymethyl)-13-(N-Boc-β-phenyl isoserinyl)-baccatin III (10),
7-[O-(2,2,2-trichloroethoxy)methyl]-13-(N.Hoc-β-phenyl isoserinyl)-baccatin III (21),
7-[O-(2,2,2-trichloroethoxy)methoxymethyl]-13-(N-Boc-β-phenyl isoserinyl)-baccatin III (22),
7-(O-methylthiomethyl) taxol (42),
7-(O-methylthiomethyl)-13.(N-Boc-β-phenyl isoserinyl)-baccatin III (44) and
7-(O-phenylthiomethyl) taxol (46);
7-O-methyl Taxol (47)
7-[O-ethyl(1-thioethyl)] Taxol (49)
13-(N-(t-butylaminocarbonyl)-b-phenyl isoserinyl)-baccatin III 7-O-methylthiomethyl ether (55)
13-(N-(t-butylaminocarbonyl)-b-phenyl isoserinyl)-baccatin III 7-O-methyl ether (56)
13-(N-Boc-2'-TES-b-phenyl isoserinyl)-baccatin III 7-O-methyl ether (58) more preferably:
7-(O-ethoxymethyl)-13-(N-(t-butylaminocarbonyl)-β-phenyl isoserinyl)-baccatin III (14) and
7-(O-methoxymethyl)-13-(N-(t-butylaminocarbonyl)-β-phenyl isoserinyl)-baccatin III (27).

A preferred embodiment of the subject invention are compounds of Formula I where R₁ is preferably phenyl or phenyl substituted with halo, R₂ is -NHC(O)NHC(CH₃)₃, R₃ and R₅ are -H, R₄ is -OH, and R₃₀ is -OH or -OCOCH₃.

The compounds of Formula I include both the 7-α and 7-β configuration of the 7-ether substitution.

Preferred members of the moiety -O-C₅-C₁₅ heteroalkyl include:

OCH₂-(2- or 3-furyl), OCH₂(2- or 3-pyrrolyl), OCH₂(2-, 3, or 4-pyridyl), OCH₂(2-, 4- or 5-imidazoyl) and OCH₂(3-, 4- or 5-isoxazolyl).

An embodiment of the present invention are 7-deoxy-7-W-taxol analogs of general Formula I wherein:
R₁ is selected from the group consisting of -CH₃, -C₆H₅ or phenyl substituted with one, 2 or 3 C₁-C₄ alkyl, C₁-C₃ alkoxy, halo, C₁-C₃ alkylthio, trifluoromethyl, C₂-C₆ dialkylamino, hydroxy or nitro;
R₂ is selected from the group consisting of -H, -NHC(O)C₁-C₁₀alkyl (preferably -NHC(O)C₄-C₆alkyl), -NHC(O)phenyl, -NHC(O)phenyl substituted with one, 2 or 3 C₁-C₄ alkyl, C₁-C₃ alkoxy, halo, C₁-C₃ alkylthio, trifluoromethyl, C₂-C₆ dialkylamino, hydroxy or nitro, -NHC(O)C(CH₃)=CHCH₃, -NHC(O)OC(CH₃)₃, -NH₂, -NHSO₂-4-methylphenyl, -NHC(O)(CH₂)₃COOH, -NHC(O)-4-(SO₃H)phenyl, -OH, -NHC(O)-1-adamantyl, -NHC(O)O-3-tetrahydrofuranyl, -NHC(O)O-4-tetrahydropyranyl, -NHC(O)CH₂C(CH₃)₃, -NHC(O)C(CH₃)₃, -NHC(O)OC₁-C₁₀alkyl, -NHC(O)NHC₁-C₁₀alkyl, -NHC(O)NHC(CH₃)₃, -NHC(O)NHPh substituted with one, 2 or 3 C₁-C₄ alkyl, C₁-C₃ alkoxy, halo, C₁-C₃ alkylthio, trifluoromethyl, C₂-C₆ dialkylamino, or nitro, -NHC(O)C₃-C₈cycloalkyl;
R₃ is selected from the group consisting of -H, -NHC(O)phenyl or -NHC(O)OC(CH₃)₃; with the overall proviso that one of R₂ and R₃ is -H but R₂ and R₃ are not both -H;
R₄ is -H or selected from the group consisting of -OH, -OAc (-OC(O)CH₃), -OC(O)OCH₂C(Cl)₃, -OCOCH₂CH₂NH₃⁺ HCOO⁻, -NHC(O)phenyl, -NHC(O)OC(CH₃)₃, -OCOCH₂CH₂COOH and pharmaceutically acceptable salts thereof, -OCO(CH₂)₃COOH and pharmaceutically acceptable salts thereof, and -OC(O)-Z-C(O)-R' [where Z is ethylene (-CH₂CH₂-), propylene (-CH₂CH₂CH₂-), -CH=CH-, 1,2-cyclohexane or 1,2-phenylene, R' is -OH, -OH base, -NR'₂R'₃, -OR'₃, -SR'₃, -OCH₂C(O)NR'₄R'₅ where R'₂ is -H or -CH₃, R'₃ is -(CH₂)ₙNR'₆R'₇ or (CH₂)ₙN+R'₆R'₇R'₈ X⁻ where n is 1-3, R'₄ is -H or -C₁-C₄alkyl, R'₅ is -H, -C₁-C₄alkyl, benzyl, hydroxyethyl, -CH₂CO₂H or dimethylaminoethyl, R'₆ and R'₇ are -CH₃, -CH₂CH₃, benzyl or R'₆ and R'₇ together with the nitrogen of NR'₆R'₇ form a pyrrolidino, piperidino, morpholino, or N-methylpiperizino group; R'₈ is -CH₃, -CH₂CH₃ or benzyl , X⁻ is halide, and base is NH₃, (HOC₂H₄)₃N, N(CH₃)_{3,} CH₃N(C₂H₄)₂NH⁻, NH₂(CH₂)₆NH₂, N-methylglucamine, NaOH or KOH], -OC(O) (CH₂)ₙNR²R³ [where n is 1-3, R² is -H or -C₁-C₃alkyl and R³ -H or -C₁-C₃alkyl], -OC(O)CH(R")NH₂ [where R" is selected from the group consisting of -H, -CH₃, -CH₂CH (CH₃)₂, -CH(CH₃)CH₂CH₃, -CH(CH₃)₂, -CH₂phenyl, -(CH₂)₄NH₂, -CH₂CH₂COOH, -(CH₂)₃ NHC(=NH)NH₂], the residue of the amino acid proline, -OC(O)CH=CH₂, -C(O)CH₂CH₂C(O)NHCH₂CH₂SO₃⁻ Y⁺, -OC(O)CH₂ CH₂C(O)NHCH₂CH₂CH₂SO₃ ⁻Y⁺ wherein Y⁺ is Na⁺ or N⁺(Bu)₄, -OC(O)CH₂CH₂C(O)OCH₂ CH₂OH;
R₅ is -H or -OH, with the overall proviso that when R₅ is -OH, R₄ is -H and with the further proviso that when R₅ is -H, R₄ is other than -H;
R₃₀ is -H, -OH or -OC(O)CH₃; and
pharmaceutically acceptable salts thereof when the compound contains either an acidic or basic functional group.

Another embodiment of the present invention are 7-deoxy-7-W-taxol analogs of general Formula I wherein:
R₁ is selected from the group consisting of -CH₃, -C₆H₅ or phenyl substituted with one, 2 or 3 C₁-C₄ alkyl, C₁-C₃ alkoxy, halo, C₁-C₃ alkylthio, trifluoromethyl, C₂-C₆ dialkylamino, hydroxy or nitro;
R₂ is selected from the group consisting of -H, -NHC(O)C₁-C₁₀alkyl (preferably -NHC(O)C₄-C₆alkyl), -NHC(O)phenyl, -NHC(O)phenyl substituted with one, 2 or 3 C₁-C₄ alkyl, C₁-C₃ alkoxy, halo, C₁-C₃ alkylthio, trifluoromethyl, C₂-C₆ dialkylamino, hydroxy or nitro, -NHC(O)C(CH₃)=CHCH₃, -NHC(O)OC(CH₃)₃, -NH₂, -NHSO₂-4-methylphenyl, -NHC(O)CH₂)₃COOH, -NHC(O)-4-(SO₃H)phenyl, -OH, -NHC(O)-1-adamantyl, -NHC(O)O-3-tetrahydrofuranyl, -NHC(O)O-4-tetrahydropyranyl, -NHC(O)CH₂C(CH₃)₃, -NHC(O)C(CH₃)₃, -NHC(O)OC₁-C₁₀alkyl, -NHC(O)NHC₁-C₁₀alkyl, -NHC(O)NHC(CH₃)₃, -NHC(O)NHPh substituted with one, 2 or 3 C₁-C₄ alkyl, C₁-C₃ alkoxy, halo, C₁-C₃ alkylthio, trifluoromethyl, C₂-C₆ dialkylamino, or nitro, -NHC(O)C₃-C₈cycloalkyl; and
W is selected from the group consisting of
   O-methyl;
   O-propyl;
   O-allyl;
   O-methoxymethyl;
   O-ethoxymethyl;
   O-methoxyethoxymethyl;
   O-benzyloxymethyl;
   O-(2,2,2-trichloroethoxy)methyl;
   O-(2,2,2-trichloroethoxy)methoxymethyl;
   O-methylthiomethyl; and
   O-phenylthiomethyl
R₃, R₄, R₅ and R₃₀ are as defined above.

A further preferred embodiment of the present invention are 7-deoxy-7-W-taxol analogs of general Formula I wherein:
R₁ is selected from the group consisting of -CH₃, -C₆H₅ or phenyl substituted with one, 2 or 3 C₁-C₄ alkyl, C₁-C₃ alkoxy, halo, C₁-C₃ alkylthio, trifluoromethyl, C₂-C₆ dialkylamino, hydroxy or nitro;
R₂ is selected from the group consisting of -H, -NHC(O)C₁-C₁₀ alkyl (preferably -NHC(O)C₄-C₆alkyl), -NHC(O)phenyl, -NHC(O)phenyl substituted with one, 2 or 3 C₁-C₄ alkyl, C₁-C₃ alkoxy, halo, C₁-C₃ alkylthio, trifluoromethyl, C₂-C₆ dialkylamino, hydroxy or nitro, -NHC(O)C(CH₃)=CHCH₃, -NHC(O)OC(CH₃)₃, -NH₂, -NHSO₂-4-methylphenyl, -NHC(O)(CH₂)₃COOH, -NHC(O)-4-(SO₃H)phenyl, -OH, -NHC(O)-1-adamantyl, -NHC(O)O-3-tetrahydrofuranyl, -NHC(O)O-4-tetrahydropyranyl, -NHC(O)CH₂C(CH₃)₃, -NHC(O)C(CH₃)₃, -NHC(O)OC₁-C₁₀alkyl, -NHC(O)NHC₁-C₁₀alkyl, NHC(O)NHC(CH₃)₃, -NHC(O)NHPh substituted with one, 2 or 3 C₁-C₄ alkyl, C₁-C₃ alkoxy, halo, C₁-C₃ alkylthio, trifluoromethyl, C₂-C₆ dialkylamino, or nitro, -NHC(O)C₃-C₈cycloalkyl;
W is selected from the group consisting of
   O-ethoxymethyl;
   O-methoxyethoxymethyl;
   O-benzyloxymethyl;
   O-(2,2,2-trichloroethoxy)methyl;
   O-(2,2,2-trichloroethoxy)methoxymethyl;
   O-methylthiomethyl; and
   O-phenylthiomethyl;
   and
R₃, R₄, R₅ and R₃₀ are as defined above.

In compounds of Formula I, W is preferably selected from the group consisting of
O-methyl;
O-propyl;
O-allyl;
O-methoxymethyl;
O-ethoxymethyl;
O-methoxyethoxymethyl;
O-benzyloxymethyl;
O-(2,2,2-trichloroethoxy)methyl;
O-(2,2,2-trichloroethoxy)methoxymethyl;
O-methylthiomethyl; and
O-phenylthiomethyl;
more preferably
O-methoxymethyl;
O-ethoxymethyl;
O-methoxyethoxymethyl;
O-benzyloxymethyl;
O-(2,2,2-trichloroethoxy)methyl;
O-(2,2,2-trichloroethoxy)methoxymethyl;
O-methylthiomethyl; and
O-phenylthiomethyl.

Examples of -O-C₅-C₁₅ heteroalkyl include: -OCH₂(2- or 3-furyl), -OCH₂(2-or 3-pyrrolyl), -OCH₂(2-, 3- or 4-pyridyl), -OCH₂(2-, 3-, 4-, 5-, 6-, 7- or 8-quinolinyl), -OCH₂(1-, 3-, 4-, 5-, 6-, 7- or 8-isoquinolinyl), -OCH₂(2-, 4- or 5-imidazoyl), -OCH₂(3-, 4- or 5-pyrazolyl), -OCH₂(2-pyrazinyl), -OCH₂(2-, 4-, 5- or 6-pyrimidinyl), -OCH₂(2-, 3-, 4-, 5-, 6- or 7-indolyl) and -OCH₂(3-, 4- or 5-isoxazolyl).

Examples of C₁-C₆ alkyl include straight and branched alkyl chains, including for example methyl, ethyl, isopropyl, t-butyl, isobutyl and 2-methyl-pentyl.

Examples of C₁-C₃ alkoxy are methoxy, ethoxy, propoxy and isomeric forms thereof.

The present invention also provides a process for preparing oxazolidines of Formula 5 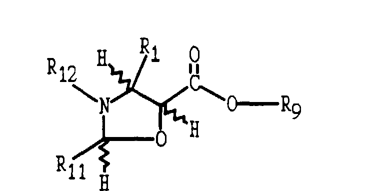 in which
R₁ is as defined above;
R₉ is selected from C₁-C₆alkyl; R₁₁ is phenyl substituted with -(OC₁-C₂alkyl)ₙ where n is 1 to 3;
R₁₂ is selected from the group consisting of -C(O)H, -C(O)C₁-C₁₀alkyl (preferably -C(O)C₄-C₆alkyl), -C(O)phenyl, -C(O)phenyl substituted with one, 2 or 3 C₁-C₄ alkyl, C₁-C₃ alkoxy, halo, C₁-C₃ alkylthio, trifluoromethyl, C₂-C₆ dialkylamino, hydroxy or nitro, -C(O)C(CH₃)=CHCH₃, -C(O)OC(CH₃)₃, -C(O)OCH₂phenyl, -SO₂-4-methylphenyl, -C(O)(CH₂)₃COOH, -C(O)-4(SO₃H)phenyl, -C(O)-1-adamantyl, -C(O)O-3-tetrahydrofuranyl, -C(O)O-4-tetrahydropyranyl, -C(O)CH₂C(CH₃)₃, -C(O)C(CH₃)₃, -C(O)OC₁-C₁₀alkyl, -C(O)NHC₁-C₁₀alkyl, -C(O)NHPh substituted with one, 2 or 3 C₁-C₄ alkyl, C₁-C₃ alkoxy, halo, C₁-C₃ alkylthio, trifluoromethyl, C₂-C₆ dialkylamino, or nitro, or -C(O)C₃-C₈cycloalkyl, -C(O)C(CH₂CH₃)₂CH₃, -C(O)C(CH₃)₂CH₂Cl, -C(O)C(CH₃)₂CH₂CH₃, -C(O)-1-phenyl-1-cyclopentyl, -C(O)-1-methyl-1-cyclohexyl, -C(S)NHC(CH₃)₃, -C(O)NHC(CH₃)₃ or -C(O)NHPh; which comprises reacting a hydroxy-amine of Formula 3 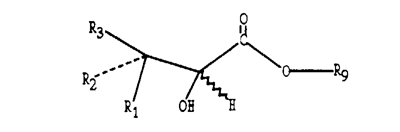 in which R₁ and R₃ are as defined above and R₂ is selected from the group consisting of -NHC(O)H,-NHC(O)C₁-C₁₀alkyl (preferably -NHC(O)C₄-C₆alkyl), -NHC(O)phenyl, -NHC(O)phenyl substituted with one, 2 or 3 C₁-C₄ alkyl, C₁-C₃ alkoxy, halo, C₁-C₃ alkylthio, trifluoromethyl, C₂-C₆ dialkylamino, hydroxy or nitro, -NHC(O)C(CH₃)=CHCH₃, -NHC(O)OC(CH₃)₃, -NHC(O)OCH₂phenyl, -NHSO₂-4-methylphenyl, -NHC(O)(CH₂)₃COOH, -NHC(O)-4-(SO₃H)phenyl, -NHC(O)-1-adamantyl, -NHC(O)O-3-tetrahydrofuranyl, -NHC(O)O-4-tetrahydropyranyl, -NHC(O)CH₂C(CH₃)₃, -NHC(O)C(CH₃)₃, -NHC(O)OC₁-C₁₀alkyl, -NHC(O)NHC₁-C₁₀alkyl, -NHC(O)NHPh substituted with one, 2 or 3 C₁-C₄ alkyl, C₁-C₃ alkoxy, halo, C₁-C₃ alkylthio, trifluoromethyl, C₂-C₆ dialkylamino, or nitro, or -NHC(O)C₃-C₈cycloalkyl, -NHC(O)C(CH₂CH₃)₂CH₃, -NHC(O)C(CH₃)₂CH₂Cl, -NHC(O)C(CH₃)₂CH₂CH₃, -NHC(O)-1-phenyl- 1-cyclo-pentyl, -NHC(O)-1-methyl-1-cyclohexyl, -NHC(S)NHC(CH₃)₃, -NHC(O)NHC(CH₃)₃ or -NHC(O)NHPh;
   with (1) an electron rich benzaldehyde of Formula 4A 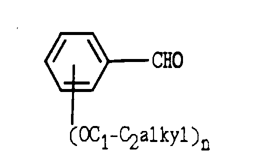 or (2) an electron rich acetal of Formula 4 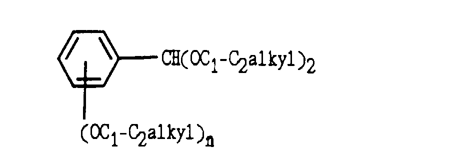 where n is 1-3.

In addition, the present invention provides a process of preparing 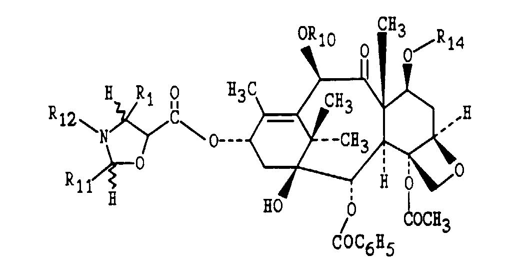 which comprises reacting an oxazolidine free acid of Formula 7 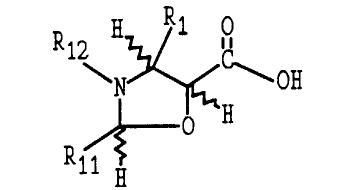 with a baccatin compound of Formula 8 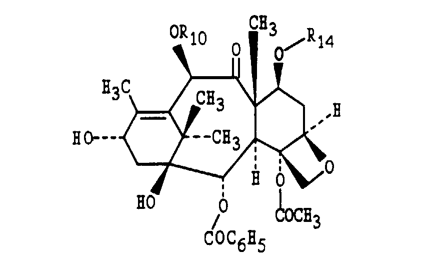 in the presence of a dehydrating agent. Wherein R₁₀ and R₁₄, being the same or different, are selected from the group consisting of -C(O)C₁-C₆alkyl (preferably -C(O)CH₃), -C(O)OC₁-C₆alkyl, -C(O)OCH₂CX₃ where X is Halo, -C(O)OCH₂CH₂SiR₂₀ (where R₂₀ is C₁-C₆ alkyl), or -Si(R₂₀)₃ or R₁₄ is is selected from the group consisting of
-C₁-C₁₀alkyl,
-C₃-C₁₀ unsaturated alkyl (preferably allyl, crotyl),
-C₅-C₁₅ heteroalkyl [e.g. -CH₂(2- or 3-furyl), -CH₂(2- or 3-pyrrolyl), -CH₂(2-, 3, or 4-pyridyl), -CH₂(2-, 3-, 4-, 5-, 6-, 7- or 8-quinolinyl), -CH₂(1-, 3-, 4-, 5-, 6-, 7- or 8-isoquinolinyl), -CH₂(2-, 4- or 5-imidazoyl), -CH₂(3-, 4- or 5-pyrazolyl), -CH₂(2-pyrazinyl), -CH₂(2-, 4-, 5- or 6-pyrimidinyl), -CH₂(2-, 3-, 4-, 5-, 6- or 7-indolyl), -CH₂(3-, 4- or 5-isoxazolyl); preferably -CH₂(2- or 3-furyl), -CH₂(2- or 3-pyrrolyl), -CH₂(2-, 3- or 4-pyridyl), -CH₂(2-, 4- or 5-imidazoyl), -CH₂(3-, 4- or 5-isoxazolyl)],
-O-CH(R²¹)OR²² where
   R²¹ is -H, -C₁-C₆ alkyl, and
   R²² is -C₁₋C₁₀alkyl, -C₃-C₁₀ unsaturated alkyl (preferably allyl, crotyl ),-C₅-C₁₅ heteroalkyl [e.g. -CH₂(2- or 3-furyl), -CH₂(2- or 3-pyrrolyl), -CH₂(2-, 3, or 4-pyridyl), -CH₂(2-, 3-, 4-, 5-, 6-, 7- or 8-quinolinyl), -CH₂(1-, 3-, 4-, 5-, 6-, 7- or 8-isoquinolinyl), -CH₂(2-, 4- or 5-imidazoyl), -CH₂(3-, 4- or 5-pyrazolyl), -CH₂(2-pyrazinyl), -CH₂(2-, 4-, 5- or 6-pyrimidinyl), -CH₂(2-, 3-, 4-, 5-, 6- or 7-indolyl), -CH₂(3-, 4- or 5-isoxazolyl); preferably -CH₂(2- or 3-furyl), -CH₂(2- or 3-pyrrolyl), -CH₂(2-, 3, or 4-pyridyl), -CH₂(2-, 4- or 5-imidazoyl), -CH₂(3-, 4- or 5-isoxazolyl)]
or when R²¹ and R²² are taken together to form a ring with 4 to 6 carbon atoms,
-CH(R²⁸)S(O)ₘAr
   where Ar is phenyl or phenyl substituted with one, 2 or 3 C₁-C₄ alkyl, C₁-C₃ alkoxy, halo, C₁-C₃ alkylthio, trifluoromethyl, C₂-C₆ dialkylamino, or nitro, or
   -CH(R²⁸)S(O)ₘ,CH₂R²⁸
      where R²⁸ is
      C₁-C₆ alkyl,
      -C₃-C₁₀ unsaturated alkyl (preferably allyl, crotyl),
      -(CH₂)_{q}phenyl where q is 1-6,
      -(CH₂)_{q}phenyl where q is 1-6 and substituted with one, 2 or 3 C₁-C₄ alkyl, C₁-C₃ alkoxy, halo, C₁-C₃ alkylthio, trifluoromethyl, C₂-C₆ dialkylamino, or nitro,
      -naphthyl,
      -naphthyl substituted with one, 2 or 3 C₁-C₄ alkyl, C₁-C₃ alkoxy, halo, C₁-C₃ alkylthio, trifluoromethyl, C₂-C₆ dialkylamino, or nitro, -C₅-C₁₅ heteroalkyl [e.g. -(2- or 3-furyl), -(2- or 3-pyrrolyl), -(2-, 3, or 4-pyridyl), -(2-, 3-, 4-, 5-, 6-, 7- or 8-quinolinyl), -(1-, 3-, 4-, 5-, 6-, 7- or 8-isoquinolinyl), -(2-, 4- or 5-imidazoyl), -(3-, 4- or 5-pyrazolyl), - (2-pyrazinyl), (2-, 4-, 5- or 6-pyrimidinyl), -(2-, 3-, 4-, 5-, 6- or 7-indolyl), -(3-, 4- or 5-isoxazolyl); preferably -(2- or 3-furyl), -(2- or 3-pyrrolyl), -(2-, 3, or 4-pyridyl), -(2-, 4- or 5-imidazoyl), -(3-, 4- or 5-isoxazolyl)]

   or when R²⁸ and R²⁸ are taken together to form a ring with 4 to 6 carbon atoms;
   m is O to 2; and
   R₁₁ and R₁₂ are as defined above.

The compounds of this invention (Formula I) may be prepared by the procedure(s) as shown in Charts A', A", A"' and B.

As used in Charts 2-20, the terms R₂₀, R₂₃, R₂₄, R₂₅, R₂₆ and R₂₇ are defined as follows:
R₂₀ is selected from the group consisting of
-C₁C₁₀alkyl,
-C₃-C₁₀ unsaturated alkyl (preferably allyl, crotyl),
-C₅-C₁₅ heteroalkyl [e.g. -CH₂(2- or 3-furyl), -CH₂(2- or 3-pyrrolyl), -CH₂(2-, 3, or 4-pyridyl), -CH₂(2-, 3-, 4-, 5-, 6-, 7- or 8-quinolinyl), -CH₂(1-, 3-, 4-, 5-, 6-, 7- or 8-isoquinolinyl), -CH₂(2-, 4- or 5-imidazoyl), -CH₂(3-, 4- or 5-pyrazolyl), -CH₂(2-pyrazinyl), -CH₂(2-, 4-, 5- or 6-pyrimidinyl), -CH₂(2-, 3-, 4-, 5-, 6- or 7-indolyl), -CH₂(3-, 4- or 5-isoxazolyl); preferably -CH₂(2- or 3-furyl), -CH₂(2- or 3-pyrrolyl), -CH₂(2-, 3- or 4-pyridyl), -CH₂(2-, 4- or 5-imidazoyl), -CH₂(3-, 4- or 5-isoxazolyl)],
-O-CH(R²¹)OR²² where
   R²¹ is -H, -C₁-C₆ alkyl, and
   R²² is -C₁-C₁₀alkyl, -C₃-C₁₀ unsaturated alkyl (preferably allyl, crotyl), -C₅-C₁₅ heteroalkyl [e.g. -CH₂(2- or 3-furyl), -CH₂(2- or 3-pyrrolyl), -CH₂(2-, 3, or 4-pyridyl), -CH₂(2-, 3-, 4-, 5-, 6-, 7- or 8-quinolinyl), -CH₂(1-, 3-, 4-, 5-, 6-, 7- or 8-isoquinolinyl), -CH₂(2-, 4- or 5-imidazoyl), -CH₂(3-, 4- or 5-pyrazolyl), -CH₂(2pyrazinyl), -CH₂(2-, 4-, 5- or 6-pyrimidinyl), -CH₂(2-, 3-, 4-, 5-, 6- or 7-indolyl), -CH₂(3-, 4- or 5-isoxazolyl); preferably -CH₂(2- or 3-furyl), -CH₂(2- or 3-pyrrolyl), -CH₂(2-, 3, or 4-pyridyl), -CH₂(2-, 4- or 5-imidazoyl), -CH₂(3-, 4- or 5-isoxazolyl)]
or when R²¹ and R²² are taken together to form a ring with 4 to 6 carbon atoms,
-CH(R²⁸)S(O)ₘAr
   where Ar is phenyl or phenyl substituted with one, 2 or 3 C₁-C₄ alkyl, C₁-C₃ alkoxy, halo, C₁-C₃ alkylthio, trifluoromethyl, C₂- C₆ dialkylamino, or nitro,
   or

-CH(R²⁸)S(O)ₘCH₂R²⁸
   where R²⁸ is
   C₁-C₆ alkyl,
   -C₃-C₁₀ unsaturated alkyl (preferably allyl, crotyl),
   -(CH₂)_{q}phenyl where q is 1-6,
   -(CH₂)_{q}phenyl where q is 1-6 and substituted with one, 2 or 3 C₁-C₄ alkyl, C₁-C₃ alkoxy, halo, C₁-C₃ alkylthio, trifluoromethyl, C₂-C₆ dialkylamino, or nitro,
   -naphthyl,
   -naphthyl substituted with one, 2 or 3 C₁-C₄ alkyl, C₁-C₃ alkoxy, halo, C₁-C₃ alkylthio, trifluoromethyl, C₂-C₆ dialkylamino, or

   - nitro,: -C₅-C₁₅ heteroalkyl [e.g. -(2- or 3-furyl), -(2- or 3-pyrrolyl), -(2-, 3, or 4-pyridyl), -(2-, 3-, 4-, 5-, 6-, 7- or 8-quinolinyl), -(1-, 3-, 4-, 5-, 6-, 7- or 8-isoquinolinyl), -(2-, 4- or 5-imidazoyl), -(3-, 4- or 5-pyrazolyl), - (2-pyrazinyl), (2-, 4-, 5- or 6-pyrimidinyl), -(2-, 3-, 4-, 5-, 6-or 7-indolyl), -(3-, 4- or 5-isoxazolyl); preferably -(2- or 3-furyl), -(2- or 3-pyrrolyl), -(2-, 3, or 4-pyridyl), -(2-, 4- or 5-imidazoyl), -(3-, 4- or 5-isoxazolyl)]
or when R²⁸ and R²⁸ are taken together to form a ring with 4 to 6 carbon atoms;
m is 0 to 2;
R₂₃ is selected from the group consisting of -H, -C₁-Cₗ₀alkyl (preferably -C₄-C₆alkyl), -phenyl, -phenyl substituted with one, 2 or 3 C₁-C₄ alkyl, C₁-C₃ alkoxy, halo, C₁-C₃ alkylthio, trifluoromethyl, C₂-C₆ dialkylamino, hydroxy or nitro, C(CH₃)=CHCH₃, -OC(CH₃)_{3,} -OCH₂phenyl, -SO₂-4-methylphenyl, -(CH₂)₃COOH, 4-(SO₃H)phenyl, -1-adamantyl, -O-3-tetrahydrofuranyl, -O-4-tetrahydropyranyl,-CH₂C(CH₃)₃, -C(CH₃)₃, -OC₁-C₁₀alkyl, -NHC₁-C₁₀alkyl, -NHPh substituted with one, 2 or 3 C₁-C₄ alkyl, C₁-C₃ alkoxy, halo, C₁-C₃ alkylthio, trifluoromethyl, C₂-C₆ dialkylamino, or nitro, or -C₃-C₈cycloalkyl, -C(CH₂CH₃)₂CH₃, -C(CH₃)₂CH₂Cl, -C(CH₃)₂CH₂CH₃, --1-phenyl-1-cyclopentyl, -1-methyl-1-cyclohexyl, -C(S)NHC(CH₃)₃, -NHC(CH₃)₃ or -NHPh.
R₂₄ is preferably Troc and TES.
R₂₅ is phenyl substituted with -(OC₁-C₂alkyl)ₙ where n is 1 to 3;
R₂₆ is -H; and
R₂₇ is selected from the group consisting of -C(O)C₁-C₆alkyl (preferably -C(O)CH₃), -C(O)OC₁-C₆alkyl, -C(O)OCH₂CX₃ where X is Halo, -C(O)OCH₂CH₂SiR₂₀ (where R₂₀ is C₁-C₆ alkyl), or -Si(R₂₀)₃, preferably Troc and TES.

The preparation of 3-azido-2-hydroxy-carboxylic acid esters 1 may be prepared as described in the literature (see Denis, J-N.; Correa, A; Greene, A. E. *J.* *Org. Chem.,* 1990, *55*, 1957). These materials are readily hydrogenated to the free amines 2, even though the literature intentionally avoids this intermediate by preparing the hydroxy-acylated intermediate prior to the reduction of the azide. The amine 2 is sufficiently stable that no problem is encountered in isolating it and directly using it to prepare the N-acylated free hydroxy compounds 3. Compounds 3 have been utilized by protection of the hydroxy group, hydrolysis of the ester to the acid, and condensation directly with a baccatin III derivative or after conversion to the oxazinone (European Patent 0 428 376 A1, US 436235). These procedures are distinctly inferior because they require large excesses of the acylating agent and generally do not proceed beyond about 60% completion. Procedures have also been described using a beta-lactam intermediate but these also require large excesses of reagent or the introduction of very strong bases such as LDA which makes them more difficult to perform and unsuitable for certain analogs (Ojima, I.; Habus, I.; Zhao, M.; George, G. I.; Jayasinghe, L. R. *J. Org. Chem.,* 1991, *56*, 1681, EP 0 400 971 A2). A very effective condensation procedure involving the conversion of the hydroxy-amine derivative 3 to an oxazolidine with 2 non hydrogen substituents at the 2 position was described by Commerçon, A; Bézard, D.; Bernard, F.; Bourzat, J. D. in *Tetrahedron Lett.,* 1992, *33,* 5185 and Patent WO 92/09589. The condensation proceeds in very high yield but the removal of the protecting group requires sufficiently strong acid that sensitive taxol analogs are destroyed under the deprotection conditions. We have modified and improved this procedure by formation of the oxazolidines 5 not with a ketone, as the above workers have used but, with an electron rich benzaldehyde 4.

Such chemistry was recently described by Didier, E.; Fouque, E.; Taillepied, I, Commercon, A. Tetrahedron Lett. 1994, 35, 2349. The oxazolidines derived from the benzaldehyde 4 are produced as a mixture of diastereomers but these have been separated in some cases and the diastereomers have been shown to be equally useful when carried on in the synthesis. The oxazolidines 5 are readily hydrolyzed to the salts 6 and the acids 7. The acid is labile and needs to be used shortly after preparation. Both oxazolidine isomers are equally effective in the condensation reaction with the protected baccatins 8 giving an excellent yield of the oxazolidine protected taxol analogs 9. More importantly, both oxazolidine isomers from these electron rich benzaldehydes are readily hydrolyzed under very mild acid conditions allowing deprotection without causing undesired transformations of highly acid sensitive taxol derivatives such as 10 which are the subject of this invention. There are references to the use of electron rich aldehydes for the protection of 1,2-diols as dioxolanes but no previous reference to the use of such aldehydes for the protection of 2-hydroxy protected amines except for the Didier reference cited above. The deprotection may be carried out such that both the oxazolidine and the 7 protected hydroxyl of 9 are removed at the same time or each may be removed independently. Additionally described is the deprotection of selected urethane analogs 10 to the free amine 11 (Chart B). These are then reconverted to a variety of amine acylated analogs 10.

The conversion of azide 1 to the amine 2 is effected by reduction as is known in the art. Thus, the reaction may be carried out by hydrogenation in the presence of a variety of hydrogenation catalysts such as palladium, platinum, rhodium, or ruthenium. Alternatively, the azide may be reduced by treatment with a phosphine such as triphenyl or tributyl phosphine or by an acid such as hydrochloric, sulfuric, trifluoroacetic or hydrobromic in the presence of a metal such as zinc, iron, or tin. These reactions may be effected in a solvent such as ethanol, methanol, ethyl acetate, methyl t-butyl ether or tetrahydrofuran and the like. The conversion of amine 2 to its acylated derivative 3 is effected by treatment of the amine in pyridine or a non basic solvent such as methylene chloride or tetrahydrofuran containing a tertiary amine such as triethyl amine or ethyl diisopropyl amine with an acylation agent. If 3 is a urethane, 2 is treated with an agent such as benzylchloroformate, 2,2,2-trichloroethoxycarbonyl chloride, di-tert-butyldicarbonate, or other urethane forming agent as is known in the art. If 3 is an amide, 2 is treated with an acylating agent such as an acyl halide, and acyl anhydride, or other acylating agent as is known in the art. If 3 is a urea or a thiourea, 2 is treated with an agent such as alkyl or aryl isocyanate, alkyl or aryl isothiocyanate, or other urea or thiourea forming agent as is known in the art.

An alternate method for the preparation of compounds of formula 3 (where R₂ = R₁₂NH-, R₃ = -H, and R₉ = -H) is shown in Chart A". The penultimate compound shown in Chart A" is a compound of formula 3 wherein R₂ = R₁₂NH-, R₃ = -H, and R₉ = -*t*-Bu. In Chart A", TMS is a trimethylsilyl group, TMSCl is chlorotrimethylsilane, and LDA is lithium diisopropyl amide.

Another alternate method for the preparation of compounds of formula 3 (where R₂ = R₁₂NH-, R₃ = -H, and R₉ = -H) is shown in Chart A"'. In Chart A"', Tₛ is a *p*-toluenesulfonyl (tosyl) group.

The hydroxy acids prepared in Charts A" and A"' may further be converted to compounds of formula 3 where R₂ = R₁₂NH-, R₃ = -H, and R₉ = -CH₃ by reaction with diazomethane or esterification by other methods known in the art.

The hydroxy amide or urethane 3 is converted to the oxazolidine 5 by treatment with an electron rich benzaldehyde or its acetal such as dimethyl or diethyl acetal 4 and an acid catalyst such as p-toluene sulfonic acid, pyridinium p-toluene sulfonate or other acid catalysts known in the art in a solvent such as tetrahydrofuran, toluene, methylene chloride, or other aprotic solvent. Examples of electron rich benzaldehydes include but are not limited to 2-, 3-, 4-methoxybenzaldehyde; 2,4-, 3,5-, 2,5-dimethoxybenzaldehyde; 2,4,6-trimethoxybenzaldehyde; and 4-ethoxybenzaldehyde. The preferred benzaldehyde is 2,4-dimethoxybenzaldehyde. The oxazolidine formation is generally carried out by heating to reflux to distill both the solvent and to carry off the evolved water or alcohol. The ester of 5 is hydrolyzed to the salt 6 by treatment with an alkali or quaternerary amine hydroxide or by an alkali carbonate or other base as known in the art in a solvent such as water, methanol, ethanol, or other protic solvent. The reaction may by carried out from -78°C to 100°C. The product 6 is stable and may be isolated by evaporation of the solvents and stored as a solid or the reaction may be used directly to convert 6 to the acid 7 by treatment with acid. Generally, 7 is obtained by treating an aqueous solution of 6 in a separatory funnel with sufficient acid such as hydrochloric, sulfuric, potassium hydrogen sulfate, or the like, and partitioning the desired acid into an organic solvent such as ethyl acetate, methylene chloride, ether, or the like and evaporation of the solvent. The resultant acid 7 is sufficiently pure and stable for use in the next reaction but in general is not sufficiently stable for long term storage. The acid 7 is condensed with the baccatin derivative 8 to form the ester 9 with a dehydrating agent. Most preferred for this procedure is a carbodiimide such as dicyclohexyl carbodiimide, diisopropyl carbodiimide, di-p-tolyl carbodiimide, ethyl dimethylaminopropyl carbodiimide hydrochloride salt, or the like, and a basic catalyst, preferably 4-dimethylaminopyridine. The reaction is generally carried out in an aprotic solvent such as toluene, benzene, tetrahydrofuran, dioxane, or the like at 25°C to 100°C. Other dehydration procedures for the formation of 9 may be used such as conversion of 7 to its mixed ester with a sulfonic acid such as with toluenesulfonyl chloride or benzenesulfonyl chloride, or formation of the acid halide from the dried 6 in the presence of oxalyl chloride as is known in the art for acid sensitive carboxylic acids. The oxazolidines 9 may be deprotected so that the protecting oxazolidine and the groups blocking the hydroxyl at the baccatin 7 position are individually removed in either order or both removed together depending on the protecting group at the 7 position and on the reaction conditions. If R₁₄ is an acid labile group such as a silyl ether, then hydrolysis of the oxazolidine may be run under mild acid conditions and leads to the 7 position deprotection as well, giving 10MZ directly. Conditions for such conversions include hydrolysis in aqueous acetic acid, aqueous alcoholic acid of 0.01 to 0.1 N at 0°C to 50°C, or alcoholic acid of 0.01 to 0.1 N at 0°C to 50°C. Alternatively, the protection at the 7 position could be removed at a second step if it is not acid labile. For example, the trichloroethoxycarbonyl group at position 7 could be removed from 10MY (Chart B) by reduction as is known in the art to give 10MZ. Depending on the nature of the protecting group on the nitrogen (i.e. R₂ or R₃) of 10MZ (Chart B) the protecting group can be removed to give 11Z. For example, when R₂ is PhCH₂OC(O)NH, it may be removed by mild hydrogenolysis. Conditions for such conversions include reduction with hydrogen over a metal catalyst such as palladium in a solvent such as ethanol or ethyl acetate at room temperature and from one to three atmospheres of pressure. Other methods are known in the art. The resultant amine 11Z may be reconverted to a amide or urethane 10MZ (Chart B) by acylation procedures as described for the conversion of 2 to 3 above. The product 10MZ may be protected on the 2' hydroxyl to give 12MZ (Chart B). For example, the 2' hydroxyl may be acylated with trichloroethoxycarbonyl chloride in pyridine or other aromatic amine solvents, or in a non basic solvent such as toluene, methylene chloride, or tetrahydrofuran containing a tertiary amine base. The reaction may be run at -50°C to 100°C. Other methods for such acylations are well known in the art.

The reaction of taxol, taxol analogs 10MZ (R₁₀ is acetate or other suitable acyl moiety), baccatin III, or baccatin III analogs 8 (R₁₀ is acetate or other suitable acyl moiety) with hydrazine comprises a particularly advantageous method for preparation of 10-deacetyl taxol, 10-deacyl taxol analogs (10MZ, R₁₀ = H), 10-deacetyl baccatin III, and 10-deacyl baccatin III analogs (8, R₁₀ = H). Whereas the reported method (Samaranayake, *G.; et. al., J. Org. Chem.,* 1991,*56,* 5114) for removal of the acyl group from this position of taxol and baccatin structures, i.e., zinc bromide in methanol, gives a number of other products in addition to the desired deacylation product, the reaction with hydrazine gives almost exclusively the desired deacylation product. The reaction may be performed at room temperature in an organic solvent and usually requires as little time as 15 min or as much as 24 hr, depending on the substrate. The preferred solvent for the reaction is 95% ethanol and 98% hydrazine is the preferred form of the reagent.

The compounds Formula I of this invention [where R₄₀ is not equal to -C(O)C₆H₅)] can be prepared by the procedure shown in Chart D according to the method of Chaudhary, A. G.; *et.al., J.* Am. Chem. Soc., 1994, *116,* 4097-8.)

A general procedure for synthesizing the compounds of Formula I is set forth below.

The taxol analog III of chart 2 may be converted to a 2'- protected derivative IV by reaction with a trialkylchlorosilane in an aprotic solvent such THF, pyridine or DMF in the presence of a base such as imidazole or pyridine or with an alkoxy-carbonylchloride such as trichloroethylchloroformate, benzyloxychloroformate or allyloxychloroformate in an aprotic solvent such as methylene chloride or pyridine and an added base such as pyridine, triethyl amine or diisopropyl ethyl amine. A taxol analog III may be converted to a 2',3'-oxazolidine derivative V as described in Chart A' for the conversion of 3 to 5.

The baccatin analog VI of chart 3 may be converted to the 7-protected baccatin VII by reaction with a trialkylchlorosilane in an aprotic solvent such THF, pyridine or DMF in the presence of a base such as imidazole or pyridine or with an alkoxycarbonylchloride such as trichloroethylchloroformate, benzyloxychloroformate or allyloxychloroformate in an aprotic solvent such as methylene chloride or pyridine and an added base such as pyridine, triethyl amine or diisopropyl ethyl amine. The 7-protected baccatin VII of chart 3 may condensed with the oxazolidine acid VIII to form the ester IX with a dehydrating agent. Most preferred for this procedure is a carbodiimide such as dicyclohexyl carbodiimide, diisopropyl carbodiimide, di-p-tolyl carbodiimide, ethyl dimethylaminopropyl carbodiimide hydrochloride salt, or the like, and a basic catalyst, preferably 4-dimethylaminopyridine. The reaction is generally carried out in an aprotic solvent such as toluene, benzene, tetrahydrofuran, dioxane, or the like at 25°C to 100°C. Other dehydration procedures for the formation of IX may be used such as conversion of VIII to its mixed ester with a sulfonic acid such as with toluenesulfonyl chloride or benzenesulfonyl chloride, or formation of the acid halide from an dried alkali metal salt of VIII in the presence of oxalyl chloride as is known in the art for acid sensitive carboxylic acids. The 7-protected oxazolidine IX may be selectively deprotected to the 7-hydroxy oxazolidine V. If R²⁷ is a trialkyl silyl group the conversion of IX to V may be effected with a fluoride such as tetrabutyl ammonium fluoride, pyridinium fluoride or triethyl ammonium trihydrofluoride in an inert solvent such as THF or methylene chloride. If R²⁷ is a protecting group such as trichloroethoxycarbonyl it may be removed by reduction with zinc or other metal in the presence of a weak acid such a acetic acid or ammonium chloride in an solvent such as acetic acid or methanol or aqueous mixtures of such solvents.

A 2'-protected taxol analog X of Chart 4 with R²⁰ as an alkoxymethyl- or aryloxymethyl ether may be made from an 2'-protected-7-hydroxy-taxol IV by reaction with a chloromethyl alkyl or chloromethylaryl ether as is known in the art (Braun, H.; Hild, W. Angew. Chem. Int. Ed. Eng. 1984, 23, 723; Danishefsky, S.; Barbachyn, M. J. Am. Chem. Soc. 1985, 107, 7761; McCarvey, G. J.; Bajiva, J. S. J. Org. Chem. 1984, 49, 409; Falck, J. R.; Yadageri, P. J. Org. Chem. 1989, 54, 5851; Andreev, V. M.; Fonchenko, Z. V.; Cherkayev, G. V.; Mochalin, V. B.; Kheifits, L. A. Khim. Farm. Zh. 1990, 24, 50; Swindel, C. S.; Kraus, N. E.; Horwitz, S. B.; Ringel, I. J. Med. Chem. 1991, 34, 1176; Wu, Z. F.; Fraserreid, B.; Mootoo, D. R. Tetrahedron Lett. 1988, 29, 6549). A 2'-protected taxol analog X of Chart 4 with R²⁰ as an alkyl, allyl, or alkarylether may be made from an 2'-protected-7-hydroxy-taxol IV by the methods shown is charts 7-20 or by reaction with a diazo alkane or aryl diazo compound in the presence of a transition metal catalyst such as rhodium, ruthenium or palladium in an aprotic solvent such as THF, dioxane, or DMF at a temperature of -20 °C to 150 °C. A taxol analog XI may be prepared from the 2'-protected analog X by deprotection of the 2'-protecting group as is known in the art.

An oxazolidinyl-taxol analog XII of Chart 5 with R²⁰ as an alkoxymethyl- or aryloxymethyl ether may be made from an oxazolidinyl-7-hydroxy-taxol V by reaction with a chloromethyl alkyl or chloromethylaryl ether as is known in the art (Braun, H.; Hild, W. Angew. Chem. Int. Ed. Eng. 1984, 23, 723; Danishefsky, S.; Barbachyn, M. J. Am. Chem. Soc. 1985, 107, 7761; McCarvey, G. J.; Bajiva, J. S. J. Org. Chem. 1984, 49, 409; Falck, J. R.; Yadageri, P. J. Org. Chem. 1989, 54, 5851; Andreev, V. M.; Fonchenko, Z. V.; Cherkayev, G. V.; Mochalin, V. B.; Kheifits, L. A. Khim. Farm. Zh. 1990, 24, 50; Swindel, C. S.; Kraus, N. E.; Horwitz, S. B.; Ringel, I. J. Med. Chem. 1991, 34, 1176; Wu, Z. F.; Fraserreid, B.; Mootoo, D. R. Tetrahedron Lett. 1988, 29, 6549.). An oxazolidinyl taxol or analog XII of Chart 5 with R²⁰ as an alkyl-, allyl, or alkarylether may be made from an oxazolidinyl-7-hydroxy-taxol V by the methods shown in charts 7-20 or by reaction with a diazo alkane or aryl diazo compound in the presence of a transition metal catalyst such as rhodium, ruthenium or palladium in an aprotic solvent such as THF, dioxane, or DMF at a temperature of -20 °C to 150 °C. A taxol analog XI may be prepared from an oxazolidinyl analog XII by hydrolysis in aqueous acetic acid, aqueous alcoholic acid of 0.01 to 0.1 N at 0°C to 50°C, or alcoholic acid of 0.01 to 0.1 N at 0°C to 50° C or other method as is known in the art.

The baccatin analog VI of chart 6 may be converted to the 7-ether baccatin XIII in the same manner that IV of chart 4 is converted to X. The 7-ether baccatin XIII of chart 6 may condensed with the oxazolidine acid VIII to form the ester XII with a dehydrating agent as described for the condensation of VII with VIII of chart 3. The oxazolidine XII may be deprotected to the analog XI as described for Chart 5.

A 2'-protected taxol 7-ether XIV of chart 7 can be prepared from a 2-protected taxol analog IV by reaction with a dialkyl sulfide and benzoyl peroxide (Medina, J. C.; Soloman, M.; Kyler, K S. Tetrahedron Lett. 1988, 29, 3773.) or by reaction with a chloroalkylthioalkyl ether in the presence of a strong base such as sodium hydride or silver nitrate and a tertiary base such as triethyl amine or diisopropyl ethyl amine in an aprotic solvent such as THF, dioxane, or methylene chloride (Holton, R. A.; Davis, R. G. Tetrahedron Lett. 1977, 533, Suzuki, K; Inanaga, J.; Yamaguchi, M. Chem. Lett. 1979, 1277). Similarly, a 2'-protected taxol 7-arythioalkyl ether XV of chart 7 can be prepared from a 2-protected taxol analog IV by reaction with a aryl alkyl sulfide and benzoyl peroxide (Medina, J. C.; Soloman, M.; Kyler, K S. Tetrahedron Lett. 1988, 29, 3773.) or by reaction with a chloroalkylthioaryl ether in the presence of a strong base such as sodium hydride or silver nitrate and a tertiary base such as triethyl amine or diisopropyl ethyl amine in an aprotic solvent such as THF, dioxane, or methylene chloride (Holton, R. A.; Davis, R. G. Tetrahedron Lett. 1977, 533, Suzuki, K; Inanaga, J.; Yamaguchi, M. Chem. Lett. 1979, 1277).

A 2'-protected taxol 7-alkylthioalkyl XIV of Chart 8 may be oxidized to a sulfoxide XVI by sodium metaperiodate and alcoholic solvent or by other method known in the art (Carrasco, M.; Jones, R. J.; Kamel S.; Rapoport, H.; Truong, T. Org. Syn. 1991, 20 29; Johnson, C.; Keiser, L. Org. Syn. Coll Vol V , 1973, 791) or by other methods known in the art. Similarly, a 2'-protected taxol 7-arythioalkyl ether XV of Chart 9 may be oxidized to a sulfoxide XVIII by sodium metaperiodate and alcoholic solvent or by other method known in the art (Carrasco, M.; Jones, R. J.; Kamel S.; Rapoport, H.; Truong, T. Org. Syn. 1991, 20 29; Johnson, C.; Keiser, L. Org. Syn. Coll Vol V , 1973, 791) or by other methods known in the art.

A 2'-protected taxol 7-alkylthioalkyl ether XIV of Chart 8 may be oxidized to a sulfone XVII by meta chloroperbenzoic acid in aprotic solvents such as methylene chloride or THF or by hydrogen peroxide in aprotic or protic solvents such as methylene chloride, methanol, or ethanol (Carpino, L. A.; McAdams, L. V. Org. Syn. Coll. Vol. VI, 1988, 403; Paquette, L. A.; Carr, R. V. C. Org. Syn, 1985, 64, 157) or by other methods known in the art. Similarly, a 2'-protected taxol 7-arylthioalkyl XV of Chart 9 may be oxidized to a sulfone XIX by meta chloroperbenzoic acid in aprotic solvents such as methylene chloride or THF or by hydrogen peroxide in aprotic or protic solvents such as methylene chloride, methanol, or ethanol (Carpino, L. A.; McAdams, L. V. Org. Syn. Coll. Vol. VI, 1988, 403; Paquette, L. A.; Carr, R. V. C. Org. Syn, 1985, 64, 157) or by other methods known in the art.

A 2'-protected taxol 7-arylthiooxomethylether XVIII (R²⁸ = H) of Chart 10 may be alkylated to a 2'-protected taxol 7-arylthiooxoalkylether XVIII (R²⁸ = alkyl) by treatment with a strong base such as sodium hydride, lithium diethyl amide, lithium hexamethyl disilazide or similar strong base in an aprotic solvent such as THF, ether, dioxane or 1,2-dimethoxyethane followed by treatment with an alkylating agent such as an alkyl iodide, alkyl bromide, or alkyl alcohol sulfonate ester. Similarly, a 2'-protected taxol 7-arylthiodioxomethylether XIX (R²⁸ = H) of Chart 11 may be alkylated to a 2'-protected taxol 7-arylthiodioxoalkylether XIX (R²⁸ = alkyl) by treatment with a strong base such as sodium hydride, lithium diethyl amide, lithium hexamethyl disilazide or similar strong base in an aprotic solvent such as THF, ether, dioxane or 1,2-dimethoxyethane followed by treatment with an alkylating agent such as an alkyl iodide, alkyl bromide, or alkyl alcohol sulfonate ester.

A 2',3'-oxazolidine protected taxol 7-ether XXII of chart 12 can be prepared from a 2',3'-oxazolidine protected taxol analog V by reaction with a dialkyl sulfide and benzoyl peroxide (Medina, J. C.; Soloman, M.; Kyler, K S. Tetrahedron Lett. 1988, 29, 3773.) or by reaction with a chloroalkylthioalkyl ether and a tertiary base such as triethyl amine or diisopropyl ethyl amine in an aprotic solvent such as THF, dioxane, or methylene chloride. Similarly, a 2',3'-oxazolidine protected taxol 7-arythioalkyl ether XXIII of chart 12 can be prepared from a 2',3'-oxazolidine protected taxol analog V by reaction with a aryl alkyl sulfide and benzoyl peroxide (Medina, J. C.; Soloman, M.; Kyler, K S. Tetrahedron Lett. 1988, 29, 3773.) or by reaction with a chloroalkylthioaryl ether and a tertiary base such as triethyl amine or diisopropyl ethyl amine in an aprotic solvent such as THF, dioxane, or methylene chloride. A 2',3'-oxazolidine taxol 7-alkylthioalkyl XXII of Chart 13 may be oxidized to a sulfoxide XXIV by sodium metaperiodate and alcoholic solvent or by other method known in the art (Carrasco, M.; Jones, R. J.; Kamel S.; Rapoport_{,} H.; Truong, T. Org. Syn. 1991, 20 29; Johnson, C.; Keiser, L. Org. Syn. Coll Vol V, 1973, 791) or by other methods known in the art. Similarly a 2',3'-oxazolidine taxol 7-arythioalkyl ether XXXIII of Chart 14 may be oxidized to a sulfoxide by sodium metaperiodate and alcoholic solvent or by other method known in the art (Carrasco, M.; Jones, R. J.; Kamel S.; Rapoport, H.; Truong, T. Org. Syn. 1991, 20 29; Johnson, C.; Keiser, L. Org. Syn. Coll Vol V, 1973, 791) or by other methods known in the art.

A 2',3'-oxazolidine protected taxol 7-alkylthioalkyl ether XXII of Chart 13 may be oxidized to a sulfone XXV by meta chloroperbenzoic acid in aprotic solvents such as methylene chloride or THF or by hydrogen peroxide in aprotic or protic solvents such as methylene chloride, methanol, or ethanol (Carpino, L. A.; McAdams, L. V. Org. Syn. Coll. Vol. VI, 1988, 403; Paquette, L. A.; Carr, R. V. C. Org. Syn, 1985, 64, 157) or by other methods known in the art. Similarly, a 2',3'-oxazolidine protected taxol 7-arylthioalkyl XXIII of Chart 14 may be oxidized to a sulfone XXVII by meta chloroperbenzoic acid in aprotic solvents such as methylene chloride or THF or by hydrogen peroxide in aprotic or protic solvents such as methylene chloride, methanol, or ethanol (Carpino, L. A.; McAdams, L. V. Org. Syn. Coll. Vol. VI, 1988, 403; Paquette, L. A.; Carr, R. V. C. Org. Syn, 1985, 64, 157) or by other methods known in the art.

A 2',3'-oxazolidine protected taxol 7-arylthiooxomethylether XXVI (R²⁸ = H) of Chart 15 may be alkylated to a 2',3'-oxazolidine protected taxol 7-arylthio-oxoalkylether XXVI (R²⁸ = alkyl) by treatment with a strong base such as sodium hydride, lithium diethyl amide, lithium hexamethyl disilazide or similar strong base in an aprotic solvent such as THF, ether, dioxane or 1,2-dimethoxyethane followed by treatment with an alkylating agent such as an alkyl iodide, alkyl bromide, or alkyl alcohol sulfonate ester. Similarly, a 2',3'-oxazolidine protected taxol 7-arylthiodioxomethylether XXVII R²⁸ = H) of Chart 16 may be alkylated to a 2',3'-oxazolidine protected taxol 7-arylthiodioxoalkylether XXVII (R²⁸ = alkyl) by treatment with a strong base such as sodium hydride, lithium diethyl amide, lithium hexamethyl disilazide or similar strong base in an aprotic solvent such as THF, ether, dioxane or 1,2-dimethoxyethane followed by treatment with an alkylating agent such as an alkyl iodide, alkyl bromide, or alkyl alcohol sulfonate ester.

A 2'-protected taxol 7-alkylthioalkyl XIV (Chart 17), 2'-protected taxol 7-arythioalkyl ether XV (Chart 18), 7-methylthiooxomethylether XVI (Chart 8, R²⁸ = H), 7-alkylthiooxoalkylether XVI (Chart 8, R²⁸ = alkyl), 7-methylthiodioxo-3 methylether XVII (Chart 8, R²⁸ = H), 7-alkylthiodioxoalkylether XVII (R²⁸ = alkyl), 7-arylthiooxomethylether XVIII (Chart 9, R²⁸ = H), 7-arylthiooxoalkylether XVIII (Chart 10, R²⁸ = alkyl), 7-arylthiodioxomethylether XIX (Chart 9, R²⁸ = H), or a 7-arylthiodioxodkylether XIX (Chart 11, R²⁸ = alkyl) may be desulfurized with Raney Ni (Pettit, G. R.; Van Tamelen, E. E. Organic Reactions, 1962, 12, 356) to the respective 2-protected taxol ethers XX. The 2'-protected taxol ethers XX may be deprotected to the taxol 7 ether analogs as described earlier for the conversion of XII to XI (Chart 5).

A 2',3'-oxazolidine protected taxol 7-alkylthioalkyl XXII (Chart 19), 2',3'-oxazolidine protected taxol 7-arythioalkyl ether XXIII (Chart 20), 2',3'-oxazolidine protected taxol 7-methylthiooxomethylether XXIV (Chart 13, R²⁸ = H), 2',3'-oxazolidine protected taxol 7-alkylthiooxoalkylether XXIV (Chart 13, R²⁸ = alkyl), 2',3'-oxazolidine protected taxol 7-methylthiodioxomethylether XXV (Chart 13, R²⁸ = H), 2',3'-oxazolidine protected taxol 7-alkylthiodioxoalkylether XXV (Chart 13, R²⁸ = alkyl), 2',3'-oxazolidine protected taxol 7-arylthiooxomethylether XXVI (Chart 14, R²⁸ = H), 2',3'-oxazolidine protected taxol 7-arylthiooxoalkylether XXVI (Chart 15, R²⁸ = alkyl), 2',3'-oxazolidine protected taxol 7-arylthiodioxomethylether XXVII (Chart 14, R²⁸ = H), or a 2',3'-oxazolidine protected taxol 7-arylthiodioxoalkylether XXVII (Chart 16, R²⁸ = alkyl) may be desulfurized with Raney Ni (Pettit, G. R.; Van Tamelen, E. E. Organic Reactions, 1962, 12, 356) to the respective 2',3'-oxazolidine protected taxol ethers XXVIII. The 2',3'-oxazolidine protected taxol ethers XXVIII may be deprotected to a 7-ether analog XXI as described earlier for the conversion of XII to XI (Chart 6).

### Example 1 Preparation of 13-(N-Boc-2'-TES-β-phenyl isoserinyl)-baccatin III (2)

13-(N-Boc-β-phenyl isoserinyl)-baccatin III (1, 1.36 g, 1.6 mmol) is dissolved in dry pyridine (16 mL) and the solution cooled to 0°C. To this is added chlorotriethylsilane (0.3 mL, 1.76 mmol). The reaction is allowed to stir at 0°C for 2 hrs. After stirring overnight at room temperature TLC still shows the presence of some starting material. The reaction is recooled to 0°C and chlorotriethylsilane (0. 3 mL, 1.76 mmol) is added again. After each of three more two h periods additions of chlorotriethylsilane (0.2 mL, 0.25 mL, and 0.20 mL) is repeated. The reaction is then warmed to room temperature and stirred overnight. TLC then shows no starting material remaining. The solution is extracted twice with saturated CuSO₄. The aqueous layers are re-extracted with ethyl acetate. The organic layers are combined, filtered through sodium sulfate, and concentrated in vacuo. The residue is chromatographed over a column of silica gel (150 g) packed in 1:9 EtOAc: Hexane. The column is eluted with (1:9) EtOAc: Hexane (300 mL), (1:4) EtOAc: Hexane (1 L), (1:3) EtOAc: Hexane (500 mL), and (1:1) EtOAc: Hexane (1 L) collecting 70 mL fractions. 13-(N-Boc-2'-TES-β-phenyl isoserinyl)-baccatin III (2, 1.24 g 80% yield) as a white solid is found on evaporation of fractions 53-69.

Proton NMR (CDCl₃; TMS): δ 0.39 (m, 6H); 0.78 (m, 9H); 1.90 (m, 4H); 2.25 (s, 3H); 2.39 (m); 2.53 (s); 2.50-2.63 (m); 3.83 (d, 1H); 4.20 (d, 1H); 4.34 (d, 1H); 4.47 (m, 1H); 4.55 s, 1H); 5.00 (d, 1H); 5.28 (m, 1H); 5.49 (m, 1H); 5.68 (d, 1H); 6.30 (m, 2H); 7.28 (m); 7.37 (m, 2H); 7.50 (m, 2H); 7.61 (m, 1H); 8.12 (d, 2H)
Mass Spec (FAB-High Res.) Theory: 964.4514 Found: 964.4528

### Example 2 Preparation of 7-(O-ethoxymethyl)-13-(N-Boc-2'-TES-β-phenyl isoserinyl)-baccatin III (3)

13-(N-Boc-2'-TES-β-phenyl isoserinyl)-baccatin III (2, 100mg, 0.104mM) is stirred at RT under nitrogen in methylene chloride (1 mL). To the solution are added chloromethyl ethyl ether (58 mL, 0.624 mM) and diisopropylethyl amine (109 mL, 0.624 mM). After 2 days the reaction is found to be complete by TLC. The reaction is then partitioned between methylene chloride-water. The layers are separated and the water layer re-extracted with methylene chloride. The organic layers are dried over sodium sulfate, combined and evaporated under vacuum. The crude product is chromatographed over silica gel (10 g), eluting with (20-80) acetone-hexane. Fractions of 3 mL are collected, analyzing them by TLC. The product is found in fractions 13-28, which upon combining and evaporating under vacuum leave 7-(O-ethoxymethyl)-13-(N-Boc-2'-TES-β-phenyl isoserinyl)-baccatin III (3, 104 mg, 98% yield) as a white solid.

TLC (silica gel): (30-70) acetone-hexane; R_{f}: 0.48

Proton NMR (CDCl₃; TMS): δ 0.3-0.50 (m, 6H); 0.74-0.84 (t, 9H); 1.10-1.20 (t, 3H); 1.21 (s, 3H); 1.26 (s, 3H); 1.33 (s, 9H); 1.76 (s, 3H); 1.96 (s, 3H); 2.21 (s, 3H); 2.33-2.46 (m, 1H); 2.52 (s, 3H); 2.78-2.94 (m, 1H); 3.35-3.48 (m, 1H); 3.60-3.74 (m, 1H); 3.85-3.94 (d, 1H); 4.10-4.20 (m, 1H); 4.15-4.23 (d, 1H); 4.30-4.37 (d, 1H); 4.56 (s, 1H); 4.75 (s, 2H); 4.92-5.00 (d, 1H); 5.20-5.33 (bd, 1H); 5.43-5.55 (bd, 1H); 5.64-5.73 (d, 1H); 6.20-6.31 (t, 1H); 6.37 (s, 1H); 7.14-7.34 (m, 3H); 7.34-7.43 (t, 2H); 7.43-7.55 (t, 2H); 7.55-7.64 (t, 1H); 8.08-8.16 (d, 2H).

### Example 3 Preparation of 7-(O-ethoxymethyl)-13-(N-Boc-β-phenyl isoserinyl)-baccatin III (4)

7-(O-ethozymethyl)-13-(N-Boc-2'-TES-β-phenyl isoserinyl)-baccatin III (3, 104 mg, 0.102mM) is stirred at RT under nitrogen in 0.1N HCl in MeOH (1 mL, prepared from 71 mL acetyl chloride and 10 mL MeOH). TLC after 15 min shows no starting material to be present. The reaction is then partitioned between ethyl acetate-5% sodium bicarbonate. The layers are separated and the water layer reextracted with ethyl acetate. The organic layers are dried over sodium sulfate, combined and evaporated under vacuum. The crude product is chromatographed over 10g silica gel, eluting with (30-70) acetone-hexane. Fractions of 3 mL are collected, analyzing them by TLC. The product is found in fractions 17-29, which upon combining and evaporating under vacuum leave 7-(O-ethoxymethyl)-13-(N-Boc-β-phenyl isoserinyl)-baccatin III (4, 71mg, 77% yield) as a white solid.

TLC (silica gel): 40-60 acetone-hexane; R_{f}: 0.64

Proton NMR (CDCl₃; TMS): δ 1.10-1.20 (t, 3H); 1.21 (s, 3H); 1.22 (s, 3H); 1.35 (s, 9H); 1.75 (s, 3H); 1.88 (s, 3H); 2.21 (s, 3H); 2.36 (s, 3H); 2.76-2.90 (m, 1H); 3.33-3.49 (m, 1H); 3.55 (bs, 1H); 3.59-3.73 (m, 1H); 3.80-3.90 (d, 1H); 4.03-4.22 (m, 2H); 4.24-4.34 (d, 1H); 4.61 (bs, 1H); 4.73 (s, 2H); 4.86-4.96 (d, 1H); 5.19-5.31 (bd, 1H); 5.41-5.54 (bd, 1H); 5.60-5.70 (d, 1H); 6.09-6.23 (t, 1H); 6.34 (s, 1H); 7.25-7.43 (m, 5H); 7.43-7.54 (t, 2H); 7.54-7.65 (t, 1H); 8.00-8.13 (d, 2H).

Mass Spec (FAB, *m/z)* (M+H)⁺ measured at 908.4089; theory for C₄₈H₆₂N₁O₁₆ is 908.4068; 908, 627, 585, 105, 59, 57.

### Example 4 Preparation of 7-(O-methoxyethoxymethyl)-13-(N-Boc-2'-TES-β-phenyl isoserinyl)-baccatin III (5)

13-(N-Boc-2'-TES-β-phenyl isoserinyl)-baccatin III (2, 100mg, 0.104mM) is stirred at RT under nitrogen in methylene chloride (1 mL) and the solution treated with MEM chloride (71 mL, 0.624 mM) and diisopropylethyl amine (109 mL, 0.624 mM). The reaction is allowed to react for 2 days, at which point it is still incomplete. Additional MEM chloride (71 mL, 0.624 mM) and disopropylethyl amine (109 mL, 0.624 mM) is added. The reaction is allowed to react for 3 more days, when reaction is found to be complete. The reaction is partitioned between methylene chloride-water. The layers are separated and the water layer re-extracted with methylene chloride. The organic layers are dried over sodium sulfate, combined and evaporated under vacuum. The crude product is chromatographed over silica gel (11 g), eluting with 20-80 acetone-hexane. Fractions of 3 mL are collected, analyzing them by TLC. 7-(O-methoxyethoxymethyl)-13-(N-Boc-2'-TES-β-phenyl isoserinyl)-baccatin III (5, 101 mg, 93% yield) as a white solid is found in fractions 17-37 after combining and evaporating under vacuum.

TLC (silica gel): (30-70) acetone-hexane; R_{f}: 0.44

Proton NMR (CDCl₃; TMS): δ 0.3-0.51 (m, 6H); 0.75-0.85 (t, 9H); 1.21 (s, 3H); 1.26 (s, 3H); 1.33 (s, 9H); 1.76 (s, 3H); 1.95 (s, 3H); 2.20 (s, 3H); 2.31-2.45 (m, 1H); 2.52 (s, 3H); 2.80-2.95 (m, 1H); 3.35 (s, 3H); 3.47-3.60 (m, 1H); 3.51 (s, 2H); 3.70-3.82 (m, 1H); 3.86-3.95 (d, 1H); 4.13-4.24 (m, 2H); 4.29-4.37 (d, 1H); 4.55 (s, 1H); 4.75-4.87 (q, 2H); 4.92-5.00 (d, 1H); 5.20-5.34 (bd, 1H); 5.44-5.56 (bd, 1H); 5.65-5.73 (d, 1H); 6.19-6.33 (t, 1H); 6.36 (s, 1H); 7.23-7.43 (m, 5H); 7.44-7.54 (t, 2H); 7.54-7.64 (t, 1H); 8.06-8.16 (d, 2H).

### Example 5 Preparation of 7-(O-methoyethoxymethyl)-13-(N-Boc-β-phenyl isoserinyl)-baccatin III (6)

7-(O-methoxyethoxymethyl)-13-(N-Boc-2'-TES-β-phenyl isoserinyl)-baccatin III (5, 101 mg, 0.096mM) is stirred at RT under nitrogen in (80-20) HOAc-water (2 mL). The reaction is found to be complete by TLC in 5 hours. The reaction mixture is then freeze-dried. The crude product is chromatographed over silica gel (10g), eluting with (30-70) acetone-hexane. Fractions of 3 mL are collected, analyzing them by TLC. 7-(O-methoxyethoxymethyl)-13-(N-Boc-β-phenyl isoserinyl)-baccatin III (6, 90 mg, 100 % yield) as a white solid is found in fractions 23-48 upon combining and evaporating under vacuum.

TLC (silica gel): (30-70) acetone-hexane; R_{f}: 0.27

Proton NMR (CDCl₃; TMS): δ 1.20 (s, 3H); 1.24 (s, 3H); 1.35 (s, 9H); 1.75 (s, 3H); 1.87 (s, 3H); 2.21 (s, 3H); 2.36 (s, 3H); 2.81-2.96 (m, 1H); 3.34 (s, 3H); 3.50 (s, 2H); 3.46-3.60 (m, 1H); 3.66 (bs, 1H); 3.72-3.82 (m, 1H); 3.82-3.91 (d, 1H); 4.08-4.23 (m, 2H); 4.26-4.36 (d, 1H); 4.63 (bs, 1H); 4.73-4.85 (m, 2H); 4.88-4.98 (d, 1H); 5.21-5.34 (bd, 1H); 5.52-5.62 (bd, 1H); 5.62-5.71 (d, 1H); 6.14-6.26 (t, 1H); 6.33 (s, 1H); 7.28-7.43 (m, 5H); 7.43-7.55 (t, 2H); 7.56-7.68 (t, 1H); 8.02-8.16 (d, 2H).

Mass Spec (FAB, *m/z)* (M+H)⁺ measured at 938.4166; theory for C₄₉H₆₄N₁O₁₇ is 938.4174; 938, 882, 878, 657, 105, 89, 59, 57.

### Example 6 Preparation of 7-(O-methoxymethyl)-13-(N-Boc-2'-TES-β-phenyl isoserinyl)-baccatin III (7)

13-(N-Boc-2'-TES-β-phenyl isoserinyl)-baccatin III (2, 100 mg, 0.104mM) is stirred at RT under nitrogen in dry THF (1 mL) and the solution treated with chloromethyl methyl ether (47 mL, 0.624 mM) and diisopropylethyl amine (109 mL, 0.624 mM). The reaction is allowed to stand at RT overnight, during which the THF evaporates to about one half volume and a precipitate forms. The precipitate is redissolved by the addition of methylene chloride (0.5 mL). The reaction is found to be incomplete at this point by TLC, so it is concentrated to one half volume and treated with chloromethyl methyl ether (47 mL, 0.624 mM) and diisopropylethyl amine (109 mL, 0.624 mM). The reaction is then allowed to proceed an additional 4 days, when reaction is complete. The reaction is partitioned between methylene chloride-water. The layers are separated and the water layer re-extracted with methylene chloride. The organic layers are dried over sodium sulfate, combined and evaporated under vacuum. The crude product is chromatographed over silica gel (10 g), eluting with (20-80) acetone-hexane. Fractions of 3 mL are collected, analyzing them by TLC. The product is found in fractions 15-26, which upon combining and evaporating under vacuum leave 7-(O-methoxymethyl)-13-(N-Boc-2'-TES-β-phenyl isoserinyl)-baccatin III (7, 76 mg, 72 % yield) as a white solid.

TLC (silica gel): (30-70) acetone-hexane; R_{f}: 0.44

Proton NMR (CDCl₃; TMS): δ 0.3-0.52 (m, 6H); 0.74-0.85 (t, 9H); 1.22 (s, 3H); 1.26 (s, 3H); 1.33 (s, 9H); 1.77 (s, 3H); 1.97 (s, 3H); 2.21 (s, 3H); 2.32-2.47 (m, 1H); 2.53 (s, 3H); 2.75-2.90 (m, 1H); 3.30 (s, 3H); 3.86-3.94 (d, 1H); 4.10-4.20 (m, 1H); 4.15-4.24 (d, 1H); 4.30-4.36 (d, 1H); 4.56 (s, 1H); 4.63-4.70 (d, 1H); 4.70-4.79 (d, 1H); 4.92-5.02 (d, 1H); 5.22-5.34 (bd, 1H); 5.44-5.56 (bd, 1H); 5.67-5.74 (d, 1H); 6.20-6.30 (t, 1H); 6.39 (s, 1H); 7.24-7.34 (m, 3H); 7.34-7.44 (t, 2H); 7.45-7.55 (t, 2H); 7.56-7.65 (t, 1H); 8.07-8.16 (d, 2H).

### Example 7 Preparation of 7-(O-methoxymethyl)-13-(N-Boc-2'-β-phenyl isoserinyl)-baccatin III (8)

7-(O-Methoxymethy)-13-(N-Boc-2'-TES-β-phenyl isoserinyl)-baccatin III (7, 76 mg, 0.075mM) is dissolved at RT under nitrogen in (80-20) HOAc-water (2 mL). After a few minutes, a precipitate forms. The precepitate is redissolved by the addition of THF (2 ml). The reaction is allowed to stand for 1.5 days, some of the THF evaporating. At this point the reaction if found to be complete by TLC. The reaction mixture is then freeze-dried. The crude product is chromatographed over silica gel (10 g) silica gel, eluting with (30-70) acetone-hexane. Fractions of 3 mL are collected, analyzing them by TLC. The product is found in fractions 15-29, which upon combining and evaporating under vacuum leave 7-(O-methoxymethyl)-13-(N-Boc-2'-β-phenyl isoserinyl)-baccatin III (8, 63 mg, 94 % yield) as a white solid.

TLC (silica gel): (30-70) acetone-hexane; R_{f}: 0.28

Proton NMR (CDCl₃; TMS): δ 1.21 (s, 3H); 1.23 (s, 3H); 1.35 (s, 9H); 1.76 (s, 3H); 1.90 (s, 3H); 2.21 (s, 3H); 2.37 (s, 3H); 2.72-2.87 (m, 1H); 3.29 (s, 3H); 3.56 (bs, 1H); 3.80-3.90 (d, 1H); 4.04-4.20 (m, 2H); 4.25-4.34 (d, 1H); 4.54-4.68 (d, 1H); 4.58 (bs, 1H); 4.68-4.75 (d, 1H); 4.87-4.96 (d, 1H); 5.20-5.31 (bd, 1H); 5.42-5.56 (bd, 1H); 5.62-5.70 (d, 1H); 6.10-6.23 (t, 1H); 6.36 (s, 1H); 7.24-7.44 (m, 5H); 7.44-7.54 (t, 2H); 7.54-7.66 (t, 1H); 8.01-8.13 (d, 2H).

Mass Spec (FAB, *m/z)* (M+H)⁺ measured at 894.3943; theory for C4₇H₆₀N₁O₁₆ is 894.3912; 894, 838, 613, 571, 553, 105, 57.

### Example 8 Preparation of 7-Benzyloxymethyl-13-(N-Boc-2'-TES-β-phenyl isoserinyl)-baccatin III (9)

13-(N-Boc-2'-TES-β-phenyl isoserinyl)-baccatin III (2, 100mg, 0.104mM) is stirred at RT under nitrogen in methylene chloride (1 ML) and the solution treated with benzyl chloromethyl ether (104 mL, 0.624 mM, 80% pure) and diisopropylethyl amine (109 mL, 0.624 mM). The reaction is allowed to stand for 2 days, when it is complete as found by TLC. The reaction is then partitioned between methylene chloride-water. The layers are separated and the water layer re-extracted with methylene chloride. The organic layers are dried over sodium sulfate, combined and evaporated under vacuum. The crude product is chromatographed over silica gel (15 g), eluting with (20-80) acetone-hexane. Fractions of 3 mL are collected, analyzing them by TLC. The product is found in fractions 19-33, which upon combining and evaporating under vacuum leave 7-(O-benzyloxymethyl)-13-(N-Boc-2'-TES-β-phenyl isoserinyl)-baccatin III (9, 92 mg, 81 % yield) as a white solid.

TLC (silica gel): (30-70) acetone-hexane; R_{f}: 0.50

Proton NMR (CDCl₃; TMS): δ 0.3-0.53 (m, 6H); 0.70-0.85 (t, 9H); 1.22 (s, 3H); 1.26 (s, 3H); 1.33 (s, 9H); 1.79 (s, 3H); 1.95 (s, 3H); 2.20 (s, 3H); 2.33-2.46 (m, 1H); 2.51 (s, 3H); 2.83-2.98 (m, 1H); 3.88-3.96 (d, 1H); 4.15-4.29 (m, 2H); 4.30-4.38 (d, 1H); 4.40-4.49 (d, 1H); 4.55 (s, 1H); 4.64-4.74 (d, 1H); 4.86 (s, 2H); 4.90-5.00 (d, 1H); 5.21-5.34 (bd, 1H); 5.44-5.58 (bd, 1H); 5.67-5.76 (d, 1H); 6.21-6.32 (t, 1H); 6.40 (s, 1H); 7.20-7.44 (m, 5H); 7.44-7.54 (t, 2H); 7.54-7.64 (t, 1H); 8.06-8.15 (d, 2H).

### Example 9 Preparation of 7-(O-benzyloxymethyl)-13-(N-Boc-β-phenyl isoserinyl)-baccatin III (10)

7-(O-benzyloxymethyl)-13-(N-Boc-2'-TES-β-phenyl isoserinyl)baccatin III (9, 92 mg, 0.085mM) is dissolved at RT under nitrogen in (80-20) HOAc-water (2 mL). A precipitate forms after a few minutes, and this is redissolved by the addition of THF (2 mL). The reaction is allowed to stand at RT for 1.5 days and at 45° C for 3 days. The reaction is found to be nearly complete at this point by TLC. The reaction mixture is then freeze-dried. The crude product is chromatographed over silica gel (10 g), eluting with (30-70) acetone-hexane. Fractions of 3 mL are collected, analyzing them by TLC. The product is found in fractions 15-26, which upon combining and evaporating under vacuum leave 7-(O-benzyloxymethyl)-13-(N-Boc-β-phenyl isoserinyl)-baccatin III (10, 70 mg, 85 % yield) as a white solid.

TLC (silica gel): (30-70) acetone-hexane; R_{f}: 0.30

Proton NMR (CDCl₃; TMS): δ 1.22 (s, 3H); 1.23 (s, 3H); 1.35 (s, 9H); 1.78 (s, 3H); 1.87 (s, 3H); 1.95-2.10 (m, 1H); 2.21 (s, 3H); 2.36 (s, 3H); 2.82-2.96 (m, 1H); 3.48-3.58 (bd, 1H); 3.82-3.92 (d, 1H); 4.13-4.24 (m, 2H); 4.28-4.36 (d, 1H); 4.39-4.48 (d, 1H); 4.64 (bs, 1H); 4.65-4.72 (d, 1H); 4.81-4.90 (m, 2H); 4.90-4.98 (d, 1H); 5.22-5.32 (bd, 1H); 5.42-5.54 (bd, 1H); 5.62-5.71 (d, 1H); 6.12-6.24 (t, 1H); 6.37 (s, 1H); 7.21-7.43 (m, 10H); 7.43-7.54 (t, 2H); 7.55-7.65 (t, 1H); 8.04-8.15 (d, 2H).

Mass Spec (FAB, *m/z)* (M+H)⁺ measured at 970.4242; theory for C₅₃H₆₄N₁O₁₆ is 970.4225; 970, 914, 689, 647, 105, 57, 43.

### Example 9a Preparation of 7-TES-Baccatin III-(4S,5R)-N-t-buylurea-2-(2,4 dimethoxyphenyl)4-phenyl-5-oxazolidinecarboxylic acid ester (11)

Crude (4S,5R)-N-t-butyl urea-2-(2,4 dimethoxyphenyl)-4-phenyl-5-oxazolidinecarboxylic acid methyl ester (475mg,1.07mM) is dissolved in methanol (10mL), water (0.4mL) and K₂CO₃ (190mg) is added. After stirring overnight TLC shows only a spot at the origin. The solution is concentrated in vacuo and the residue partitioned between CH₂Cl₂ and 5% NaHSO₄ solution. The layers are separated and the aqueous layer extracted with EtOAc. The combined organic layers are filtered through anhydrous sodium sulfate and concentrated in vacuo leaving (4S,5R)-N-t-butyl urea-2-(2,4 dimethoxyphenyl)-4-phenyl-5-oxazolidine-carboxylic acid. 7-TES-baccatin III (500mg, 0.71 mM) is dissolved in toluene (7ml). All of the (4S,5R)-N-t-butyl urea-2-(2,4 dimethoxyphenyl)-4-phenyl-5-oxazolidine-carboxylic acid from above is added in a solution of CH₂Cl₂. The solution is heated to 80° C driving off the CH₂Cl₂ after which DCC (240mg, 1.15 mM) and DMAP (45mg, 0.36mM) are added. After 0.5 hr TLC shows little starting material so the slurry is cooled. The reaction is filtered through Celite and the filtrate concentrated in vacuo and chromatographed over a column of silica gel (80 g) in (1:3) EtOAc:hexane. The column is eluted with (1:3) EtOAc:hexane (200ml) and (1:2) EtOAc:hexane (1L) collecting 40 ml fractions. 7-TES-Baccatin III-(4S,5R)-N-t-buylurea-2-(2,4 dimethoxyphenyl)-4-phenyl-5-oxazolidinecarboxylic acid ester (11, 906 mg) is found in fractions 28-47.

Mass Spec: Theory 1111.5198 Found 1111.5189

Proton NMR (CDCl₃; TMS): δ 0.59 (m, 6H); 0.92 (m,9H); 1.20 (m); 1.92 (s, 3H); 2.13 (s, 3H); 2.19 (s, 3H); 2.50 (m, 1H); 3.84 (m); 3.92 (s, 3H); 4.13 (d, 1H); 4.26 (d, 1H); 4.48 (m, 2H); 4.88 (d, 1H); 4.95 (d, 1H); 5.55 (d, 1H); 5.69 (d, 1H); 6.50 (m); 6.72 (m); 7.27-7.64 (m, 10H); 8.06 (d, 2H)

### Example 10 Preparation of baccatin III-13-(4S,5R)-N-(t-butylaminocarbonyl)-2-(2,4- dimethoxyphenyl)4-phenyl-5-oxazolidinecarboxylic acid ester (12) and 7-epi-baccatin III-13-(4S,5R)N-(t-butylaminocarbonyl)-2-(2,4-dimethoxyphenyl)-4-phenyl-5-oxazolidinecarboxylic acid ester

Baccatin III-13-(4S,5R)-N-(t-butylaminocarbonyl)-2-(2,4-dimethoxyphenyl)-4-phenyl-5-oxazolidinecarboxylic acid ester (11, 198 mg, 0.178mM) is stirred at RT under nitrogen in dry THF (3 mL). To this solution is added tetra-n-butyl ammonium fluoride (56 mg, 0.178 mM). The reaction is followed by TLC which indicates the starting material is consumed in 45 minutes, giving two more polar products. The reaction mixture is then partitioned between ethyl acetate-5% sodium bicarbonate-brine. The aqueous layer is re-extracted with ethyl acetate. The organic layers are combined, dried over sodium sulfate and evaporated under vacuum. The crude product is chromatographed over silica gel (20 g), eluting with (50-50) ethyl acetate-hexane. Mixed fractions are rechromatographed. Fractions of 3 mL are collected, analyzing them by TLC. Baccatin III-13-(4S,SR)-N-(t-butylaminocarbonyl)-2-(2,4-dimethoxyphenyl)-4-phenyl-5-oxazolidinecarboxylic acid ester (12, 61 mg, 34 % yield) is found as a white solid on evaporation of fractions 42-56 and 7-epi-baccatin III-13-(4S,5R)-N-(t-butylaminocarbonyl)-2-(2,4-dimethoxyphenyl)-4-phenyl-5-oxazolidinecarboxylic acid ester (68 mg, 38 % yield) is found as a white solid on evaporation of fractions 24-31.

Data for 7-epi-baccatin III-13-(4S,5R)-N-(t-butylaminocarbonyl)-2-(2,4-dimethoxyphenyl)-4-phenyl-5-oxazolidinecarboxylic acid ester:

TLC (silica gel): (50-50) ethyl acetate-hexane; R_{f}; 0.39

Proton NMR (CDCl₃; TMS): δ 1.16 (s, 12H); 1.23 (s, 3H); 1.65 (s, 3H); 2.00 (s, 3H); 2.04 (s, 3H); 2.22 (s, 3H); 3.65-3.74 (bd, 1H); 3.86 (s, 3H); 3.90 (s, 3H);4.34 (s, 2H); 4.57 (s 1H); 4.80-4.92 (m, 2H); 4.94-4.98 (d, 1H); 5.58-5.61 (d, 1H); 5.73-5.78 (d, 1H); 6.23-6.33 (t, 1H); 6.49-6.56 (d, 1H); 6.53 (s, 1H); 6.70 (s, 1H); 6.86 (s, 1H); 7.26-7.58 (m, 8H); 7.58-7.66 (t, 1H); 8.01-8.09 (d, 2H).

Data for baccatin III-13-(4S,5R)-N-(t-butylaminocarbonyl)-2-(2,4-dimethoxyphenyl)-4-phenyl-5-oxazolidinecarboxylic acid ester (12):

TLC (silica gel): (50-50) ethyl acetate-hexane; Rf: 0.24

Proton NMR (CDCl₃; TMS): δ 1.16 (s, 12H); 1.28 (s, 3H); 1.66 (s, 3H); 1.90 (s, 3H); 1.98 (s, 3H); 2.26 (s, 3H); 2.43-2.55 (m, 2H); 3.73-3.81 (d, 1H); 3.84 (s, 3H); 3.91(s, 3H); 4.11-4.16 (d, 1H); 4.21-4.27 (d, 1H); 4.36-4.47 (m, 1H); 4.50 (s 1H); 4.82-4.92 (bd, 1H); 4.92-4.96 (d, 1H); 5.50-5.55 (d, 1H); 5.61-5.68 (d, 1H); 6.25-6.37 (m, 2H); 6.47-6.55 (m, 2H); 6.71(s, 1H); 7.23-7.57 (m, 8H); 7.57-7.64 (t, 1H); 8.00-8.07 (d, 2H).

### Preparation of baccatin III-13-(4S,5R)-N-(t-butylaminocarbonyl)-2-(2,4-dimethoxyphenyl)-4-phenyl-5-oxazolidinecarboxylic acid ester (12)

Baccatin III-13-(4S,5R)-N-(t-butylaminocarbonyl)-2-(2,4-dimethoxyphenyl)-4-phenyl-5-oxazolidinecarboxylic acid ester (11, 3.41 g, 3.07 mM) is stirred at RT under nitrogen in dry acetonitrile (30 mL) and the solution treated with triethyl amine trihydrofluoride (5 mL), resulting in a thick slurry which dissolves over a 7 h period. The reaction is followed by TLC and found to be essentially finished in 7.5 hr. At this point the reaction is diluted with ethyl acetate and washed with 5% sodium bicarbonate, 5% sodium bisulfate and brine. The organic layer is dried over sodium sulfate and evaporated under vacuum. The crude product is chromatographed over silica gel (300 g), eluting with (25-75, 1.5 L), (30-70, 1 L), and (40-60, 2 L) acetone-hexane. Fractions of 40 mL are collected, analyzing them by TLC. Fractions 74-92 were combined and evaporated under vacuum to give baccatin III-13-(4S,5R)-N-(t-butylaminocarbonyl)-2-(2,4-dimethoxyphenyl)-4-phenyl-5-oxazolidinecarboxylic acid ester (12, 2.26 g, 74% yield) as a white solid.

TLC (silica gel): (50-50) ethyl acetate-hexane; R_{f}: 0.24

Proton NMR (CDCl₃; TMS): δ 1.16(s, 12H); 1.28 (s, 3H); 1.66 (s, 3H); 1.90 (s, 3H); 1.98 (s, 3H); 2.26 (s, 3H); 2.43-2.55 (m, 2H); 3.73-3.81 (d, 1H); 3.84 (s, 3H); 3.91 (s, 3H); 4.11-4.16 (d, 1H); 4.21-4.27 (d, 1H); 4.36-4.47 (m, 1H); 4.50 (s 1H); 4.82-4.92 (bd, 1H); 4.92-4.96 (d, 1H); 5.50-5.55 (d, 1H); 5.61-5.68 (d, 1H); 6.25-6.37 (m, 2H); 6.47-6.55 (m, 2H); 6.71 (s, 1H); 7.23-7.57 (m, 8H); 7.57-7.64 (t, 1H); 8.00-8.07 (d, 2H).

### Example 11 Preparation of 7-(O-ethoxymethyl)-baccatin III-13-(4S,5R)-N-(t-butylaminocarbonyl)-2-(2,4-dimethoxyphenyl)-4-phenyl-5-oxazolidinecarboxylic acid ester (13)

Baccatin III-13-(4S,5R)-N-(t-butylaminocarbonyl)-2-(2,4-dimethoxyphenyl-4-phenyl-5-oxazolidinecarboxylic acid ester (12, 61 mg, 0.061 mM) is stirred at RT under nitrogen in methylene chloride (1 mL). To this solution is added chloromethyl ethyl ether (28 mL, 0.306 mM) and diisopropyl ethyl amine (53 mL, 0.306 mM). The reaction is followed by TLC which shows the reaction to be incomplete in two days. At this time chloromethyl ethyl ether (28 mL, 0.306 mM) and diisopropyl ethyl amine (53 mL, 0.306 mM) are again added. After 5 days, the reaction is partitioned between methylene chloride-water. The aqueous layer is re-extracted with methylene chloride. The organic layers are combined, dried over sodium sulfate and evaporated. The crude product is chromatographed over silica gel (10 g), eluting with a gradient of (20-80) to (30-70) acetone-hexane. Fractions of 3 mL are collected, analyzing them by TLC. Fractions 35-57 are combined and evaporated under vacuum to give 7-(O-ethoxymethyl)-baccatin III-13-(4S,SR)-N-(t-butylaminocarbonyl)-2-(2,4-dimethoxyphenyl)4-phenyl-5-oxazolidinecarboxylic acid ester (13, 44 mg, 69% yield) as a white solid.

TLC (silica gel): (30-70) acetone-hexane; R_{f}: 0.67

Proton NMR (CDCl₃; TMS): δ 1.08-1.18 (t, 3H); 1.18 (s, 9H); 1.21 (s, 3H); 1.26 (s, 3H); 1.74 (s, 3H); 1.98 (s, 3H); 2.06 (s, 3H); 2.26 (s, 3H); 2.74-2.88 (m, 1H); 3.38-3.48 (m, 1H); 3.62-3.73 (m, 1H); 3.82-3.94 (d, 1H); 3.84 (s, 3H);3.92 (s, 3H);4.10-4.21 (m 2H); 4.21-4.30 (d, 1H); 4.58 (s 1H); 4.74 (s, 2H); 4.82-4.92 (d, 1H); 4.94-4.98 (d, 1H); 5.55-5.59 (d, 1H); 5.62-5.70 (d, 1H); 6.26-6.37 (t, 1H); 6.37 (s, 1H); 6.47-6.56 (m, 2H); 6.75 (s, 1H); 7.25-7.66 (m, 9H); 8.02-8.11 (d, 2H).

### Example 12 Preparation of 7-O-ethoxymethyl)-13-(N-(t-butylaminocarbonyl)-β-phenyl isoserinyl)-baccatin III (14)

7-(O-ethoxymethyl)-baccatin III-13-(4S,5R)-N-(t-butylaminocarbonyl)-2-(2,4-dimethoxyphenyl)-4-phenyl-5-oxazolidinecarboxylic acid ester (13, 44 mg, 0.042 mM) is stirred at RT under nitrogen in (80-20) acetic acid-water (2 mL). The reaction is followed by TLC and found to be complete in 4 hours. The reaction is then freeze-dried. The crude product is purified by chromatography over silica gel (10 g), eluting with (30-70) acetone-hexane. Fractions of 3 mL are collected, analyzing them by TLC. The product is found in fractions 17-32, which are combined and evaporated under vacuum to give 7-(O-ethoxymethyl)-13-(N-(t-butylaminocarbonyl)-β-phenyl isoserinyl)-baccatin III (14, 26 mg 68 % yield) as a white solid.

TLC (silica gel): (30-70) acetone-hexane; R_{f}; 0.39

Proton NMR (CDCl₃; TMS): δ 1.09-1.17 (t, 3H); 1.20 (s, 3H); 1.21 (s, 3H); 1.23 (s, 9H); 1.75 (s, 3H); 1.87 (s, 3H); 1.88-2.01 (t, 1H); 2.20 (s, 3H); 2.23-2.32 (d, 2H); 2.39 (s, 3H); 2.75-2.88 (m, 1H); 3.34-3.45 (m, 1H); 3.58-3.70(m, 1H); 3.81 (s, 1H); 3.81-3.87 (d, 1H); 4.05-4.15 (dd, 1H); 4.15-4.20 (d, 1H); 4.27-4.31 (d, 1H);4.59-4.65 (m, 2H); 4.72 (s, 2H); 4.89-4.97 (d, 1H); 5.17-5.22 (d, 1H); 5.30-5.36 (dd, 1H); 5.63-5.70 (d, 1H); 6.09-6.19 (t, 1H); 6.33 (s, 1H); 7.27-7.41 (m, 5H); 7.43-7.54 (t, 2H); 7.57-7.65 (t, 1H); 8.06-8.12 (d, 2H).

Mass Spec (FAB, *m/z)* (M+H)⁺ measured at 907.4240; theory for C₄₈H₆₂N₂O₁₅ is 907.4228; 907, 627, 567, 281, 263, 235, 205, 136, 105, 59, 43.

### Example 17 Preparation of 7-[O-(2,2,2-trichloroethoxy)methyl]-13-(N-Boc-β-phenyl isoserinyl)-baccatin III (21) and 7-[O-(2,2,2-trichloroethoxy)methoxymethyl]-13-(N-Boc-β-phenyl isoserinyl)-baccatin III (22)

13-(N-Boc-2'-TES-β-phenyl isoserinyl)-baccatin III (2, 95 mg, 0.099mM) is stirred at RT under nitrogen in methylene chloride (1 mL) and the solution treated with a (1-1)-mixture of chloromethyl-(2,2,2-trichloroethyl) ether and chloromethyl-(2,2,2-trichloroethoxy)methyl ether (98 mL) and diisopropyl ethyl amine (109 mL, 0.624 mM). The reaction is followed by TLC, which shows the reaction not to be complete after 22 days. Thus, additional (1-1)-mixture of chloromethyl-(2,2,2-trichloroethyl) ether and chloromethyl-(2,2,2-trichloroethoxy)methyl ether (98 mL) are added as well as 1,2-dichloroethane (1 mL). The reaction is then heated in a 4 day cycle to 75°C for 8 h and allowed to stand at RT for 16 h. The reaction is then heated to 75°C continuously for 24 hours. The reaction mixture is then chromatographed over silica gel (15 g), eluting with (25-75) acetone-hexane. Fractions of 3 mL are collected, analyzing them by TLC, which indicates the presence of four compounds. The two less polar compounds are found in fractions 10-13 and the two more polar compounds in fractions 14-33. Evaporation of fractions 10-13 leaves a residue which is treated with (80-20) acetic acid water (3 mL). This reaction is then freeze dried. TLC shows the products of this reaction to be the same as the products found in fractions 14-33 above. Thus, all the residues are combined and chromatographed over an E. Merck size A HPLC silica gel column, eluting with a gradient of (20-80) to (40-60) ethyl acetate-hexane. 7-[O-(2,2,2-trichloroethoxy)methyl]-13-(N-Bob-β-phenyl isoserinyl)-baccatin III (21, 23mg, 23% yield) is found on evaporation of fractions 52-66 as a white solid and 7-[O-(2,2,2-trichloroethoxy)methoxymethyl]-13-(N-Boc-β-phenyl isoserinyl)-baccatin III (22, 23 mg, 22% yield) is found on evaporation of fractions 68-88 as a white solid.

Data for 7-[O-(2,2,2-trichloroethoxy)methyl]-13-(N-Boc-β-phenyl isoserinyl)-baccatin III (21):

TLC (silica gel): (25-75) ethyl acetate-hexane; R_{f}: 0.25

Proton NMR (CDCl₃; TMS): δ 1.20 (s, 3H); 1.24 (s, 3H); 1.35 (s, 9H); L76 (s, 3H); 1.87 (s, 3H); 1.92-2.03 (t, 1H); 2.22 (s, 3H); 2.26-2.34 (d, 2H); 2.37 (s, 3H); 2.85-2.99 (m, 1H); 3.37-3.53 (bs, 1H); 3.80-3.89 (d, 1H); 3.97-4.06 (d, 1H); 4.12-4.25 (m, 3H); 4.27-4.34 (d, 1H); 4.63 (bs, 1H); 4.87-5.03 (m, 3H); 5.18-5.30 (bd, 1H); 5.37-5.47 (bd, 1H); 5.61-5.70 (d, 1H); 6.12-6.23 (t, 1H); 6.32 (s, 1H); 7.27-7.45 (m, 5H); 7.45-7.54 (t, 2H); 7.57-7.65 (t, 1H); 8.05-8.15 (d, 2H).

Mass Spec (FAB, *m/z)* (M+H)⁺ measured at 908.4089; theory for C₄₈H₆₂N₁O₁₆ is 908.4068; 908, 627, 585, 105, 59, 57.

Data for 7-[O-(2,2,2-trichloroethoxy)methoxymethyl]-13-(N-Boc-β-phenyl isoserinyl)-baccatin III (22):

TLC (silica gel): (25-75) ethyl acetate-hexane; R_{f}: 0.17

Proton NMR (CDCl₃; TMS): δ 1.22 (s, 6H); 1.35 (s, 9H); 1.75 (s, 3H); 1.91(s, 3H); 2.21 (s, 3H); 2.25-2.32 (d, 2H); 2.37 (s, 3H); 2.69-2.82 (m, 1H); 3.35-3.48 (bs, 1H); 3.80-3.86 (d, 1H); 4.13-4.26 (m, 4H); 4.26-4.34 (d, 1H); 4.63 (bs, 1H); 4.73-4.80 (d, 1H); 4.83-5.04 (m, 4H); 5.21-5.32 (d, 1H); 5.40-5.47 (d, 1H); 5.64-5.70 (d, 1H); 6.14-6.24 (t, 1H); 6.39 (s, 1H); 7.30-7.45 (m, 5H); 7.45-7.57 (t, 2H); 7.57-7.66 (t, 1H); 8.07-8.15 (d, 2H).

Mass Spec (FAB, *m/z)* (M+H)⁺ measured at 908.4089; theory for C₄₈H₆₂N₁O₁₆ is 908.4068; 908, 627, 585, 105, 59, 57.

### Example 21 Preparation of 7-(O-methoxymethyl)-baccatin III-13-(4S,5R)-N-(t-butylaminocarbonyl)-2-(2,4-dimethoxyphenyl)-4-phenyl-5-oxazolidinecarboxylic acid ester (26)

Baccatin III-13-(4S,5R)-N-(butylaminocarbonyl)-2-(2,4-dimethoxyphenyl-4-phenyl-5-oxazolidinecarboxylic acid ester (12, 610 mg, 0.612 mM) is stirred at RT under nitrogen in methylene chloride (3 mL) and the solution treated with chloromethyl methyl ether (232 mL, 3.06 mM) and diisopropyl ethyl amine (530 mL, 3.06 mM). The reaction is followed by TLC. After 24 hours the reaction is found to be complete. The reaction is then diluted with methylene chloride and washed with 5% sodium bisulfate and 5% sodium bicarbonate, dried over sodium sulfate and evaporated under vacuum. The crude product is chromatographed over silica gel (70 g), eluting with a gradient of (25-75) to (30-70) acetone-hexane. Fractions of 20 mL are collected, analyzing them by TLC. 7-(O-methoxymethyl)-baccatin III-13-(4S,5R)-N-(t-butylaminocarbonyl)-2-(2,4-dimethoxyphenyl)-4-phenyl-5-oxazolidinecarboxylic acid ester (less polar isomer 26a, 532 mg, 84% yield) is found on evaporation of fractions 31-47 as a white solid and methoxymethyl-baccatin III-13-(4S,5R)-N-(t-butylaminocarbonyl)-2-(2,4-dimethoxyphenyl)-4-phenyl-5-oxazolidinecarboxylic acid ester (more polar isomer 26b, 62 mg, 10% yield) is found on evaporation of fractions 48-57 as a white solid.
Data for 7-(O-methoxymethyl)-baccatin III-13-(4S,5R)-N-(t-butylaminocarbonyl)-2-(2,4-dimethoxyphenyl)-4-phenyl-5-oxazolidinecarboxylic acid ester (26a):

TLC (silica gel): (30-70) acetone-hexane; R_{f}: 0.42

Proton NMR (CDCl₃; TMS): δ 1.17 (s, 12H); 1.23 (s, 3H); 1.75 (s, 3H); 1.93 (s, 3H); 2.07 (s, 3H); 2.21 (s, 3H); 2.66-2.83 (m, 1H); 3.29 (s, 3H); 3.78-3.90 (m, 1H); 3.82 (s, 3H); 3.91(s, 3H); 4.06-4.30 (m, 3H); 4.56-4.76 (m, 3H); 4.81-4.91 (d, 1H); 4.97 (s, 1H); 5.58 (s, 1H); 5.62-5.70 (d, 1H); 6.24-6.36 (t, 1H); 6.39 (s, 1H); 6.45-6.57 (m, 2H); 6.75 (s, 1H); 7.24-7.64 (m, 9H); 7.96-8.08 (d, 2H).
Data for 7-(O-methoxymethyl)-baccatin III-13-(4S,5R)-N-(t-butylaminocarbonyl)-2-(2,4-dimethoxyphenyl)-4-phenyl-5-oxazolidinecarboxylic acid ester (26b):

TLC (silica gel): (30-70) acetone-hexane; R_{f}: 0.35

Proton NMR (CDCl₃; TMS): δ 0.96 (s, 9H); 1.16 (s, 3H); 1.20 (s, 3H); 1.66 (s, 3H); 1.70 (s, 3H); 1.81 (s, 3H); 2.20 (s, 3H); 2.67-2.82 (m, 1H); 3.28 (s, 3H); 3.72-3.78 (d, 1H); 3.82 (s, 3H); 3.90 (s, 3H); 4.03-4.17 (m, 2H); 4.20-4.25 (d, 1H); 4.50-4.54 (d, 1H); 4.58-4.65 (d, 1H); 4.65-4.70 (d, 1H); 4.80-4.88 (d, 1H); 5.40-5.46 (d, 1H); 5.58-5.64 (d, 1H); 6.03-6.13 (t, 1H); 6.27 (s, 1H); 6.48-6.58 (m, 2H); 6.73 (s, 1H); 7.33-7.58 (m, 8H); 7.58-7.65 (t, 1H); 7.99-8.05 (d, 2H).

### Example 22 Preparation of 7-(O-methoxymethyl)-13-(N-(t-butylaminocarbonyl)-β-phenyl isoserinyl)-baccatin III (27)

7-(O-Methoxymethyl)-baccatin III-13-(4S,5R)-N-(t-butylaminocarbonyl)-2-(2,4-dimethoxyphenyl)-4-phenyl-5-oxazolidinecarboxylic acid ester (594 mg, 26a and 26b from example 21, 0.57 mM) is stirred at RT under nitrogen in (80-20) acetic acid-water (25 mL). The reaction is followed by TLC and found to be complete in 4 hours. The reaction is then freeze-dried. The crude residue is purified over a silica gel column (60 g), eluting with (35-65) acetone-hexane. Fractions of 15 mL are collected, analyzing them by TLC. The product is found in fractions 27-45 which are combined and evaporated under vacuum to give 7-(O-methoxymethyl)-13-(N-(t-butylaminocarbonyl)-β-phenyl isoserinyl)-baccatin III (27, 385 mg, 75% yield) as a white solid.

TLC (silica gel): (30-70) acetone-hexane; R_{f}: 0.23

Proton NMR (CDCl₃; TMS): δ 1.22 (s, 3H); 1.24 (s, 12H); 1.76 (s, 3H); 1.89 (s, 3H); 2.20 (s, 3H); 2.25-2.34 (d, 2H); 2.40 (s, 3H); 2.72-2.86 (m, 1H); 3.29 (s 2H); 3.68 (bs, 1H); 3.81-3.88 (d, 1H); 4.07-4.15 (dd, 1H); 4.15-4.22 (d, 1H); 4.26-4.34 (d, 1H); 4.55 (bs, 1H); 4.60-4.74 (m, 3H); 4.89-4.96 (d, 1H); 5.06-5.16 (bd, 1H); 5.30-5.37 (dd, 1H); 5.64-5.70 (d, 1H); 6.10-6.20 (t, 1H); 6.36 (s, 1H); 7.27-7.40 (m, 5H); 7.45-7.55 (t, 2H); 7.58-7.66 (t, 1H); 8.06-8.14 (d, 2H).

Mass Spec (FAB, *m/z)* (M+H)⁺ measured at 893.4095; theory for C₄₇H₆₁N₂O₁₅ is 893.4072; 969, 893, 613, 281, 263, 235, 205, 136, 105.

### Example 35 Preparation of 2'-TES-7-(O-methylthiomethyl) taxol (41).

2'-TES-taxol (40, 100 mg, 0.103 mM) is stirred at 0° C under nitrogen in 0.4 mL of dry acetonitrile and the solution treated with dimethyl sulfide (58 mL) and benzoyl peroxide (25 mg) 4 times at 5 minute intervals. The reaction is allowed to proceed at 0° C for 3.5 hours. It is then diluted with ethyl acetate and washed with 5% sodium bicarbonate, dried over sodium sulfate and evaporated under vacuum. The crude product is chromatographed over silica gel (10g), eluting with (30-70) ethyl acetate-hexane. Fractions of 3 mL are collected analyzing them by TLC. Fractions 24-46 contained the pure product and are combined and evaporated, leaving 2'-TES-7-methyl thiomethyl taxol (41, 54 mg, 51%) as a white solid.

TLC (silica gel): (30-70) ethyl acetate-hexane; Rf: 0.31

Proton NMR (CDCl₃; TMS): δ 0.34-0.58 (m, 6H); 0.77-0.85 (t, 9H); 1.26 (s, 3H); 1.27 (s, 3H); 1.78 (s, 3H); 1.80-1.94 (m, 2H); 2.08 (s, 3H); 2.12 (s, 3H); 2.19 (s, 3H); 2.35-2.50 (m, 1H); 2.56 (s, 3H); 2.76-2.90 (m, 1H); 3.86-3.96 (d, 1H); 4.18-4.39 (2d+1m, 3H); 4.68 (s, 2H); 4.70-4.75 (d, 1H); 4.93-5.03 (d, 1H); 5.68-5.79 (m, 2H); 6.20-6.32 (t, 1H); 6.58 (s, 1H); 7.13-7.23 (d, 1H); 7.30-7.60 (m, 10H); 7.71-7.80 (d, 2H); 7.90-7.97 (d, 1H); 8.06-8.17(d, 2H).

### Example 36 Preparation of 7-(O-methylthiomethyl) taxol (42)

2'-TES-7-(O-methylthiomethyl) taxol (41, 54 mg, 0.053mM) is stirred at RT under nitrogen in (80-20) acetic acid-water (6 mL) for 3 hours, when it is found to be complete by TLC. The reaction is then freeze-dried. The crude product is chromatographed over 5g silica gel, eluting with 50-50 ethyl acetate-hexane. Fractions of 1 mL are collected analyzing them by TLC. Fractions 11-35 contained the pure product and are combined and evaporated, leaving 7-(O-methylthiomethyl) taxol (42, 42 mg, 88% yield) as a white solid.

TLC (silica gel): (40-60) ethyl acetate-hexane; Rf: 0.23

Proton NMR (CDCl₃; TMS): δ 1.10 (s, 3H); 1.14 (s, 3H); 1.68 (s, 3H); 1.85 (s, 3H); 2.04 (s, 3H); 2.11 (s, 3H); 2.20-2.28 (d, 2H); 2.30 (s, 3H); 2.64-2.80 (m, 1H); 3.74-3.82 (d, 1H); 4.07-4.15 (d, 1H); 4.15-4.30 (d+m, 2H); 4.58 (s, 2H); 4.70-4.75 (d, 1H); 4.82-4.92 (d, 1H); 5.55-5.64 (d, 1H); 5.68-5.76 (d, 1H); 6.04-6.15 (t, 1H); 6.44 (s, 1H); 6.99-7.09 (d, 1H); 7.23-7.49 (m, 10H); 7.64-7.74 (d, 2H); 8.00-8.08(d, 2H).

Mass Spec (FAB, *m/z)* (M+H)⁺ measured at 914.3429; theory for C₄₉H₅₆N₁O₁₄S₁ is 914.3421; 990, 914, 836, 629, 286, 268, 240, 210, 121, 105, 61, 43.

### Example 37 Preparation of 7-(O-methylthiomethyl)-13-(N-Boc-2'-TES-β-phenyl isoserinyl)-baccatin III (43).

13-(N-Boc-2'-TES-β-phenyl isoserinyl)-baccatin III (2, 287 mg, 0.298 mM) is stirred at 0° C under nitrogen in dry acetonitrile (1.2 mL) and the solution treated with dimethyl sulfide (170 mL) and benzoyl peroxide (73 mg) 4 times at 5 minute intervals. The reaction is allowed to proceed at 0° C for 4 hours, when TLC shows it to be complete. It is then diluted with ethyl acetate and washed with 5% sodium bicarbonate-brine, dried over sodium sulfate and evaporated under vacuum. The crude product is chromatographed over silica gel (35g), eluting with (30-70) ethyl acetate-hexane. Fractions of 4 mL are collected analyzing them by TLC. Fractions 26-51 contained the pure product and are combined and evaporated leaving 7-(O-methylthiomethyl)-13-(N-Boc-2'-TES-β-phenyl isoserinyl)-baccatin III (43, 273 mg, 90% yield) as a white solid.

TLC (silica gel): (30-70) ethyl acetate-hexane; Rf: 0.46

Proton NMR (CDCl₃; TMS): δ 0.30-0.49 (m, 6H); 0.71-0.84 (t, 9H); 1.26 (s, 3H); 1.32 (s, 9H); 1.77 (s, 3H); 1.80-1.93 (t, 2H); 2.05 (s, 3H); 2.12 (s, 3H); 2.15 (s, 3H); 2.19 (s, 3H); 2.33-2.44 (m, 1H); 2.53 (s, 3H); 2.78-2.89 (m, 1H); 3.87-3.95 (d, 1H); 4.16-4.23 (d, H); 4.25-4.35(d+m, 2H); 4.57 (s, 1H); 4.67 (s, 1H); 4.93-5.01 (d, 1H); 5.23-5.35 (bs, 1H); 5.46-5.56 (d, 1H); 5.67-5.75 (d, 1H); 6.23-6.32 (t, 1H); 6.57 (s, 1H); 7.23-7.33 (m, 3H); 7.33-7.41 (t, 2H); 7.43-7.53 (t, 2H); 7.53-7.63 (t, 1H); 8.06-8.14(d, 2H).

### Example 38 Preparation of 7-(O-methylthiomethyl)-13-(N-Boc-β-phenyl isoserinyl)-baccatin III (44).

7-(O-Methylthiomethyl)-13-(N-Boc-2'-TES-β-phenyl isoserinyl)-baccatin III (43, 273 mg, 0.267mM) is stirred at RT under nitrogen in (80-20) acetic acid-water (30 mL) for 4.5 hours, when it is found to be complete by TLC. The reaction is then freeze-dried. The crude product is chromatographed over silica gel (30 g), eluting with (70-30) ethyl acetate-hexane. Fractions of 4 mL are collected analyzing them by TLC. Fractions 15-25 contained the pure product and are combined and evaporated, leaving 7-(O-methylthiomethyl)-13-(N-Boc-β-phenyl isoserinyl)-baccatin III (44, 225 mg, 93% yield) as a white solid.

TLC (silica gel): (30-70) ethyl acetate-hexane; Rf: 0.26

Proton NMR (CDCl₃; TMS): δ 1.20 (s, 3H); 1.34 (s, 9H); 1.75 (s, 3H); 1.79-1.96 (m, 2H); 1.89 (s, 3H); 2.11 (s, 3H); 2.14 (s, 3H); 2.18 (s, 3H); 2.23-2.32 (d, 2H); 2.36 (s, 3H); 2.73-2.85 (m, 1H); 3.54-3.63 (d, 1H); 3.83-3.91 (d, 1H); 4.13-4.21 (d, 1H); 4.25-4.34 (d+m, 2H); 4.55-4.70 (m, 3H); 4.90-4.98 (d, 1H); 5.20-5.32 (bd, 1H); 5.44-5.56 (bd, 1H); 5.64-5.72 (d, 1H); 6.14-6.24 (t, 1H); 6.54 (s, 1H); 7.24-7.44 (m, 5H); 7.44-7.53 (t, 2H); 7.54-7.64 (t, 1H); 8.04-8.14(d, 2H).

Mass Spec (FAB, *m/z)* (M+H)⁺ measured at 910.3697; theory for C₄₇H_{6O}N₁O₁₅S₁ is 910.3683; 910, 629, 587, 569, 105, 61, 57, 43.

### Example 39 Preparation of 2'-TES-7-(O-phenylthiomethyl) taxol (45).

2'-TES-taxol (40, 104 mg, 0.107 mM) is stirred at 0° C under nitrogen in dry acetonitrile (0.4 mL) and the solution treated with thioanisol (97 mL) and benzoyl peroxide (25 mg) 4 times at 5 minute intervals. The reaction is allowed to proceed at 0° C for 5 hours and in the refrigerator overnight, when TLC shows it to be complete. It is then diluted with ethyl acetate and washed with 5% sodium bicarbonate, dried over sodium sulfate and evaporated under vacuum. The crude product is chromatographed over silica gel (12 g), eluting with (30-70) ethyl acetate-hexane. Fractions of 4 mL are collected analyzing them by TLC. Fractions 18-43 contain the pure product and are combined and evaporated leaving 2'-TES-7-(O-phenylthiomethyl) taxol (45, 91 mg, 78% yield) as a white solid.

TLC (silica gel): (30-70) ethyl acetate-hexane; Rf: 0.47

Proton NMR (CDCl₃; TMS): δ 0.37-0.59 (m, 6H); 0.78-0.90 (t, 9H); 0.88 (s, 3H); 1.16 (s, 3H); 1.79 (s, 3H); 1.95 (s, 3H); 2.09 (s, 3H); 2.35-2.48 (m, 1H); 2.56 (s, 3H); 2.74-2.87 (m, 1H); 3.89-3.97 (d, 1H); 4.20-4.28 (d, 1H); 4.30-4.44 (m, 2H); 4.70-4.75 (d, 1H); 4.90-4.99 (d, 1H); 5.05 (s, 2H); 5.69-5.76 (m, 2H); 6.18-6.30 (t, 1H); 6.52 (s, 1H); 7.11-7.63 (m, 16H); 7.72-7.80 (d, 2H); 8.07-8.17(d, 2H).

### Example 40 Preparation of 7-(O-phenylthiomethyl) taxol (46).

2'-TES-7-(O-phenylhiomethyl) taxol (45, 91 mg) is stirred at RT under nitrogen in (80-20) acetic acid-water (10 mL) for 2 hours, when it is found to be complete by TLC. The reaction is then freeze-dried. The crude product is chromatographed over silica gel (10g), eluting with (70-30) ethyl acetate-hexane. Fractions of 3 mL are collected analyzing them by TLC. Fractions 10-33 contain the pure product and are combined and evaporated, leaving 7-phenylthiomethyl taxol (46, 62 mg,77%) as a white solid.

TLC (silica gel): (40-60) ethyl acetate-hexane; R_{f}:0.26.

Proton NMR (CDCl₃; TMS): δ 1.06 (s, 3H); 1.11 (s, 3H); 1.69 (s, 3H); 1.86 (s, 3H); 1.89 (s, 3H); 2.18-2.27 (d, 2H); 2.30 (s, 3H); 2.60-2.76 (m, 1H); 3.74-3.83 (d, 1H); 4.07-4.14 (d, 1H); 4.1-4.30 (m, 2H); 4.69-4.74 (d, 1H); 4.77-4.86 (d, 1H); 4.95 (s, 2H); 5.55-5.64 (d, 1H); 5.68-5.76 (dd, 1H); 6.04-6.14 (t, 1H); 6.36 (s, 1H); 7.04-7.14 (t, 2H); 7.14-7.47 (m, 14H); 7.47-7.56 (t, 1H); 7.62-7.70 (d, 2H); 7.97-8.07 (d, 2H).

Mass Spec (FAB, *m/z)* (M+H)⁺ measured at 976.3557; theory for C₅₄H₅₈O₁₄N₁S₁ is 976.3578; 1052, 976, 836, 691, 286, 268, 240, 210, 123, 105, 77, 43.

### Example 41 Preparation of 7-O-methyl Taxol (47)

7-(O-phenylthiomethyl) taxol (46, 50 mg, 0.051 mM) is stirred at 0° C under nitrogen in 2 mL abs. EtOH and the solution treated with 0.5 mL Raney Nickel in 1 mL abs. EtOH (the Raney Nickel is washed with water (5x), acetone and ethanol before use). After a few minutes the reaction is warmed to RT. The reaction is followed by TLC. After 40 minutes the reaction, is filtered through Celite, washing well with abs. EtOH. The filtrate and wash are combined and evaporated under vacuum. The crude product is chromatographed over silica gel (5 g), eluting with a gradient of (30-70) to (70-30) ethyl acetate-hexane. Fractions of 1 mL are collected, analyzing them by TLC. Fractions 55-78 contained impure product and are combined and evaporated under vacuum. Rechromatographing the impure product on HPLC grade silica gel, using (35-65) acetone-hexane as eluant, gives 7-OMe Taxol (47, 13 mg, 30% yield) as a white solid.

TLC (silica gel): (35-65) ethyl acetate-hexane; R_{f}:0.44.

Proton NMR (CDCl₃; TMS): δ 1.20 (s, 6H); 1.68 (s, 3H); 1.82 (s, 3H); 2.22 (s, 3H); 2.25-2.34 (dd, 2H); 2.38 (s, 3H); 2.64-2.80 (m, 1H); 3.34 (s, 3H); 3.63-3.74 (d, 1H); 3.74-3.91 (m, 2H); 4.12-4.22 (d, 1H); 4.25-4.34 (d, 1H); 4.74-4.84 bs, 1H); 4.90-5.01 (d, 1H); 5.60-5.70 (d, 1H); 5.74-5.84 (dd, 1H); 6.12-6.24 (t, 1H); 6.39 (s, 1H); 7.06-7.14 (d, 1H); 7.30-7.56 (m, 10H); 7.56-7.66 (t, 1H); 7.21-7.32 (d, 2H); 8.04-8.16 (d, 2H).

Mass Spec (FAB, *m/z)* (M+H)⁺ measured at 868.3534; theory for C₄₈H₅₄O₁₄N₁ is 868.3544; 868, 583, 286, 268, 240, 210, 121, 105, 43.

### Example 42 Preparation of 7-[O-ethyl(1-thioethyl)] Taxol (49)

2'-TES-taxol (40, 200 mg, 0.207 mM) is stirred at 0° C under nitrogen in dry acetonitrile (0.8 mL) and the solution treated with diethyl sulfide (0.178 mL) and benzoyl peroxide (50 mg) 4 times at 5 minute intervals. The reaction is allowed to proceed at 0° C for 3 hours, in the freezer for 5 hours and in the refrigerator overnight, following the reaction by TLC. The reaction is then diluted with ethyl acetate and washed with 5% sodium bicarbonate. The organic layer is dried over sodium sulfate and evaporated under vaccum. The resultant crude product is chromatographed over silica gel (25 g), eluting with a gradient of (30-70) to (90-10) ethyl acetate-hexane. Fractions of 5 mL are collected, analyzing them by TLC. Fractions containing 2'-TES-7-ethylthioethyl Taxol (48) are combined and evaporated under vacuum. The impure product is stirred at RT under nitrogen in (80-20) HOAc-water (3 mL) and the reaction followed by TLC and is found to be complete after 2 hours. The reaction is then freeze-dried. The product is chromatographed over HPLC grade silica gel, eluting with (45-55) ethyl acetate hexane. This still gives impure product so the chromatography is repeated using a gradient of (30-70) to (40-60) ethyl acetate-toluene. This gives 7-[O-ethyl(1-thioethyl)] Taxol (49, 17 mg) as a white solid.

TLC (silica gel): (50-50) ethyl acetate-hexane; R_{f}:0.50.

Proton NMR (CDCl₃; TMS): δ 1.12-1.32 (2s+1t, 9H); 1.50-1.56 (d, 3H); 1.67-1.84 (m, 5H); 1.92 (s, 3H); 2.18 (s, 3H); 2.38 (s, 3H); 2.54-2.70 (m, 1H); 2.70-2.84 (m, 1H); 3.68-3.74 (d, 1H); 3.82-3.90 (d, 1H); 4.13-4.23 (d, 1H); 4.26-4.35 (d, 1H); 4.50-4.60 (dd, 1H); 4.64-4.74 (q, 1H); 4.80 (bs, 1H); 4.92-5.01 (d, 1H); 5.62-5.70 (d, 1H); 5.75-5.84 (dd, 1H); 6.13-6.24 (t, 1H); 6.55 (s, 1H); 7.05-7.14 (d, 1H); 7.30-7.57 (m, 10H); 7.57-7.66 (t, 1H); 7.72-7.78 (d, 2H); 8.07-8.16 (d, 2H).

Mass Spec (FAB, *m/z*) (M+H)⁺ measured at 942.3713; theory for C₅₁H₆₀O₁₄N₁S₁ is 942.3734; 942, 880, 854, 836, 286, 268, 240, 210, 122, 105, 89, 77, 43.

### Example 44 Preparation of 13-(N-Cbz-2'-TES-b-phenyl isoserinyl)-baccatin III (51)

This material is prepared from 13-(N-Cbz-b-phenyl isoserinyl)-baccatin III (50, See U.S. Serial No. POT/US 93/11827 filed 12/13/93 and WO 94/13655 published 06/23/94 which are incorporated herein by reference) in the same manner as 13-(N-Boc-2'-TES-b-phenyl isoserinyl)-baccatin III is prepared from 13-(N-Boc-b-phenyl isoserinyl)-baccatin III in Example 1.

### Example 45 Preparation of 13-(2'-TES-b-phenyl isoserinyl)-baccatin III (52)

13-(N-Cbz-2'-TES-b-phenyl isoserinyl)-baccatin III (51, 217 mg, 0.217 mM) is stirred at RT under nitrogen in 2 mL dry THF-3 mL methanol. To the solution is added 100 mg ammonium formate and 60 mg 10% Pd/C. The reaction is allowed to proceed for 30 minutes when TLC showed the reaction to be complete. The reaction is filtered through Celite, washing with ethyl acetate. The combined filtrate and wash is washed with 5% sodium bicarbonate, dried over sodium sulfate and evaporated under vacuum. The residue is reevaporated twice with toluene and once with ethyl acetate-hexane to give 13-(2'-TES-b-phenyl isoserinyl)-baccatin III (52, 186 mg) as a white solid.

TLC: silica gel; 50-50 ethyl acetate-hexane; R_{f}:0.36.

¹H NMR (CDCL₃; TMS): d 0.43-0.57 (m, 6H); 0.78-0.92 (t, 9H); 1.03 (s, 3H); 1.14 (s, 3H); 1.54 (s, 3H); 1.73 (s, 3H); 2.08 (s, 3H); 2.21 (s, 3H); 2.38-2.52 (m, 1H); 3.61-3.69 (d, 1H); 4.01-4.12 (2d, 2H); 4.13-4.24 (2d, 2H); 4.24-4.35 (dd, 1H); 4.81-4.89 (d, 1H); 5.49-5.56 (d, 1H); 5.93-6.05 (t, 1H); 6.18 (s, 1H); 7.03-7.30 (m, 5H); 7.40-7.50 (t, 2H); 7.54-7.63 (t, 1H); 7.90-7.98 (d, 2H).

### Example 46 Preparation of l3-(N-(t-butylaminocarbonyl)-2'-TES-b-phenyl isoserinyl)baccatin III (53)

13-(2'-TES-b-phenyl isoserinyl)-baccatin III (52, 186 mg, 0.215 mM) is stirred at 0°C under nitrogen in 2 mL dry THF. To this is added by syringe t-butyl isocyanate (0.03 mL). After 5 minutes, the reaction is warmed to RT, following the reaction by TLC. After 2 hours more t-butyl isocyanate (0.01 mL ) is added. After a total reaction time of 4.5 hours, the reaction is found to me essentially complete by TLC. The reaction is evaporated under vaccum and the crude residue chromatographed over 20 g silica gel, eluting with 40-60 ethyl acetate-hexane. Fractions of 2 mL are collected, analyzing them by TLC. Fractions 20-72 contain pure product and are combined and evaporated under vacuum to give 13-(N-(t-butylaminocarbonyl)-2'-TEs-b-phenyl isoserinyl)-baccatin III (53, 193 mg) as a white solid.

TLC: silica gel; 50-50 ethyl acetate-hexane; R_{f}:0.61.

¹H NMR (CDCL₃; TMS): d 0.14-0.38 (m, 6H); 0.64-0.74 (t, 9H); 1.08 (s, 3H); 1.15 (s. 9H); 1.20 (s, 3H); 1.63 (s, 3H); 1.86 (s, 3H); 2.16 (s, 3H); 2.52 (s, 3H); 2.60-2.66 (d, 1H); 3.72-3.79 (d, 1H); 4.11-4.18 (d, 1H); 4.20-4.28 (d, 1H); 4.28-4.38 (m, 1H); 4.46-4.50 (d, 1H); 4.88-4.96 (m, 2H); 5.10-5.18 (d, 1H); 5.22-5.30 (d, 1H); 5.58-5.64 (d, 1H); 6.12-6.24 (t, 1H); 6.26 (s, 1H); 7.15-7.33 (m, 5H); 7.36-7.66 (t, 2H); 7.47-7.55 (t, 1H); 7.99-8.06 (d, 2H).

### Example 47 Preparation of l3-(N-(t-butylaminocarbonyl)-2'-TES-b-phenyl isoserinyl)-baccatin III 7-O-methylthiomethyl ether (54)

13-(N-(t-butylaminocarbonyl)-2'-TES-b-phenyl isoserinyl)-baccatin III (53, 193 mg, 0.203 mM) is stirred at 0°C under nitrogen in 2 mL acetonitlile. To this is added by syringe dimethyl sulfide ( 0.115 mL) followed by four additions 5 minutes apart of benzoyl peroxide (50 mg portions). After 30 minutes everything is in solution and after 2 hours the reaction is complete by TLC. The reaction is partitioned between ethyl acetate-5% sodium bicarbonate. The organic layer is dried over sodium sulfate and evaporated under vacuum. The crude product is chromatographed over 20 g silica gel, eluting with 30-70 ethyl acetate-hexane. Fractions of 2 mL are collected, analyzing them by TLC. Fractions 25-61 contain pure product and are combined and evaporated under vacuum to give 13-(N-(t-butylamino-carbonyl)-2'-TES-b-phenyl isoserinyl)-baccatin III 7-O-methylthiomethyl ether (54, 178 mg) as a white solid.

TLC: silica gel; 50-50 ethyl acetate-hexane; R_{f}:0.34.

¹H NMR (CDCL₃; TMS): d 0.14-0.40 (m,6H); 0.64-0.76 (t, 9H); 1.16 (s, 3H); 1.17 (s. 12H); 1.70 (s, 3H); 1.74-1.88 (m, 1H); 2.00 (s, 3H); 2.03 (s, 3H); 2.11 (s, 3H); 2.14-2.24 (m, 1H); 2.30-2.44 (m, 1H); 2.54 (s, 3H); 2.71-2.86 (m, 1H); 3.79-3.88 (d, 1H); 4.10-4.23 (m, 2H); 4.23-4.29 (d, 1H); 4.47-4.51 (d, 1H); 4.54-4.61 (d, 1H); 4.87-4.95 (d+s, 2H); 5.12-5.20 (d, 1H); 5.24-5.30 (d, 1H); 5.60-5.68 (d, 1H); 6.09-6.21 (t, 1H); 6.49 (s, 1H); 7.17-7.34 (m, 5H); 7.38-7.46 (t, 2H); 7.48-7.56 (t, 1H); 7.99-8.08 (d, 2H).

### Example 48 Preparation of 13-(N-(t-butylaminocarbonyl)-b-phenyl isoserinyl)-baccatin III 7-O-methylthiomethyl ether (55)

13-(N-(t-butylaminocarbonyl)-2'-TES-b-phenyl isoserinyl)-baccatin III 7-O-methylthiomethyl ether (54, 178 mg, 0.174mM) is stirred at RT under nitrogen in 25 ml 80-20 acetic acid-water. TLC after 5 minutes shows the reaction to be complete.The reaction is freeze-dried overnight. The crude product is chromatographed over 20 g silica gel, eluting with 50-50 ethyl acetate-hexane. Fractions of 3 mL are collected, analyzing them by TLC. Fractions 30-60 are found to contain 13-(N-(t-butylaminocarbonyl)-b-phenyl isoserinyl)-baccatin III 7-O-methylthiomethyl ether (55) as a white solid.

TLC: silica gel; 50-50 ethyl acetate-hexane; R_{f}:0.47.

¹H NMR (CDCL₃; TMS): d 1.20 (s, 3H); 1.24 (s, 12H); 1.71 (s, 3H); 2.00 (s, 3H); 2.12 (s, 3H); 2.20 (s, 3H); 2.27-2.35 (d, 2H); 2.40 (s, 3H); 2.74-2.88 (m, 1H); 3.72-3.79 (d, 1H); 3.84-3.90 (d, 1H); 4.15-4.22 (d, 1H); 4.26-4.35 (m, 2H); 4.54(s, 1H); 4.61-4.70 (m, 2H); 4.93-4.99 (d, 1H); 5.10-5.16 (d, 1H); 5.33-5.40 (dd, 1H); 5.67-5.74 (d, 1H); 6.10-6.21 (t, 1H); 6.54 (s, 1H); 7.28-7.42 (m, 5H); 7.45-7.54 (t, 2H); 7.58-7.65 (t, 1H); 8.08-8.14 (d, 2H).

Mass Spec (FAB, *m/z*) (M+H)⁺ measured at 909.3822; theory for C₄₇H₆₁O₁₄N₂S₁ is 942.3734; 281, 263, 235, 205, 182, 136, 105, 61, 43.

### Example 49 Preparation of 13-(N-(t-butylaminocarbonyl)-b-phenyl isoserinyl)-baccatin III 7-O-methyl ether (56)

A 4 mL quantity Raney Nickel wetted with absolute ethanol is stirred at 0°C under nitrogen. To this is added by syringe l3-(N-(t-butylamino-carbonyl)-b-phenyl isoserinyl)-baccatin III 7-O-methylthiomethyl ether (55, 52 mg, 0.057 mM) in 2 mL absolute ethanol. The temperature is kept at 0°C throughout the reaction and the washing process. The reaction is followed by TLC and left to go for 5 hours. The Raney Nickel is allowed to settle and the supernatant removed by suction. Repeating four times, THF (20 mL) is added, stirred 2 min and removed by suction. The combined washing are evaporated under vacuum, leaving 50 mg solid. The crude product is chromatographed over 5g HPLC grade silica gel, eluting with 50-50 ethyl acetate-hexane. Fractions of 2 mL are collected, analyzing them by TLC. Fractions 29-40 are found to contain l3-(N-(t-butylaminocarbonyl)-b-phenyl isoserinyl)-baccatin III 7-O-methyl ether (56, 18 mg) as a white solid.

TLC: silica gel; 50-50 ethyl acetate-hexane; R_{f}:0.32.

¹H NMR (CDCL₃; TMS): d 1.19 (s, 3H); 1.22 (s, 9H); 1.71 (s, 3H); 1.88,(s, 3H); 2.16 (s, 3H); 2.21 (s, 3H); 2.23-2.33 (d, 2H); 2.41 (s, 3H); 2.63-2.78 (m, 1H); 3.32 (s, 3H); 3.78-3.91 (m, 2H); 4.07-4.20 (m, 2H); 4.23-4.32 (d, 1H); 4.59 (bs, 1H); 4.91 (s, 1H); 4.93-5.01 (d, 1H); 5.24-5.34 (dd, 1H); 5.39-5.49 (d, 1H); 5.62-5.70 (d, 1H); 6.04-6.18 (t, 1H); 6.40 (s, 1H); 7.22-7.40 (m, 5H); 7.42-7.53 (t, 2H); 7.54-7.64 (t, 1H); 8.00-8.12 (d, 2H).

Mass Spec (FAB, *m/z)* (M+H)⁺ measured at 863.3992; theory for C₄₆H₅₉O₁₄N₂ is 863.3966; 583, 523, 281, 263, 235, 205, 182, 136, 105.

### Example 50 Preparation of 13-(N-(t-butylaminocarbonyl)-2'-TES-b-phenyl isoserinyl)-baccatin III 7-O-methyl ether (57)

A 8 mL quantity Raney Nickel wetted with absolute ethanol is stirred at 0°C under nitrogen. To this is added by syringe 13-(N-(t-butylaminocarbonyl)-2'-TES-b-phenyl isoserinyl)-baccatin III 7-O-methylthiomethyl ether (54, 100 mg (0.098 mM) in 2 mL absolute ethanol. The temperature is kept at 0°C throughout the reaction and the washing process. The reaction is followed by TLC and left to go for 3 hours, when it is mostly complete. The Raney Nickel is then allowed to settle and the supernatant removed by suction. Repeating nine times, THF (40 mL) is added, stirred 2 min and removed by suction. All the washings are combined and evaporated under vacuum, leaving 60 mg solid. The crude product is chromatographed over 10g silica gel, eluting with 30-70 ethyl acetate-hexane. Fractions of 3 mL are collected, analyzing them by TLC. Fractions 15-44 are combined and evaporated under vacuum to give 13-(N-(t-butylaminocarbonyl)-2'-TEs-b-phenyl isoserinyl)-baccatin III 7-O-methyl ether (57, 56 mg) as a white solid.

TLC: silica gel; 30-70 ethyl acetate-hexane; R_{f}:0.29.

¹H NMR (CDCL₃; TMS): d 0.19-0.46 (m, 6H); 0.70-0.82 (t, 9H); 1.22 (s, 3H); 1.25 (s, 3H); 1.27 (s, 9H); 1.75 (s, 3H); 2.00 (s, 3H); 2.22 (s, 3H); 2.36-2.50 (m, 1H); 2.65 (s, 3H); 3.37 (s, 3H); 3.86-3.95 (m, 2H); 4.19-4.26 (d, 1H); 4.30-4.38 (d, 1H); 4.54-4.60 (d, 1H); 4.96-5.06 (d, 1H); 5.16 (s, 1H); 5.18 (s, 1H); 5.26-5.35 (d, 1H); 5.65-5.74 (d, 1H); 6.20-6.30 (t, 1H); 6.46 (s, 1H); 7.24-7.40 (m, 5H); 7.44-7.55 (t, 2H); 7.57-7.65 (t, 1H); 8.07-8.16 (d, 2H).

### Example 51 Preparation of 13-(N-(t-butylaminocarbonyl)-b-phenyl isoserinyl)-baccatin III 7-O-methyl ether (56)

13-(N-(t-butylaminocarbonyl)-2'-TES-b-phenyl isoserinyl)-baccatin III 7-O-methyl ether (57, 56 mg, 0.057mM) is stirred at RT under nitrogen in 2 ml 80-20 acetic acid-water. TLC after 10 minutes shows the reaction to be complete. The reaction is then freeze-dried. The crude residue is chromatographed over 7 g HPLC grade silica gel. eluting with 50 mL each of 50-50 and 60-40 ethyl acetate-hexane. Fractions of 2 mL are collected, analyzing them by TLC. Fractions 22-35 are found to contained 13-(N-(t-butylaminocarbonyl)-b-phenyl isoserinyl)-baccatin III 7-O-methyl ether (56, 38 mg) as a white solid upon evaporation.

TLC: silica gel; 60-40 ethyl acetate-hexane; R_{f}:0.42.

¹H NMR (CDCL₃; TMS): d 1.20 (s, 3H); 1.22 (s, 3H); 1.23 (s, 9H); 1.72 (s, 3H); 1.89 (s, 3H); 2.21 (s, 3H); 2.23-2.32 (d, 1H); 2.41 (s, 3H); 2.63-2.78 (m, 1H); 2.78-2.92 (m, 3H); 4.12-4.20 (d, 1H); 4.26-4.33 (d, 1H); 4.57-4.63 (m, 1H); 4.70 (s, 1H); 4.92-5.00 (d, 1H); 5.20-5.34 (m, 2H); 5.60-5.68 (d, 1H); 6.07-6.17 (t, 1H); 6.41 (s, 1H); 7.24-7.40 (m, 5H); 7.44-7.53 (t, 2H); 7.56-7.65 (t, 1H); 8.05-8.11 (d, 2H).

### Example 52 Preparation of 13-(N-Boc-2'-TES-b-phenyl isoserinyl)-baccatin III (2)

13-(2'-TES-b-phenyl isoserinyl)-baccatin III (52, 360 mg, 0.401 mM) is stirred at RT under nitrogen in 2 mL dry THF. To this is added di-t-butyldicarbonate (90 mg) dissolved in 1 mL dry THF containing .06 mL triethylamine. The reaction is allowed to proceed for 20 hours, when TLC shows it to be complete. The reaction is evaporated under vaccum and the crude residue chromatographed over 40 g silica gel, eluting with 40-60 ethyl acetate-hexane. Fractions of 15 mL are collected, analyzing them by TLC. Fractions 11-23 are found to contain 13-(N-Boc-2'-TES-b-phenyl isoserinyl)-baccatin III (2, 360 mg) as a white solid.

TLC: silica gel; 40-60 ethyl acetate-hexane; R_{f}:0.46.

¹H NMR (CDCL₃; TMS): d 0.28-0.52 (m, 6H); 0.74-0.85 (t, 9H); 1.16 (s, 3H); 1.30 (s. 3H); 1.31 (s, 9H); 1.69 (s, 3H); 1.90 (s, 3H); 2.24 (s, 3H); 2.33-2.45 (m, 1); 2.53 (s, 3H); 3.80-3.88 (d, 1H); 4.15-4.23 (d, 1H); 4.28-4.37 (d, 1H); 4.39-4.50 (m, 1H); 4.55 (s, 1H); 4.94-5.04 (d, 1H); 5.21-5.34 (bd, 1H); 5.40-5.54 (bd, 1H); 5.65-5.74 (d, 1H); 6.23-6.35 (m, 2H); 7.21-7.43 (m, 5H); 7.43-7.55 (t, 2H); 7.55-7.65 (t, 1H); 8.04-8.16 (d, 2H).

### Example 53 Preparation of 13-(N-Boc-2'-TES-b-phenyl isoserinyl)-baccatin III 7-O-methyl ether (58)

A 5 mL quantity Raney Nickel wetted with absolute ethanol is stirred at 0°C under nitrogen. To this is added by syring 13-(N-Boc-2'-TES-b-phenyl isoserinyl)-baccatin III 7-O-methylthiomethyl ether (44, 50 mg , 0.055 mM) dissolved in 2 mL absolute ethanol. The temperature is kept at 0°C throughout the reaction and the washing process. The reaction is followed by TLC, no starting material remains after 30 minutes. The Raney Nickel is allowed to settle and the the supernatant removed by suction. Repeating eleven times, absolute ethanol (10 mL) is added, stirred 2 min and removed by suction. All the washings are combined and evaporated under vacuum, leaving 33 mg solid. The crude residue is chromatographed over 5g HPLC grade silica gel, eluting with a gradient of 40-60 to 60-40 ethyl acetate-hexane. Fractions of 2 mL are collected, analyzing them by TLC. Fractions 27-40 are found to contain 13-(N-Boc-2'-TES-b-phenyl isoserinyl)-baccatin III 7-O-methyl ether (58, 21mg) as a white solid.

TLC: silica gel; 40-60 ethyl acetate-hexane; R_{f}:0.25.

¹H NMR (CDCL₃; TMS): d 1.16 (s, 6H); 1.28 (s, 9H); 1.65 (s, 3H); 1.83 (s, 3H); 2.15 (s, 3H); 2.30 (s, 3H); 2.57-2.75 (m, 1H); 3.28 (s, 3H); 3.32-3.48 (m, 1H); 3.70-3.88 (m, 2H); 4.01-4.16 (d, 1H); 4.18-4.30 (d, 1H); 4.56 (s, 1H); 4.83-4.98 (d, 1H); 5.13-5.27 (d, 1H); 5.32-5.44 (d, 1H); 5.53-5.65 (d, 1H); 6.04-6.19 (t, 1H); 6.36 (s, 1H); 7.13-7.37 (m, 5H); 7.37-7.48 (t, 2H); 7.48-7.60 (t, 1H); 7.94-8.08 (d, 2H).

Mass Spec (FAB, *m/z*) (M+H)⁺ measured at 864.3805; theory for C₄H₅₈O₁₅N₁ is 864.3806; 808, 730, 583, 541, 523, 105, 77, 57, 43.

### Preparation 1 Preparation of 7-(O-allyl)-13-(N-Boc-β-phenyl isoserinyl)-baccatin III

A solution of 13-(N-Boc-β-phenyl isoserinyl)-baccatin III (2, 1 mmol) in methylene chloride is treated with allyl trichloroacetimidate (2 mmol) and trifluoromethane sulfonic acid (25 mL) and the reaction stirred 48 h at rt. The reaction is filtered and the filtrate washed with 5% aqueous sodium bicarbonate solution. The organic layer is then dried (MgSO₄ and the solvent evaporated under vacuum. The residue is purified by chromatography over silica gel, leaving 7-(O-allyl)-13-(N-Boc-β-phenyl isoserinyl)-baccatin III.
Analogous to Kloosterman, M.; de Nijs, M. P.; van Boom, J. H. *J*. *Carbohyd. Chem.* 1986,5, 2247.

### Preparation 2 Preparation of 7-(O-allyl)-13-(N-Boc-β-phenyl isoserinyl)-baccatin III

Sodium hydride (55% dispersion in mineral oil, 43 mg, 1 mmol) is washed three times, by decantation, with anhydrous n-hexane. A solution of 13-(N-Boc-β-phenyl isoserinyl)-baccatin III (2, 1 mmol) in anhydrous DMF (6 mL) is add at 0° C and the resulting mixture stirred at rt for 30 min. The resulting mixture is then treated with allyl bromide (1.3 mmol) and stirred for an additional 60 min. The reaction is then quenched with 5% aqueous ammonium chloride solution and extracted with ether. The organic layer is dried (MgSO₄ and the solvent evaporated under vacuum. The residue is purified by chromatography over silica gel, leaving 7-(O-allyl)-13-(N-Boc-β-phenyl isoserinyl)-baccatin III.
Analogous to Lakhmiri, R.; Lhoste, P.; Sinou, D. *Tetrahedron Lett.* 1989, *30,* 4669.

### Preparation 3 Preparation of 7-(O-allyl)-13-(N-Boc-β-phenyl isoserinyl)-baccatin III

Under an argon atmosphere, tris(dibenzylidineacetone)dipalladium (0.025 mmol), and 1,4-bis(biphenylphosphino)butane (0.1 mmol) are added to tetrahydrofuran (2 mL). This solution is treated with13-(N-Boc-β-phenyl isoserinyl)-baccatin III (2, 1 mmol) and allyl ethyl carbonate in tetrahydrofuran (2 mL). After stirring at 65° C for 4 h, the solvent is evaporated under vacuum. The residue is purified by chromatography over silica gel, leaving 7-(O-allyl)-13-(N-Boc-β-phenyl isoserinyl)-baccatin III.

Following the procedure described in example 40, 41 and 42 and preparations 1, 2 and 3 but using the appropriate starting material of examples 2 and 1 the following 7-ether-taxol analogs are prepared:
7-(O-methyl)-13-(N-Boc-β-phenyl isoserinyl)-baccatin III;
7-(O-methyl)-13-(N-(t-butylaminocarbonyl)-β-phenyl isoserinyl)-baccatin III;
7-(O-ethyl)-13-(N-Boc-β-phenyl isoserinyl)-baccatin III;
7-(O-ethyl)-13-(N-(t-butylarninocarbonyl)-β-phenyl isoserinyl)-baccatin III;
7-(O-propyl)-13-(N-Boc-β-phenyl isoserinyl)-baccatin III;
7-(O-propyl)-13-(N-(t-butylaminocarbonyl)-β-phenyl isoserinyl)-baccatin III;
7-(O-allyl)-13-(N-Boc-β-phenyl isoserinyl)-baccatin III;
7-(O-allyl)-13-(N-(t-butylaminocarbonyl)-β-phenyl isoserinyl)-baccatin III;
7-(O-benzyl)-13-(N-Boc-β-phenyl isoserinyl)-baccatin III;
7-(O-benzyl)-13-(N-(t-butylaminocarbonyl)-β-phenyl isoserinyl)-baccatin III;

Taxol and the other starting taxol analogs are known or can be readily prepared by known methods. See The Chemistry of Taxol, Pharmac. Ther., Vol 52, pp 1-34, 1991 as well as:
U.S. Patent Nos. 4,814,470; 4,857,653; 4,942,184; 4,924,011; 4,924,012; 4,960,790; 5,015,744; 5,059,699; 5,136,060; 5,157,049; 4,876,399; 5,227,400, 5,254,580 as well as PCT Publication No. WO 92/09589, European Patent Application 90305845.1 (Publication No. A2 0 400 971), 89400935.6 (Publication No. A1 0 366 841) and 90402333.0 (Publication No. 0 414 610 A1), 87401669.4 (A1 0 253 739), 92308608.6 (A1 0 534 708), 92308609.4 (A1 534 709), and PCT Publication Nos. WO 91/17977, WO 91/17976, WO 91/13066, WO 91/13053 All of which are incorporated herein by reference.

The compounds of the invention can be formulated per se in pharmaceutical preparations or formulated in the form of pharmaceutically acceptable salts thereof, particularly as nontoxic pharmaceutically acceptable addition salts or acceptable basic salts. These salts can be prepared from those compounds of the invention which contain acidic or basic groups according to conventional chemical methods.

Normally, the salts are prepared by reacting the free base or acid with stoichiometric amounts or with an excess thereof of the desired salt forming inorganic or organic acid in a suitable solvent or various combination of solvents. As an example, the free base can be dissolved in an aqueous solution of the appropriate acid and the salt recovered by standard techniques, for example, by evaporation of the solution. Alternatively, the free base can be dissolved in an organic solvent such as a lower alkanoyl, an ether, an alkyl ester, or mixtures thereof, for example, methanol, ethanol, ether, ethylacetate, an ethylacetate-ether solution, and the like, whereafter it is treated with the appropriate acid to form the corresponding salt. The salt is recovered by standard recovery techniques, for example, by filtration of the desired salt on spontaneous separation from the solution or it can be precipitated by the addition of a solvent in which the salt is insoluble and recovered therefrom.

The taxol derivatives of the invention can be utilized in the treatment of cancers, due to their cytotoxic, antitumor activity. The new compounds are administrable in the form of tablets, pills, powder mixtures, capsules, injectables, solutions, suppositories, emulsions, dispersions, food premix, and in other suitable form. The pharmaceutical preparation which contains the compound is conveniently admixed with a nontoxic pharmaceutical organic carrier or a nontoxic pharmaceutical inorganic carrier, usually about 0.01 mg up to 2500 mg, or higher per dosage unit, preferably 50-500 mg. Typical of pharmaceutically acceptable carriers are, for example, mannitol, urea, dextrans, lactose, potato and maize starches, magnesium stearate, talc, vegetable oils, polyalkylene glycols, ethyl cellulose, poly(vinylpyrrolidone), calcium carbonate, ethyl oleate, isopropyl myristate, benzyl benzoate, sodium carbonate, gelatin, potassium carbonate, silicic acid, and other conventionally employed acceptable carriers. The pharmaceutical preparation may also contain nontoxic auxiliary substances such as emulsifying, preserving, wetting agents, and the like as for example, sorbitan monolaurate, triethanolamine oleate, polyoxyethylene monostearate, glyceryl tripalmitate, dioctyl sodium sulfosuccinate, and the like.

Exemplary of a typical method for preparing a tablet containing the active agents is to first mix the agent with a nontoxic binder such as gelatin, acacia mucilage, ethyl cellulose, or the like. The mixing is suitably carried out in a standard V-blender and usually under anhydrous conditions. Next, the just prepared mixture can be slugged through conventional tablet machines and the slugs fabricated into tablets. The freshly prepared tablets can be coated, or they can be left uncoated. Representative of suitable coatings are the nontoxic coatings including shellac, methylcellulose, carnauba wax, styrene-maleic acid copolymers, and the like. For oral administration, compressed tablets containing 0.01 milligram, 5 milligrams, 25 milligrams, 50 milligrams, 500 milligrams, etc., up to 2500 milligrams are manufactured in the light of the above disclosure and by art known fabrication techniques well known to the art and set forth in Remington's Pharmaceutical Science, Chapter 39, Mack Publishing Co., 1965.

To formulate the tablet, the active compound, cornstarch, lactose, dicalcium phosphate and calcium carbonate are uniformly blended under dry conditions in a conventional V-blender until all the ingredients are uniformly mixed together. Next, the cornstarch paste is prepared as a 10% paste and it is blended with the just prepared mixture until a uniform mixture is obtained. The mixture is then passed through a standard light mesh screen, dried in an anhydrous atmosphere and then blended with calcium stearate, and compressed into tablets, and coated if desired. Other tablets containing 10, 50, 100, 150 mgs, etc., are prepared in a like fashion.

The following Formulation I is an example of a tablet formulation comprising a compound of the invention.

| FORMULATION I | |
|---|---|
| Ingredients: | Per tablet, mg. |
| Active compound | 50.0 |
| Cornstarch | 15.0 |
| Cornstarch paste | 4.5 |
| Calcium carbonate | 15.0 |
| Lactose | 67.0 |
| Calcium stearate | 2.0 |
| Dicalcium phosphate | 50.0 |

The manufacture of capsules containing 10 milligrams to 2500 milligrams for oral use consists essentially of mixing the active compound with a nontoxic carrier and enclosing the mixture in a polymeric sheath, usually gelatin or the like. The capsules can be in the art known soft form of a capsule made by enclosing the compound in intimate dispersion within an edible, compatible carrier, or the capsule can be a hard capsule consisting essentially of the novel compound mixed with a nontoxic solid such as talc, calcium stearate, calcium carbonate, or the like. Capsules containing 25 mg, 75 mg, 125 mg, and the like, of the novel compound, singularly or mixtures of two or more of the novel compounds are prepared, for example, as follows:

| FORMULATION II | |
|---|---|
| Ingredients | Per Capsule, mg. |
| Active compound | 50.0 |
| Calcium carbonate | 100.0 |
| Lactose, U.S.P. | 200.0 |
| Starch | 130.0 |
| Magnesium stearate | 4.5 |

The above ingredients are blended together in a standard blender and then discharged into commercially available capsules. When higher concentrations of the active agent is used, a corresponding reduction is made in the amount of lactose.

The compounds of the invention can also be freeze dried and, if desired, combined with other pharmaceutically acceptable excipients to prepare formulations suitable for parenteral, injectable administration. For such administration, the formulation can be reconstituted in water (normal, saline), or a mixture of water and an organic solvent, such as propylene glycol, ethanol, and the like.

The dose administered, whether a single dose, multiple dose, or a daily dose, will of course, vary with the particular compound of the invention employed because of the varying potency of the compound, the chosen route of administration, the size of the recipient and the nature of the patient's condition. The dosage administered is not subject to definite bounds, but it will usually be an effective amount, or the equivalent on a molar basis of the pharmacologically active free form produced from a dosage formulation upon the metabolic release of the active drug to achieve its desired pharmacological and physiological effects.

Typically the compounds of the invention can be administered by intravenous injection at doses of 1-500 mg per patient per course of treatment, preferable with doses of 2-100 mg, the exact dosage being dependent on the age, weight, and condition of the patient. An example of a suitable formulation for injection is using a solution of the compound of the invention in a mixture of polysorbate alcohol and dehydrated alcohol (e.g., 1:1) followed by dilution with 5% dextrose in water prior to infusion or injection.

The compounds of Formula I are useful for the same cancers for which taxol has been shown active, including human ovarian tumors, mammary tumors, and malignant melanoma, lung tumors, gastric tumors, colon tumors, head and neck tumors, and leukemia. See, e.g., the clinical pharmacology of taxol is reviewed by Eric K. Rowinsky and Ross C. Donehower, The Clinical Pharmacology and Use of Antimicrotubule Agents in Cancer Chemotherapeutics, Pharmac. Ther., Vol 52, pp 35-84, 1991. Clinical and preclinical studies with taxol are reviewed by William J. Slichenmyer and Daniel D. Von Hoff, Taxol: A New and Effective Anticancer Drug, Anti-Cancer Drugs, Vol. 2, pp 519-530, 1991.

The biological activity of the 7-deoxy-7-ether-taxol compounds (Formula I) of the invention has been confirmed using well known procedures. For example, comparison of the cytotoxicity of Cpd 8 with taxol itself in L1210 mouse leukemia carcinoma cells in culture indicated that the IC₉₀ (90% growth inhibitory concentration) for 7-(O-methoxymethyl)-13-(N-Boc-2'-β-phenyl isoserinyl)-baccatin III (8) was 0.0011 micrograms/ml and for taxol was 0.017 micrograms/ml. In an *in vitro* tubulin polymerization assay, conducted after the manner of F. Gaskin, et al., J. Mol. Biol., 89:737, 1974, 7-(O-ethoxymethyl)-13-(N-Boc-β-phenyl isoserinyl)-baccatin III (4) and 7-(O-methoxymethyl)-13-(N-(t-butylaminocarbonyl)-β-phenyl isoserinyl)-baccatin III (27) were was able to induce tubulin polymerization *in vitro* at 20°C in a manner very similar to taxol.

The biological activity of the compounds of this invention has been further confirmed using well known procedures against L1210 leukemia and the results set forth in Table I. The results were obtained using standard well known procedure (Li, L.H.; Kuentzel, S.L.; Murch, L.L.; Pschigoga, L.M.; and W.C. Krueger, "Comparative biological and biochemical effects of nogalamycin and its analogs on L1210 leukemia," Cancer Res. 39:4816-4822 (1979)). The results are expressed as an IC₅₀ which is the drug concentration required to inhibit cell proliferation to 50% of that of untreated control cells. Lower numbers indicated greater activity.

**TABLE I**

| Compound | L1210 (IC₅₀ *u*g/ml) |
|---|---|
| taxol | 0.017 |
| taxotere | 0.004 |
| 6 | 0.0023 |
| 8 | 0.0011 |
| 10 | 0.001 |
| 14 | 0.0014 |
| 21 | 0.0024 |
| 22 | 0.0047 |

The biological activity of the compounds of this invention has been further confirmed using well known procedures against A2780 human ovarian carcinoma and the results set forth in Table II. The results were obtained using standard well known procedure (Perez, R. P.; O'Dwyer, P. J.; Handel, L. M.; Ozols, R. F.; Hamilton, T. C. Int. J. Cancer 1991, 48, 265, Alley, M.C.; Scudiero, D. A.; Monks, A.; Hursey, M. L; Czerwinski, M. J.; Fine, D. L.; et al. Cancer Res 1988, 48, 589).

**TABLE II**

| Compound | A2780 (IC₅₀ *u*g/ml) |
|---|---|
| taxol | 0.0018 |
| taxotere | 0.0007 |
| 4 | 0.0007 |
| 14 | 0.00007 |
| 27 | 0.00004 |
| 42 | 0.00047 |
| 44 | 0.00037 |
| 46 | 0.0016 |
| 47 | 0.0007 0.00044 (retest) |
| 49 | 0.0057 |
| 55 | 0.00025 |
| 56 | 0.00034 |
| 58 | 0.00038 |

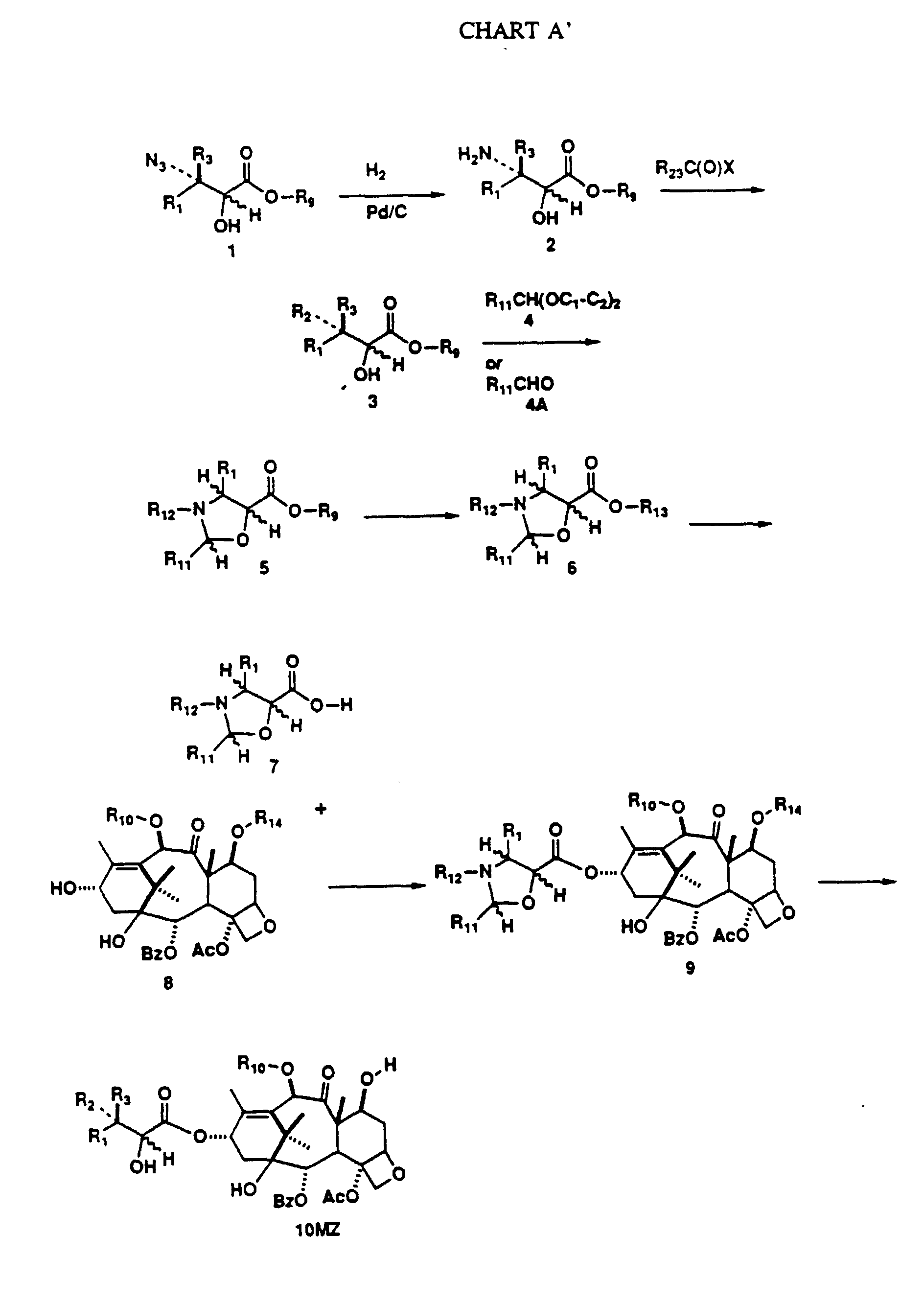

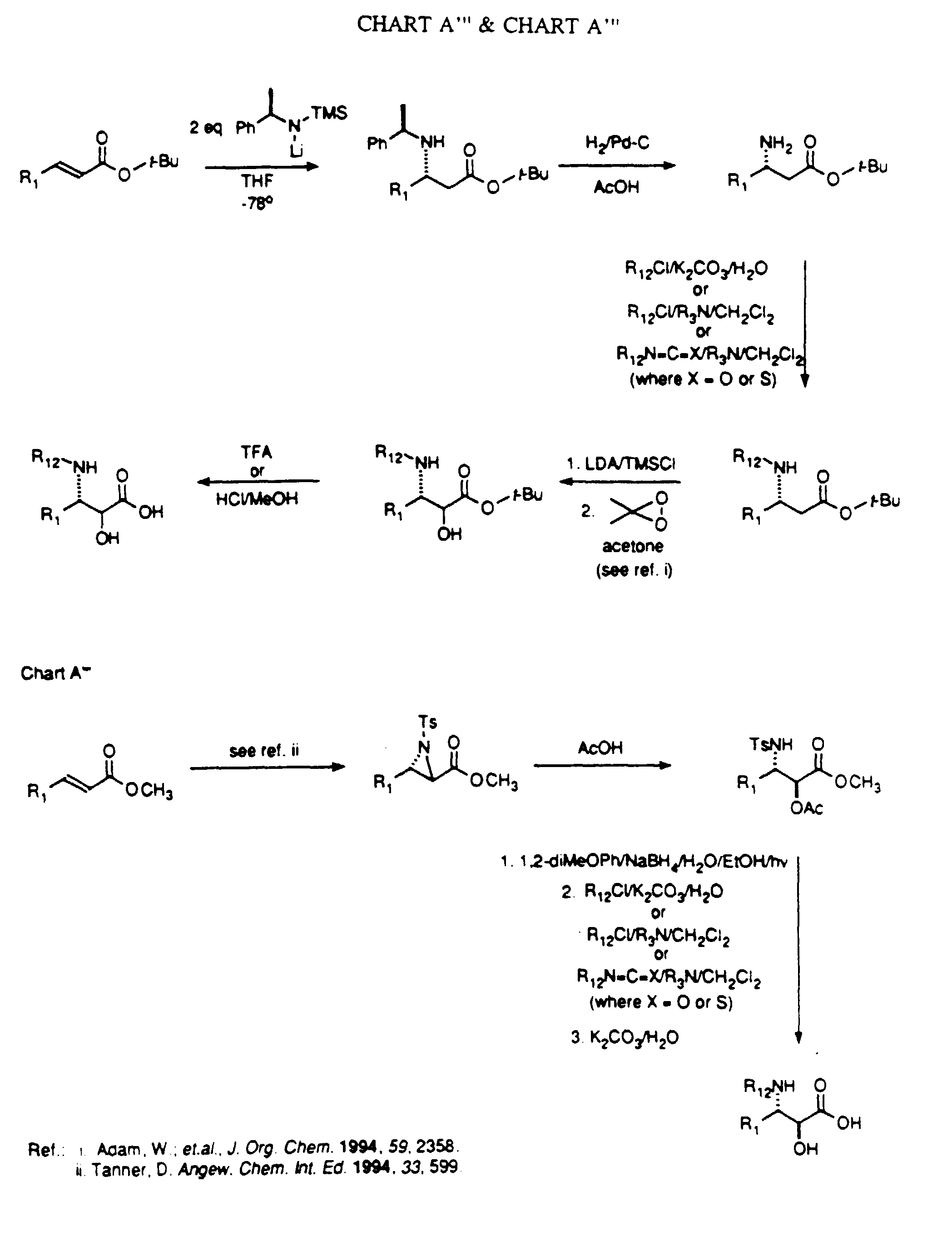

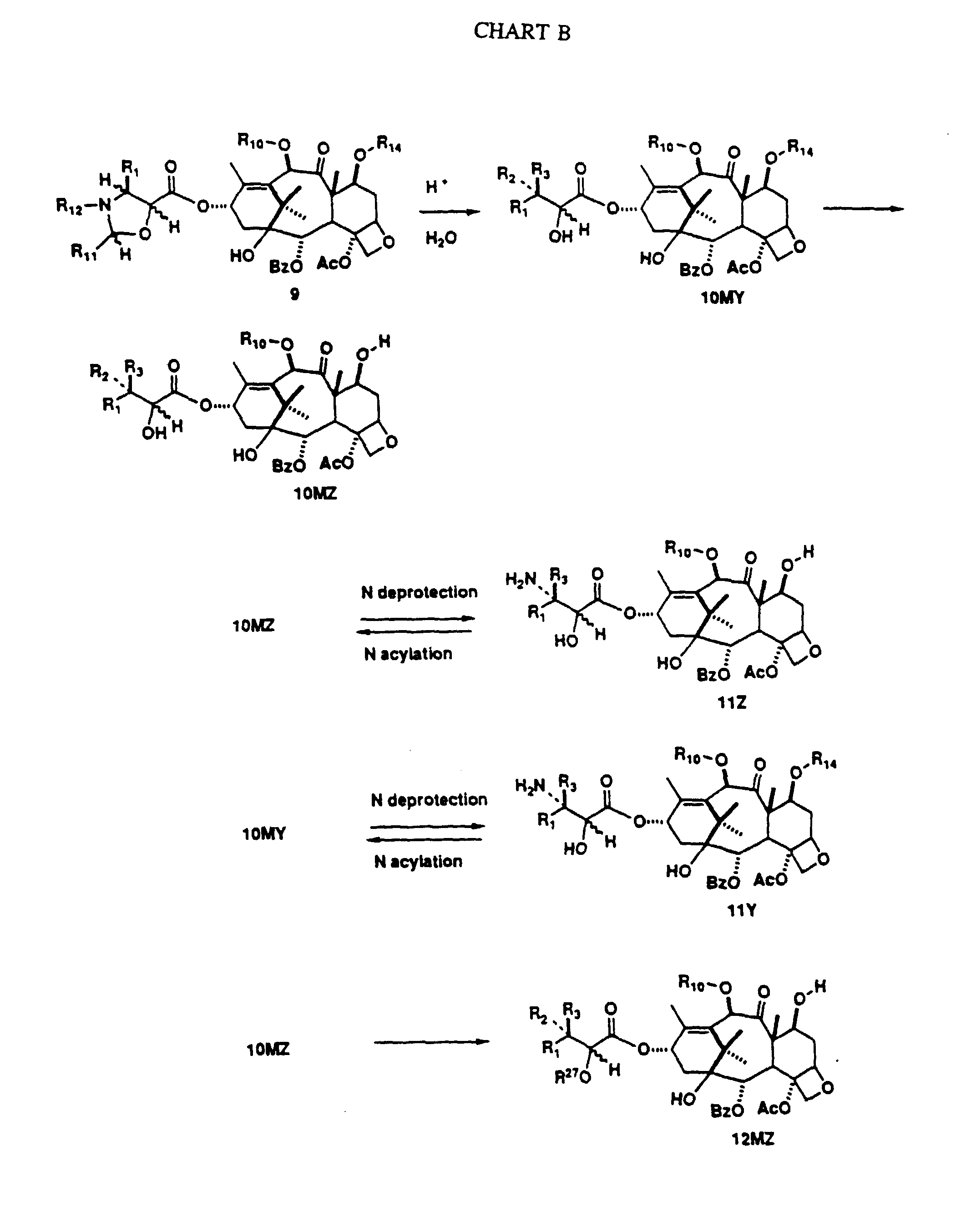

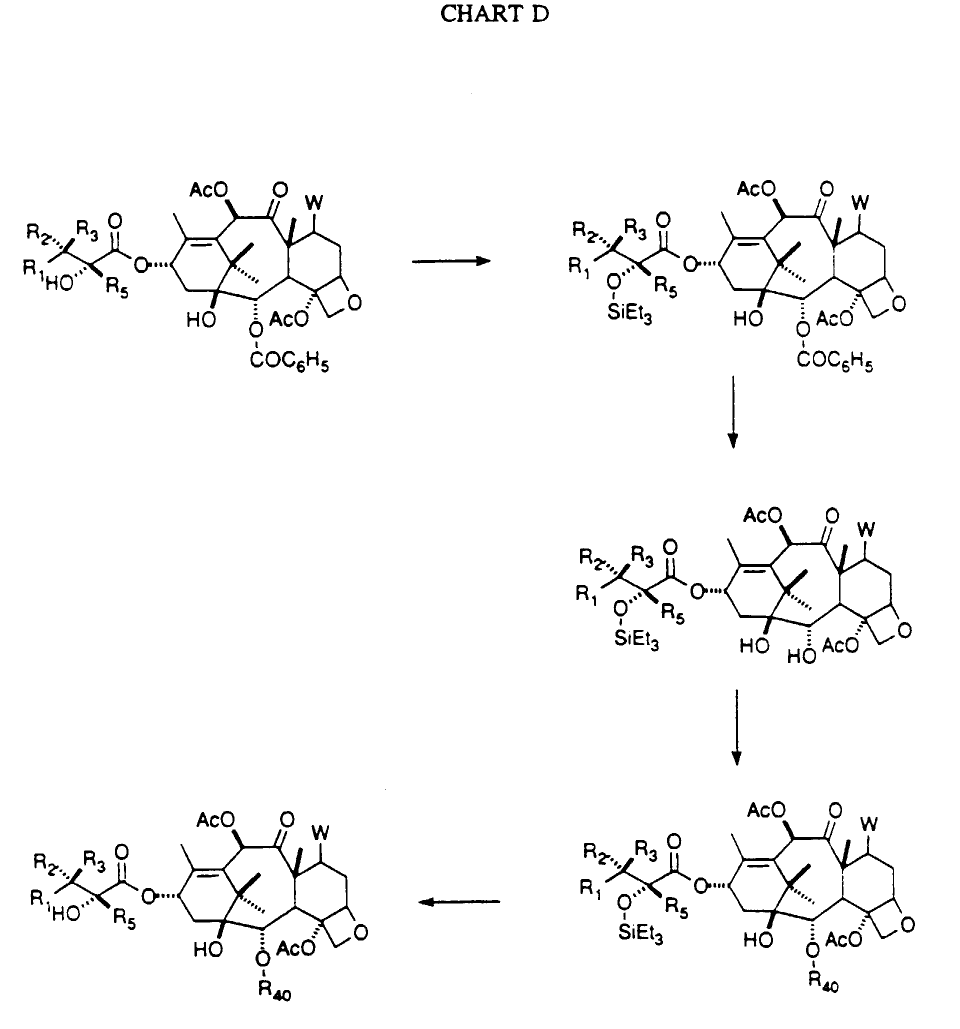

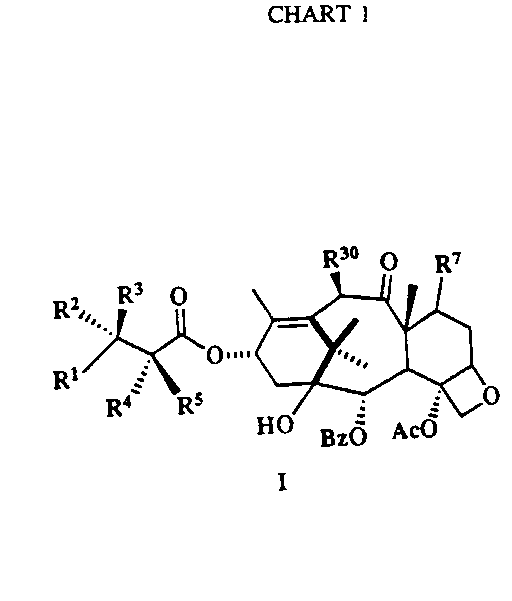

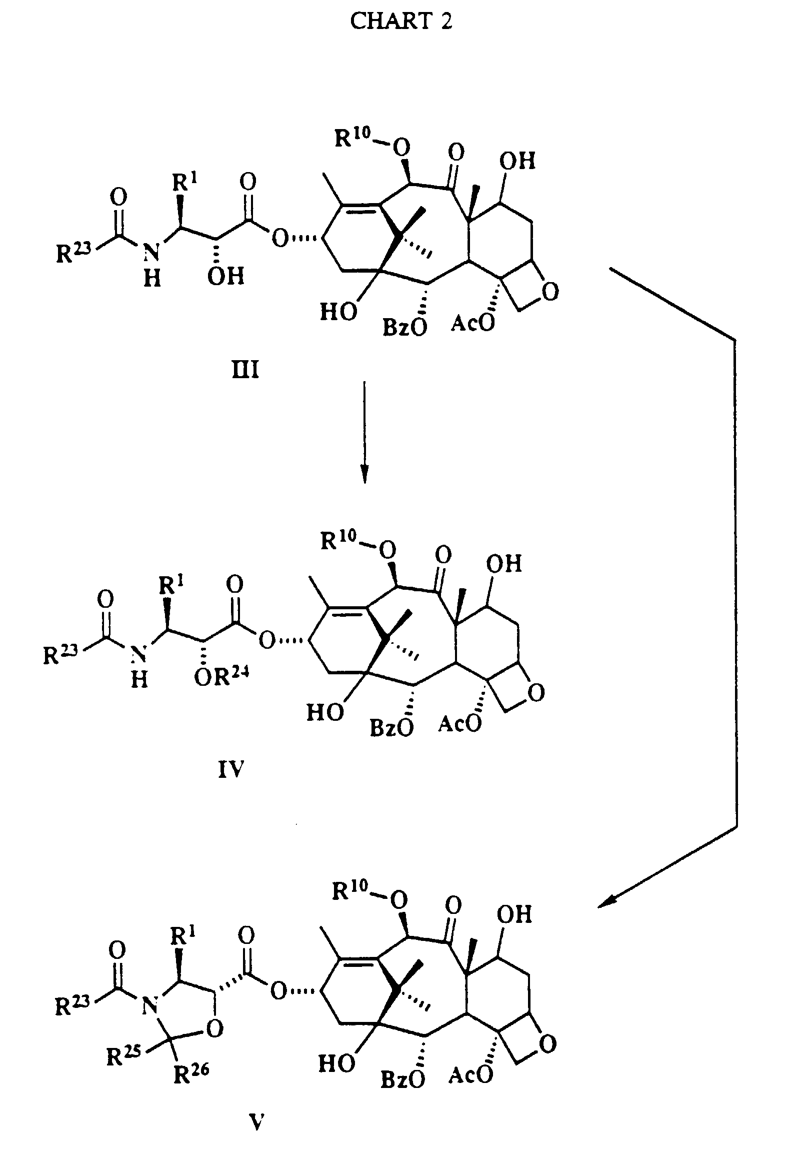

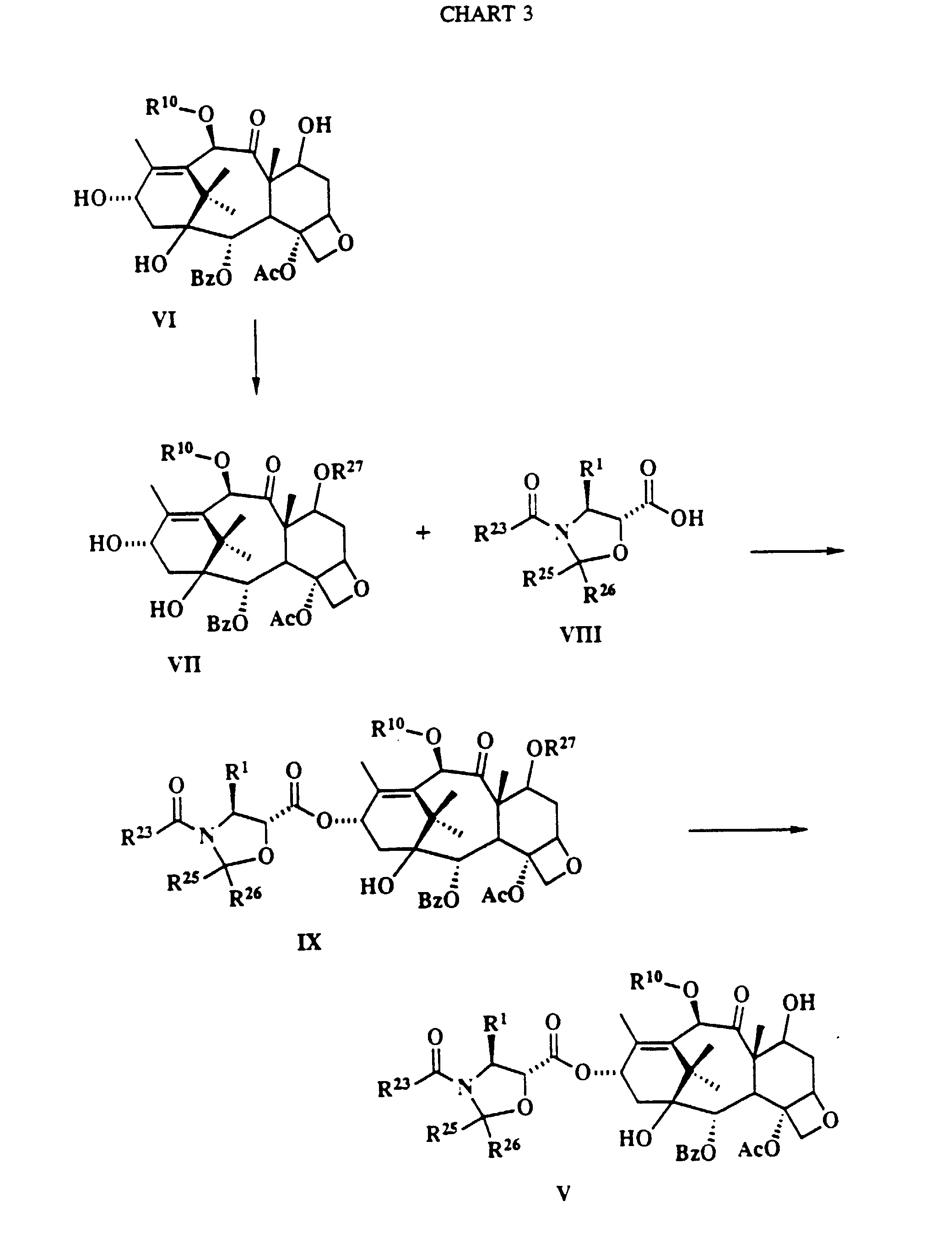

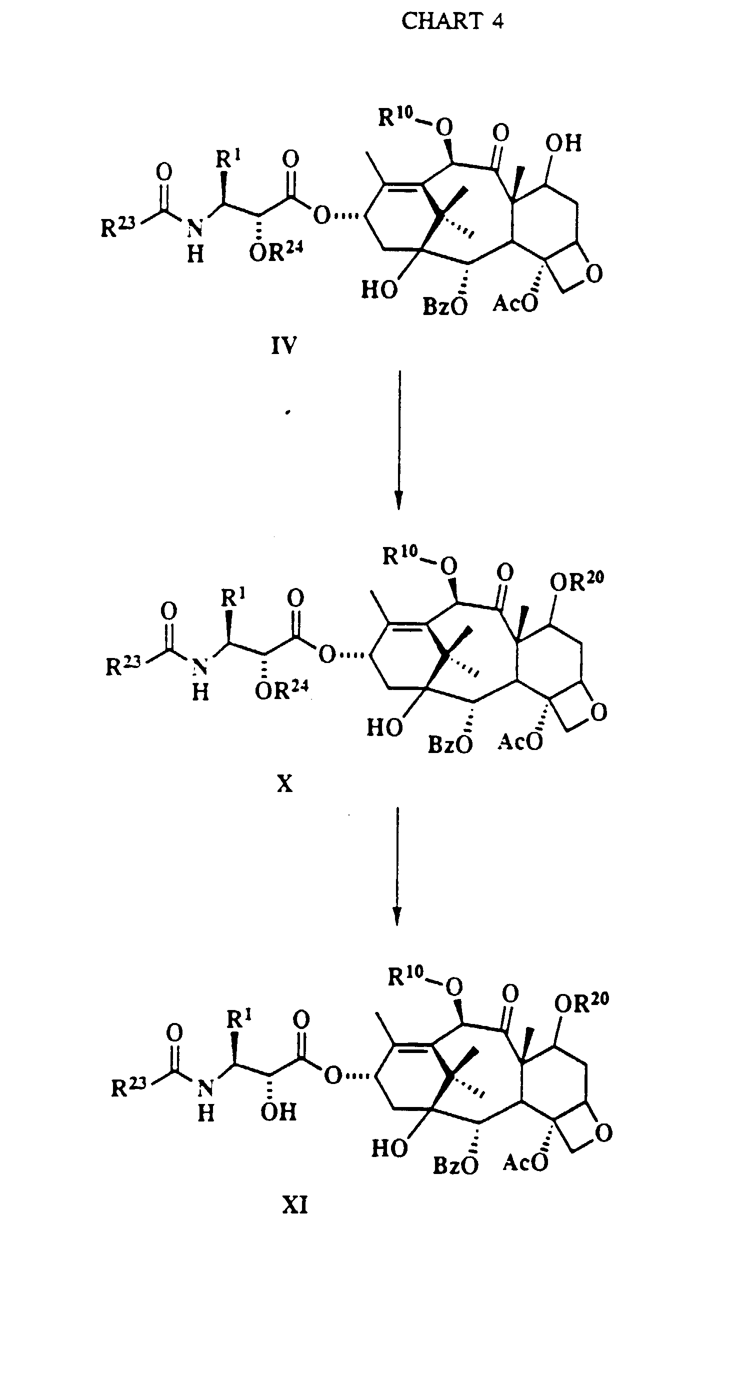

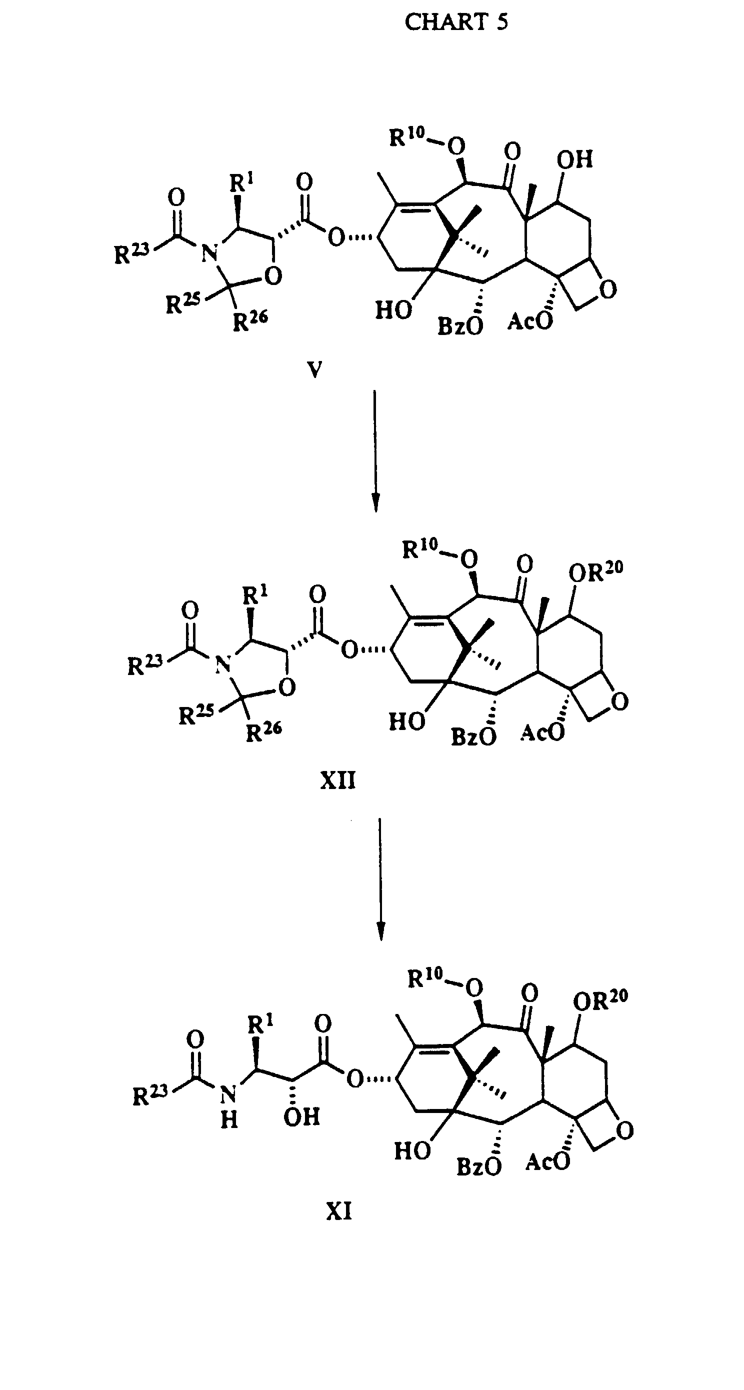

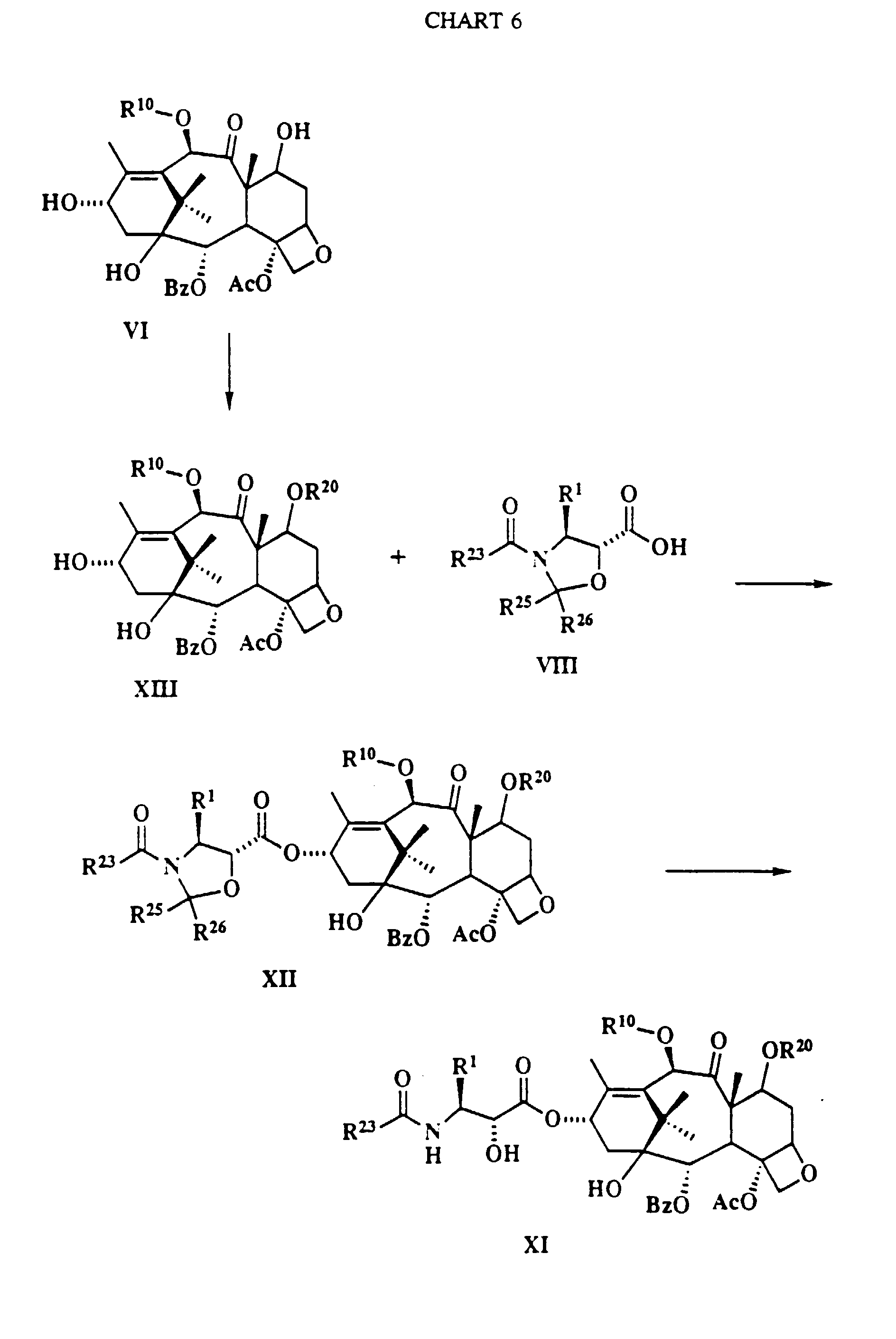

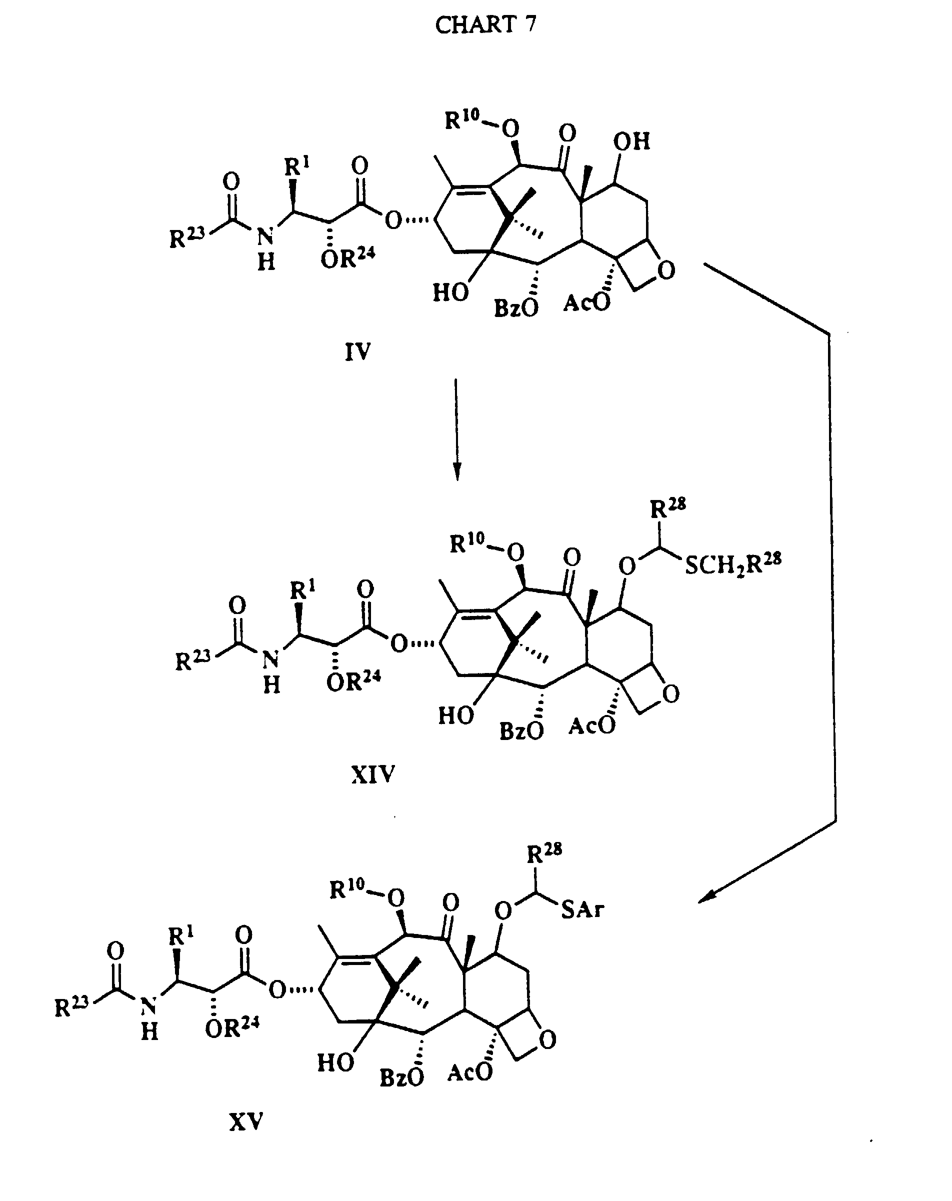

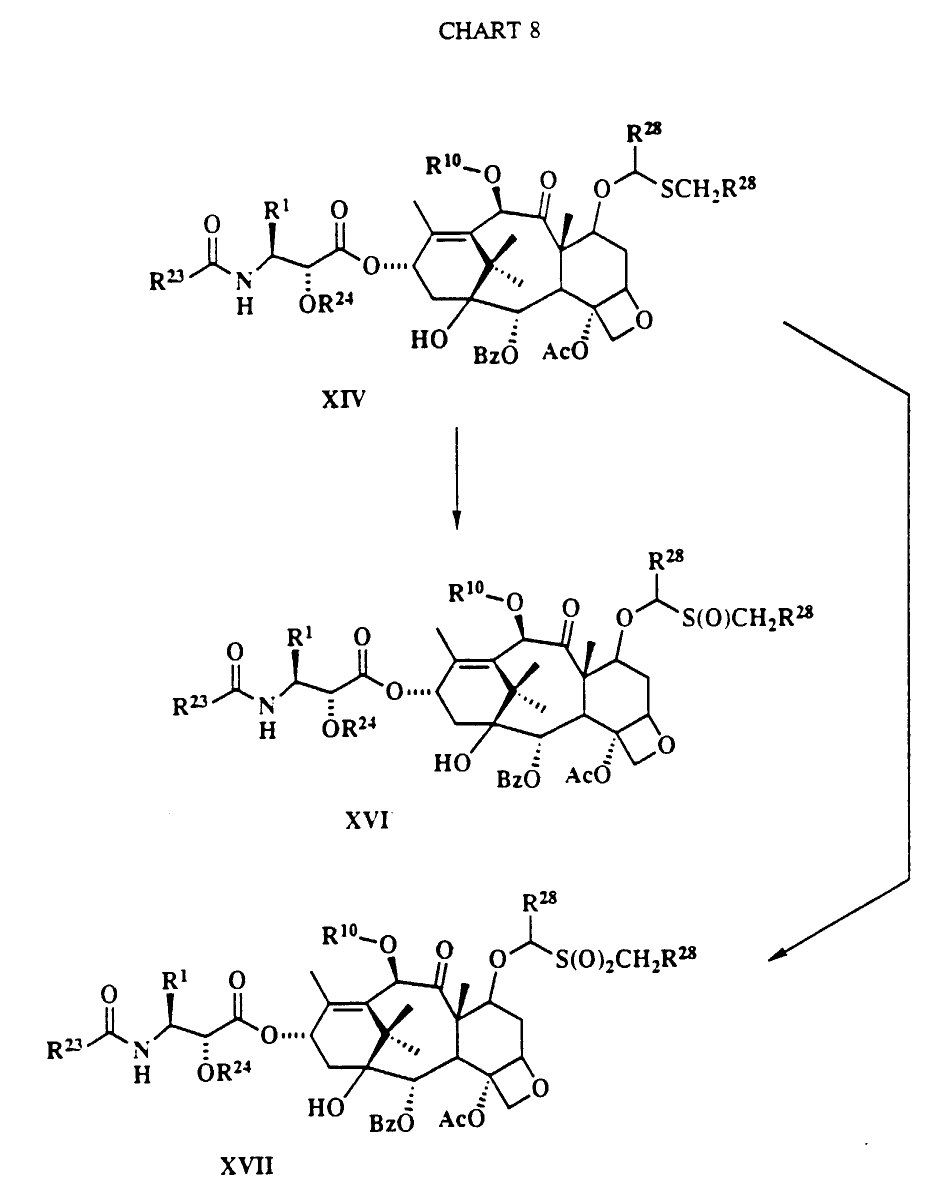

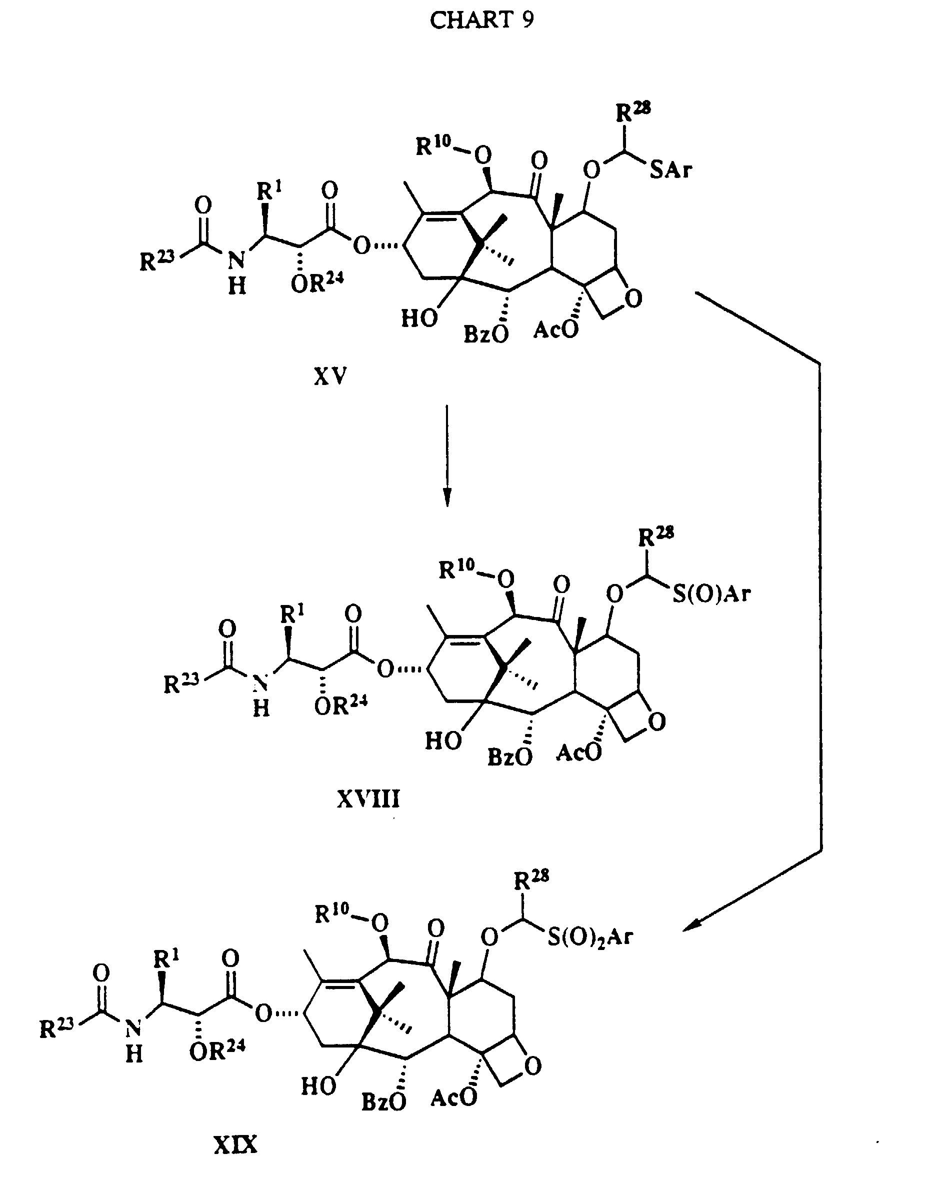

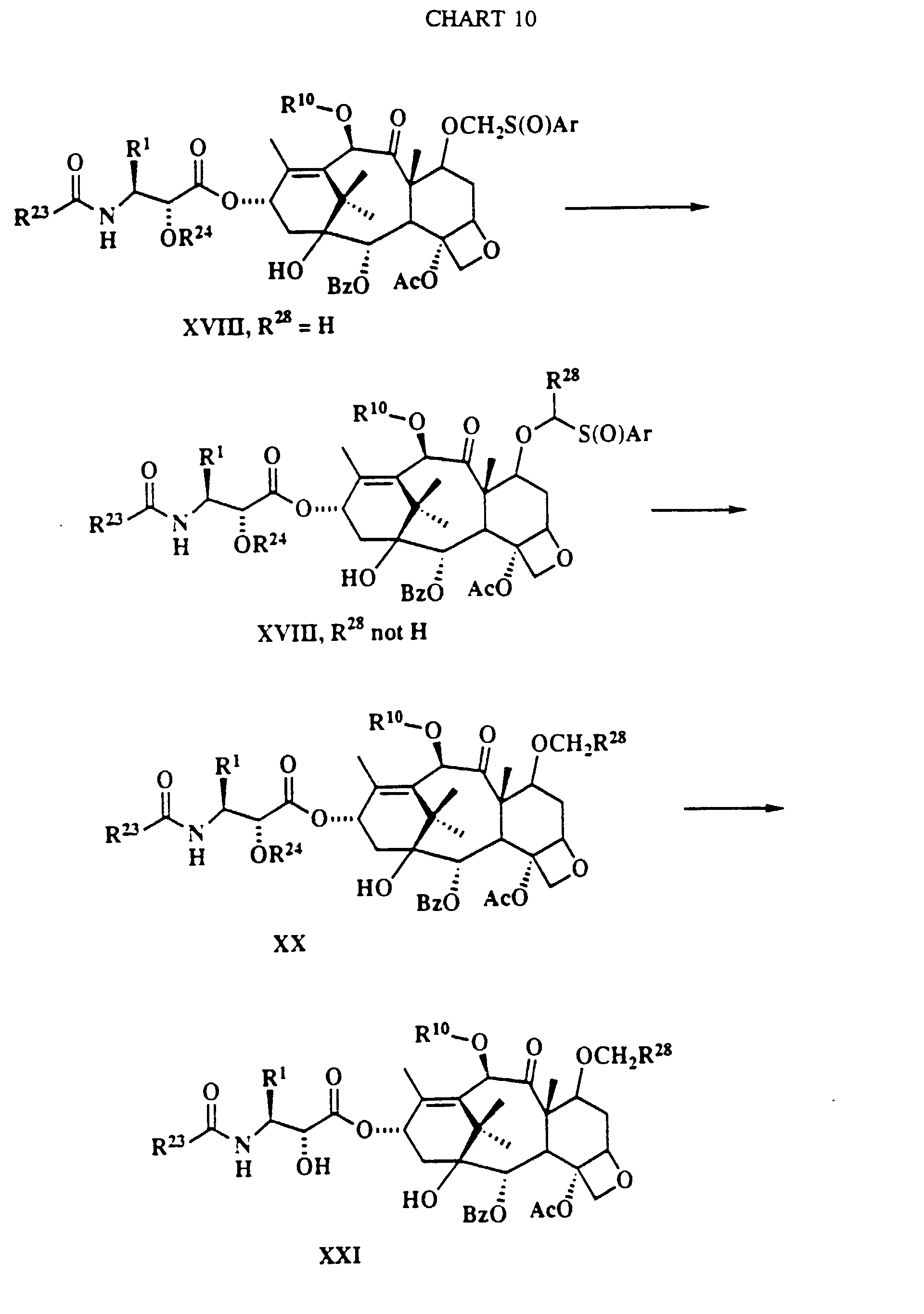

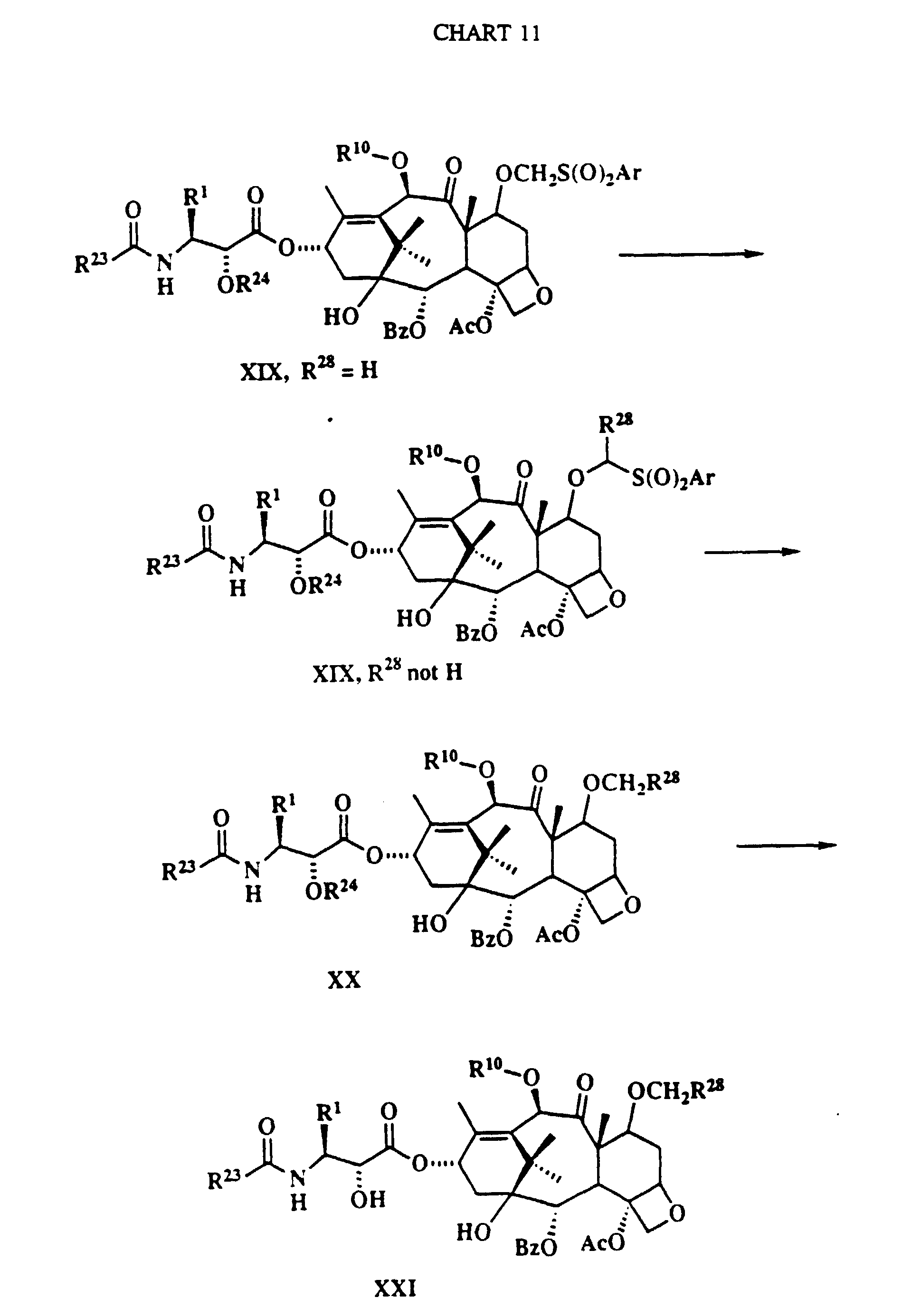

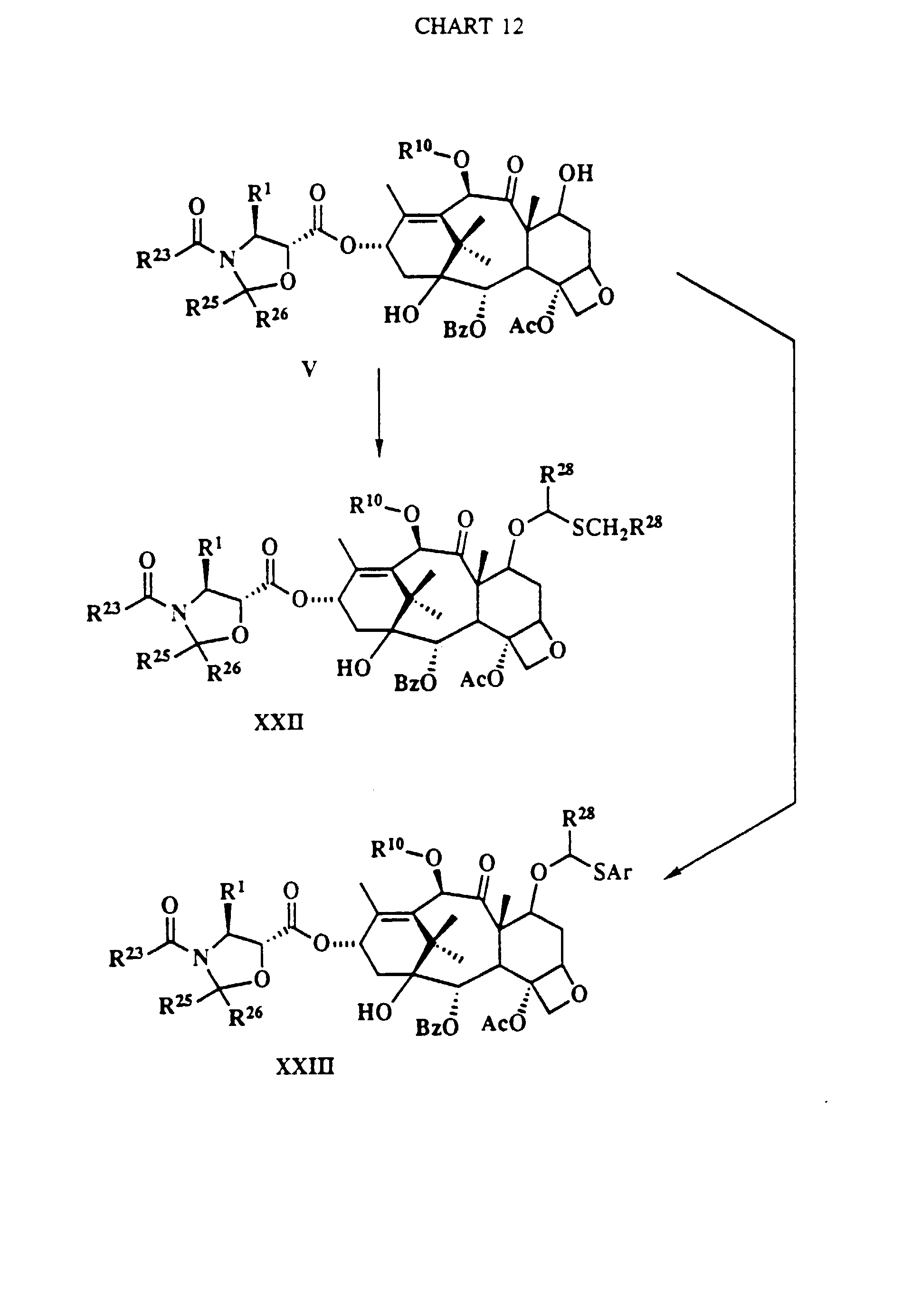

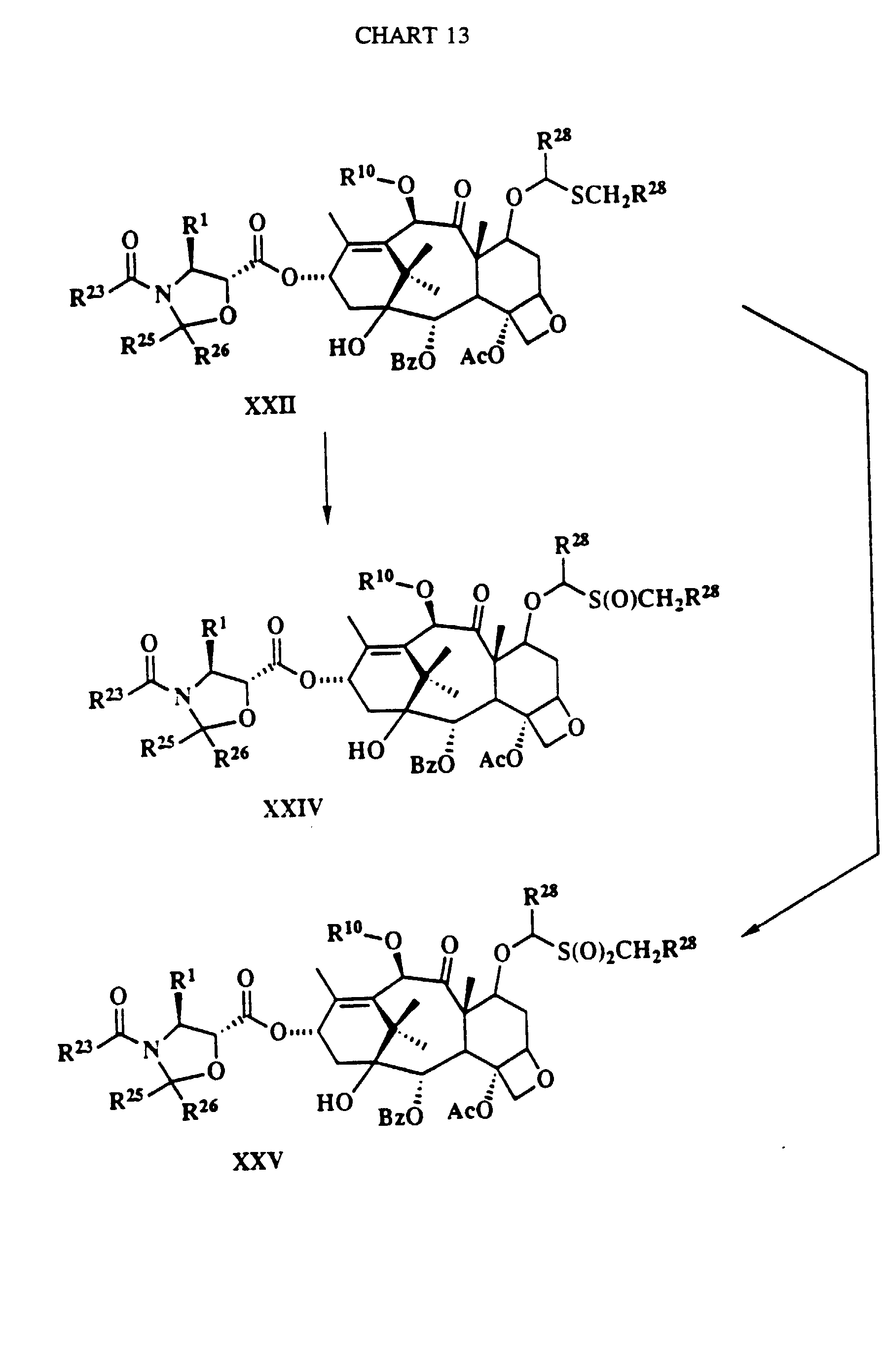

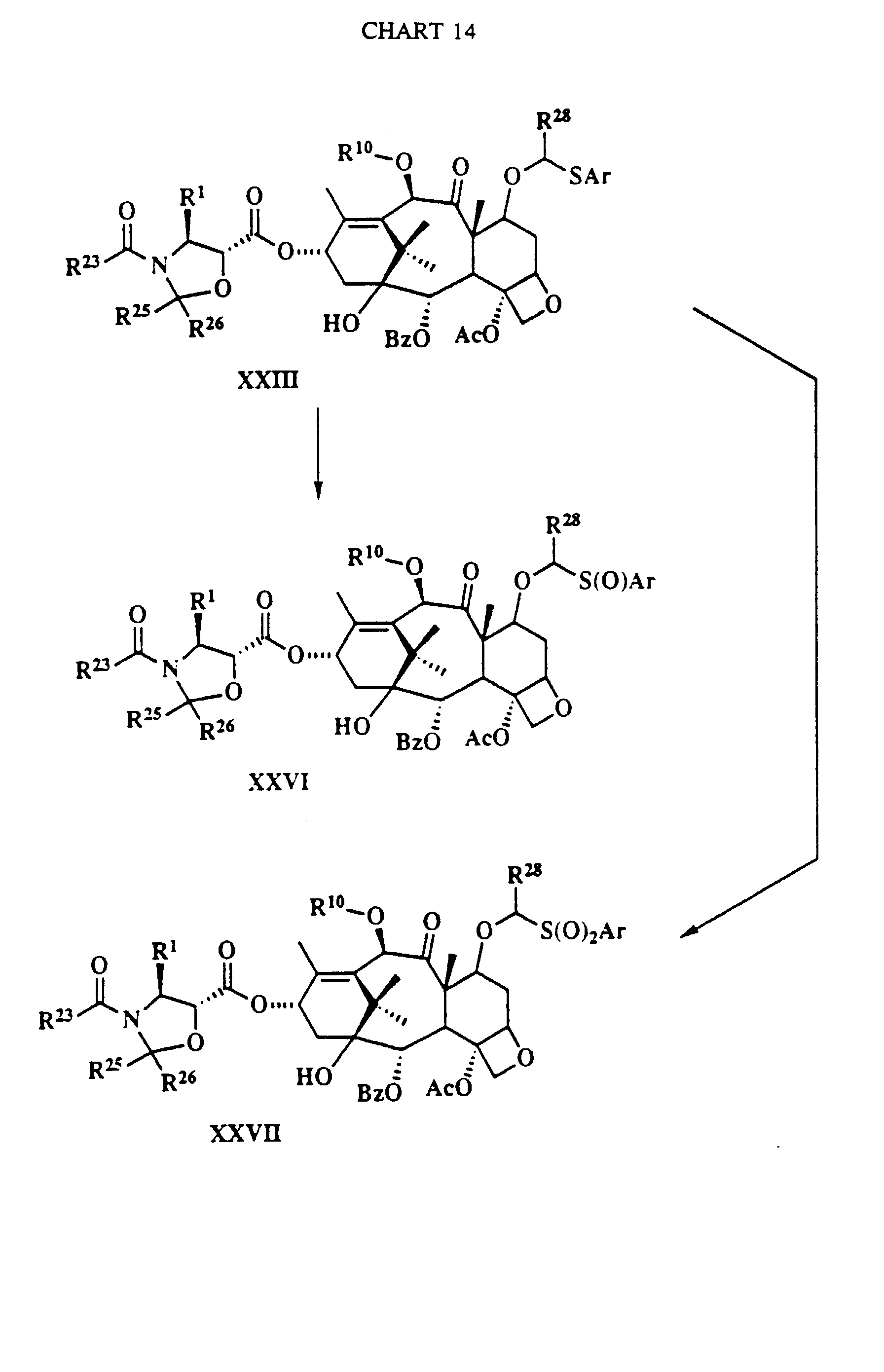

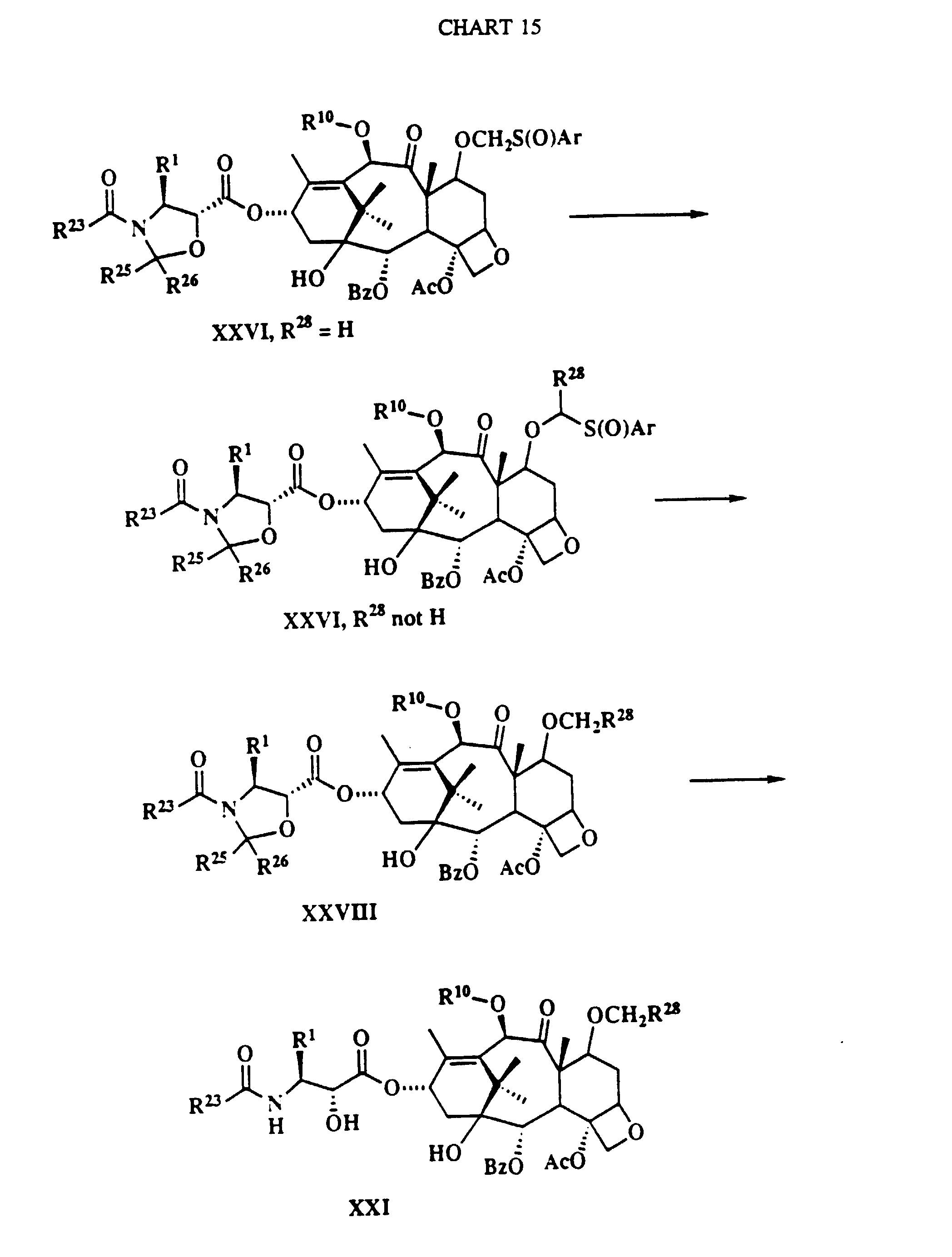

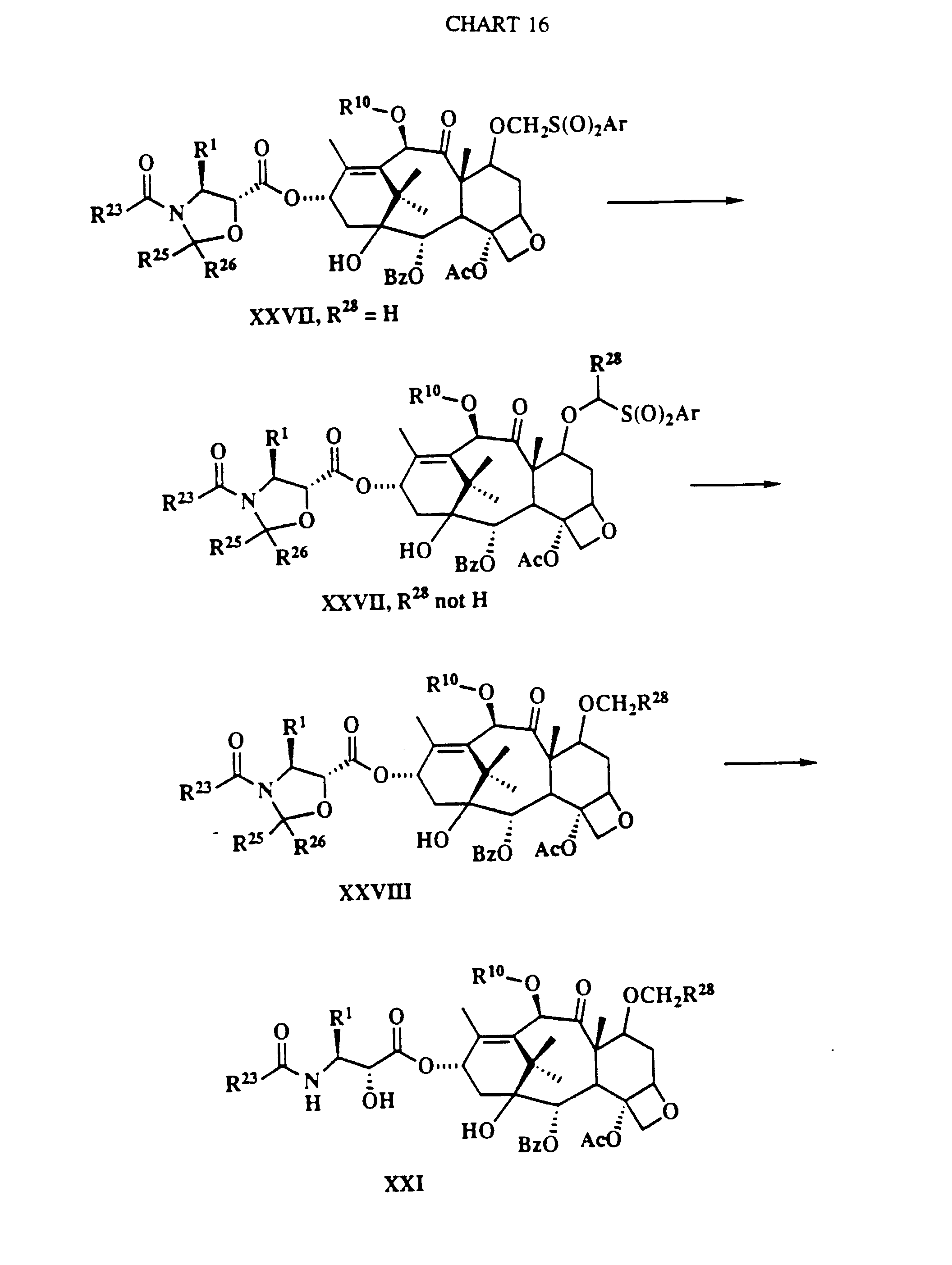

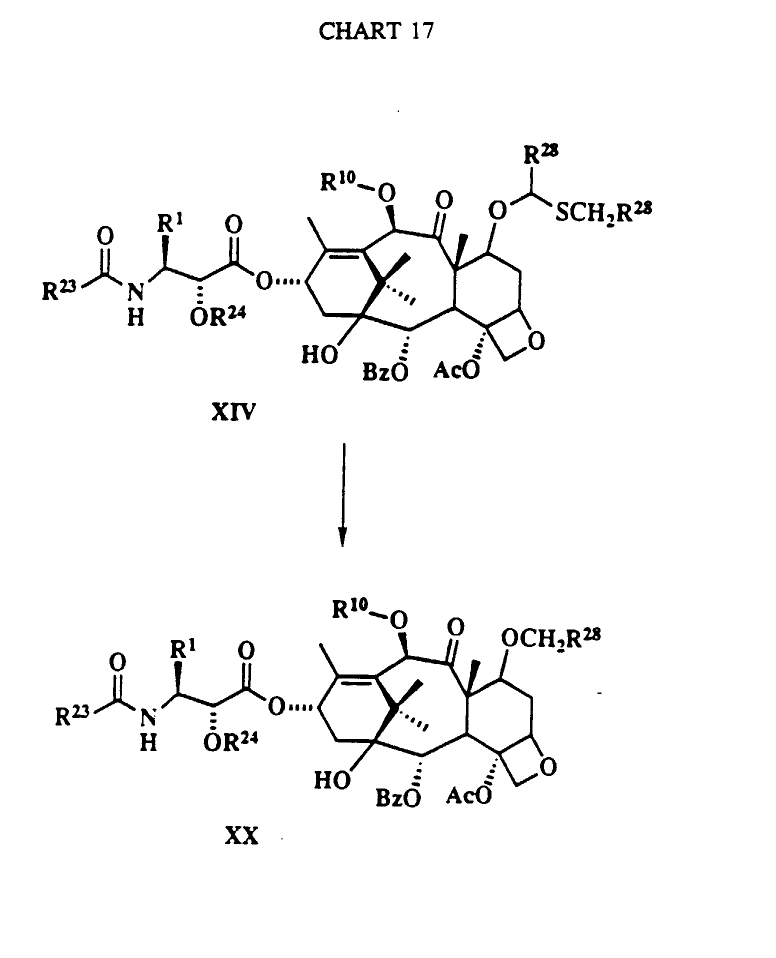

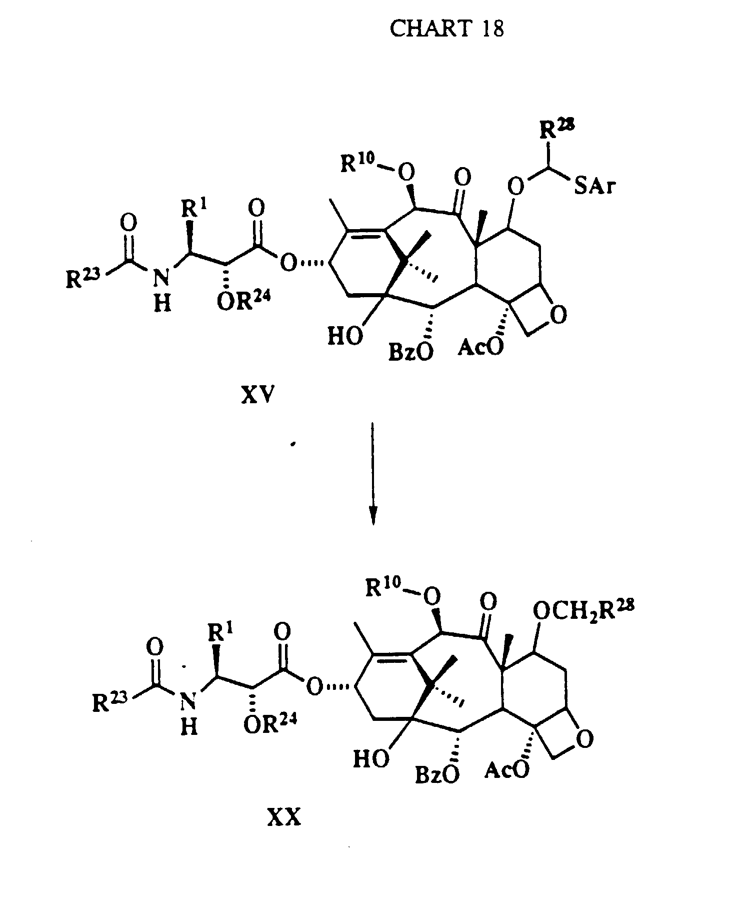

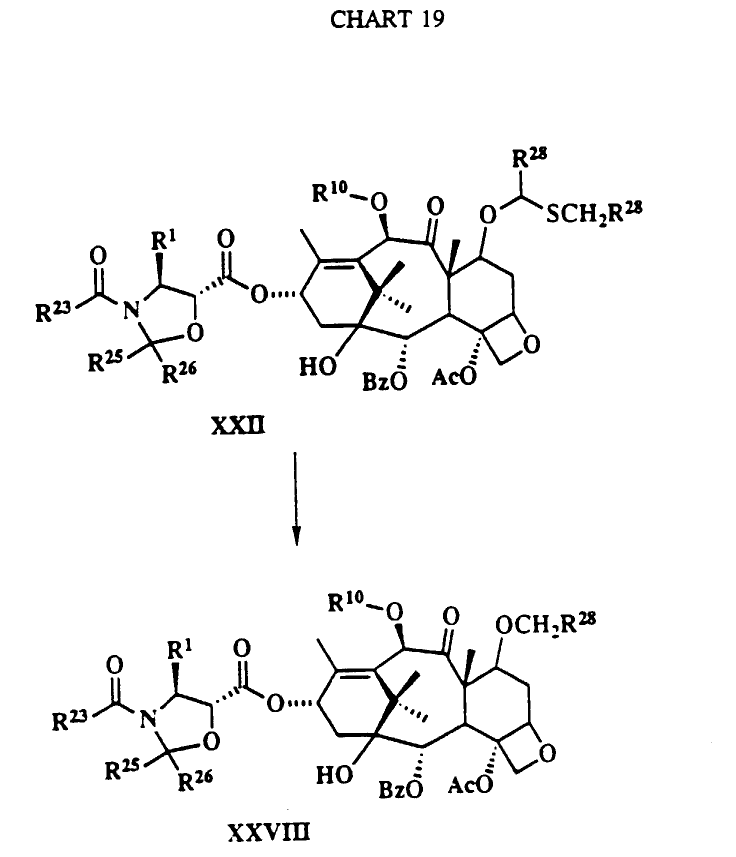

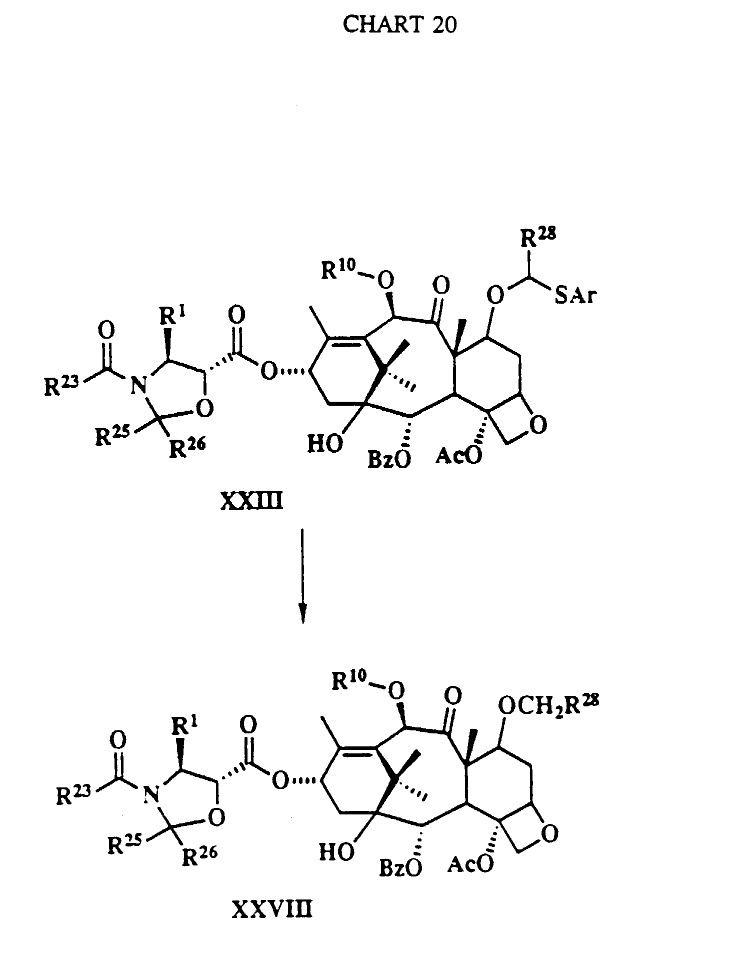

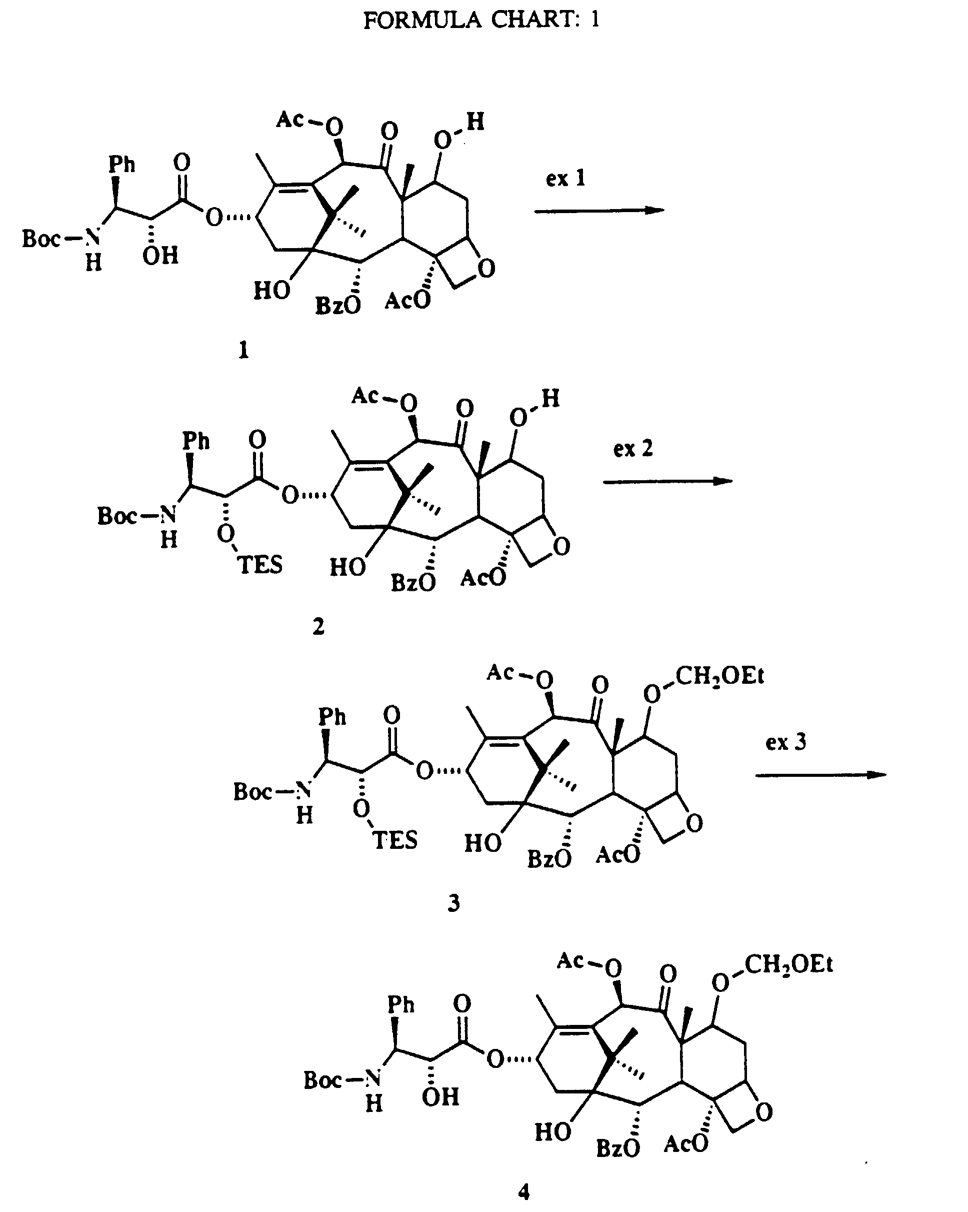

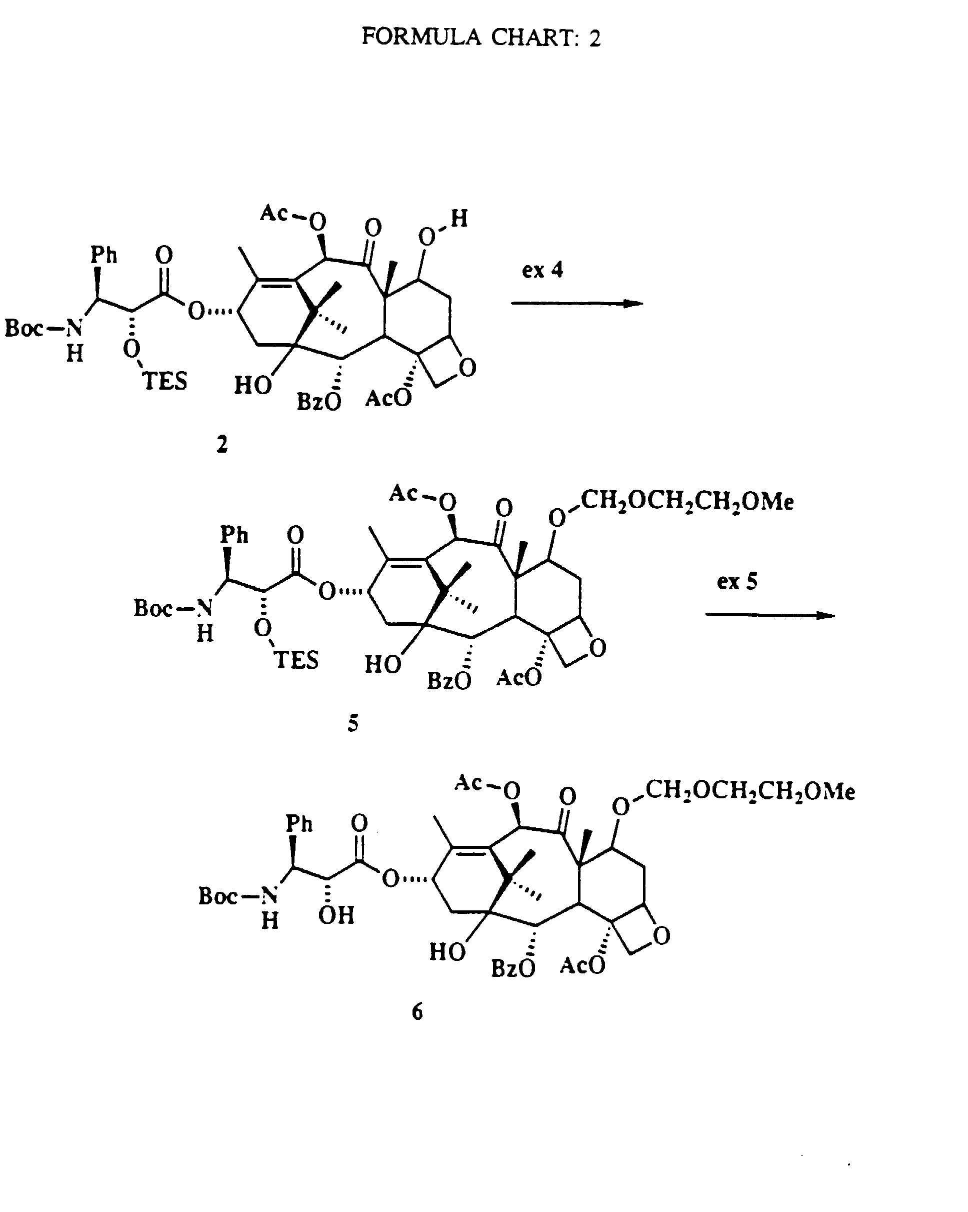

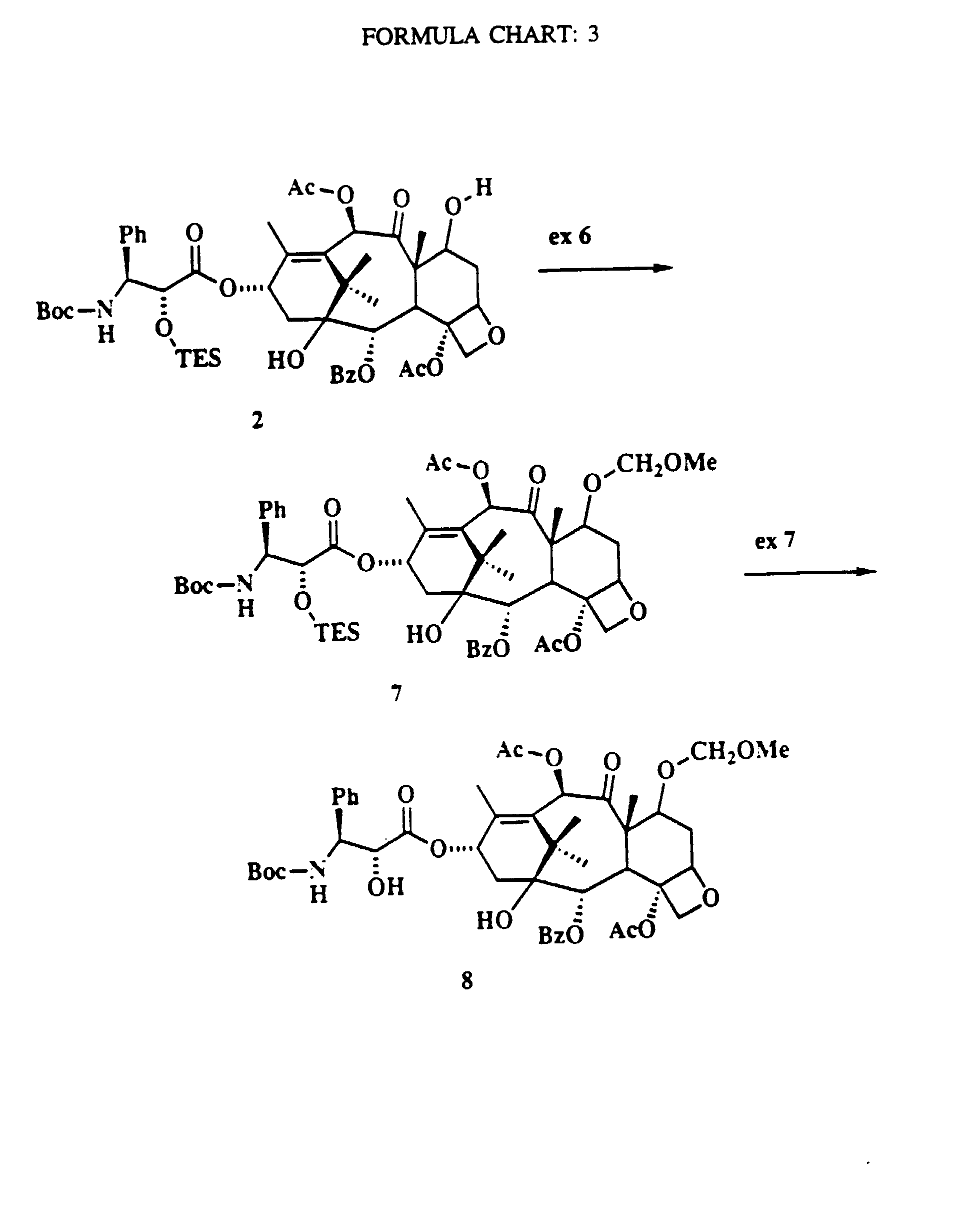

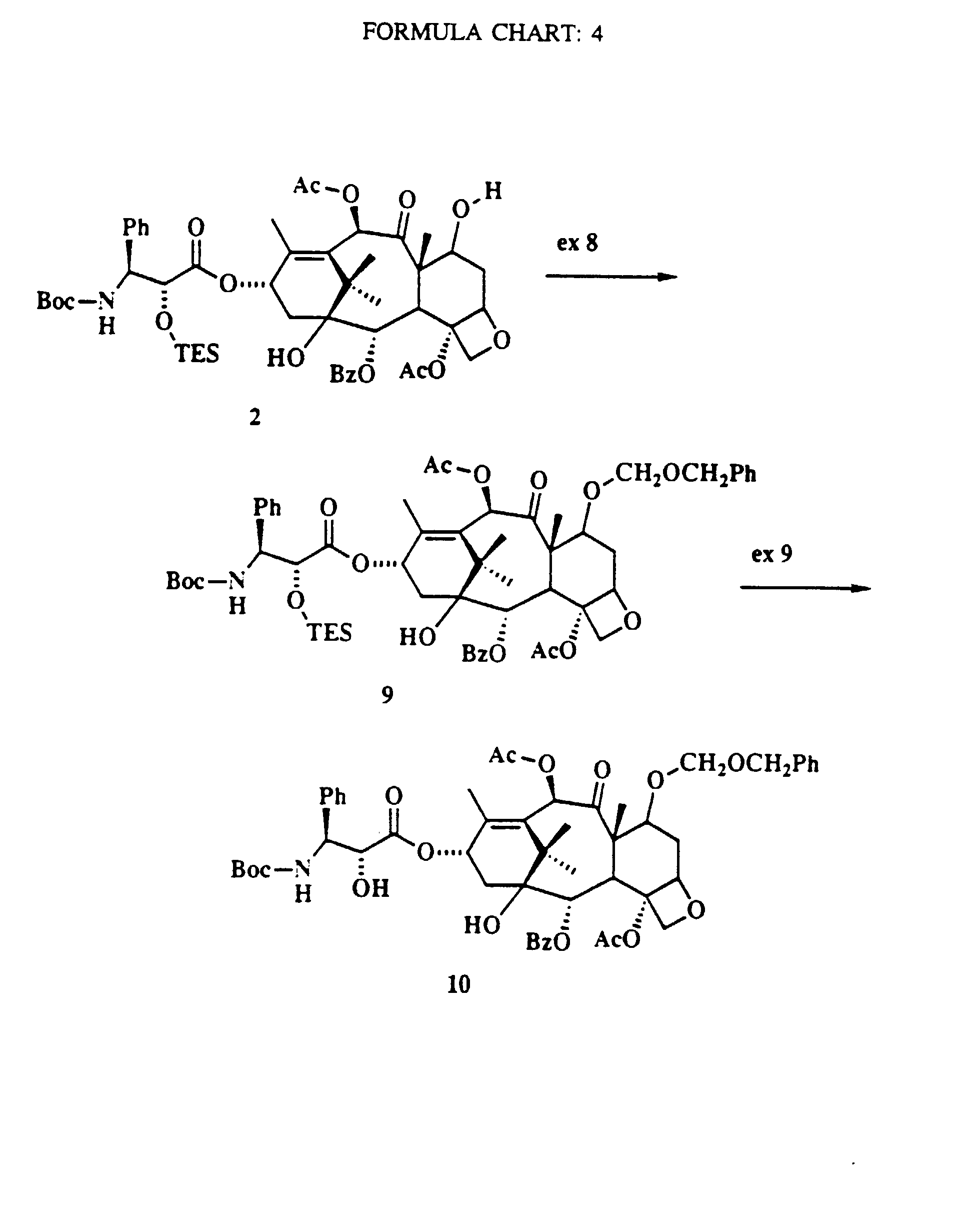

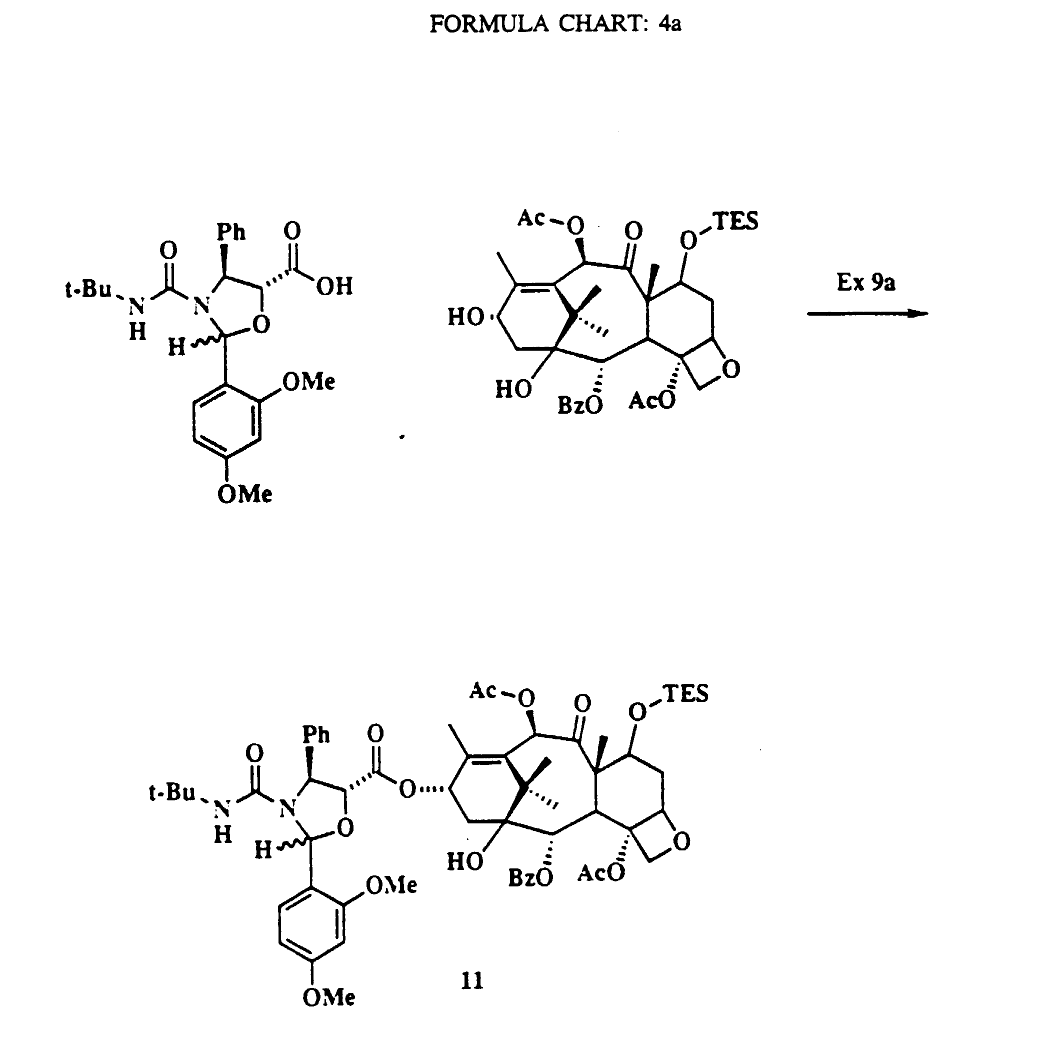

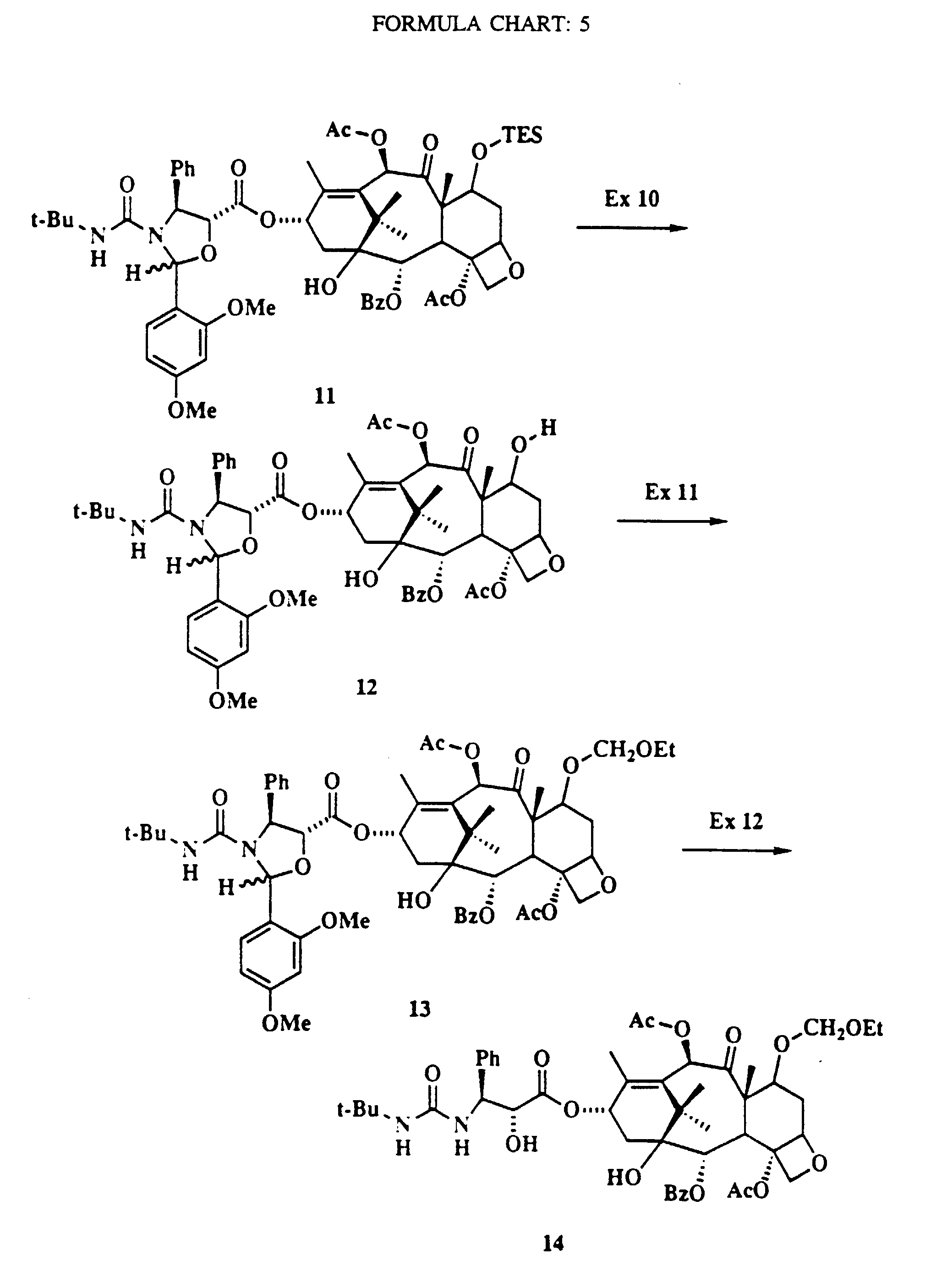

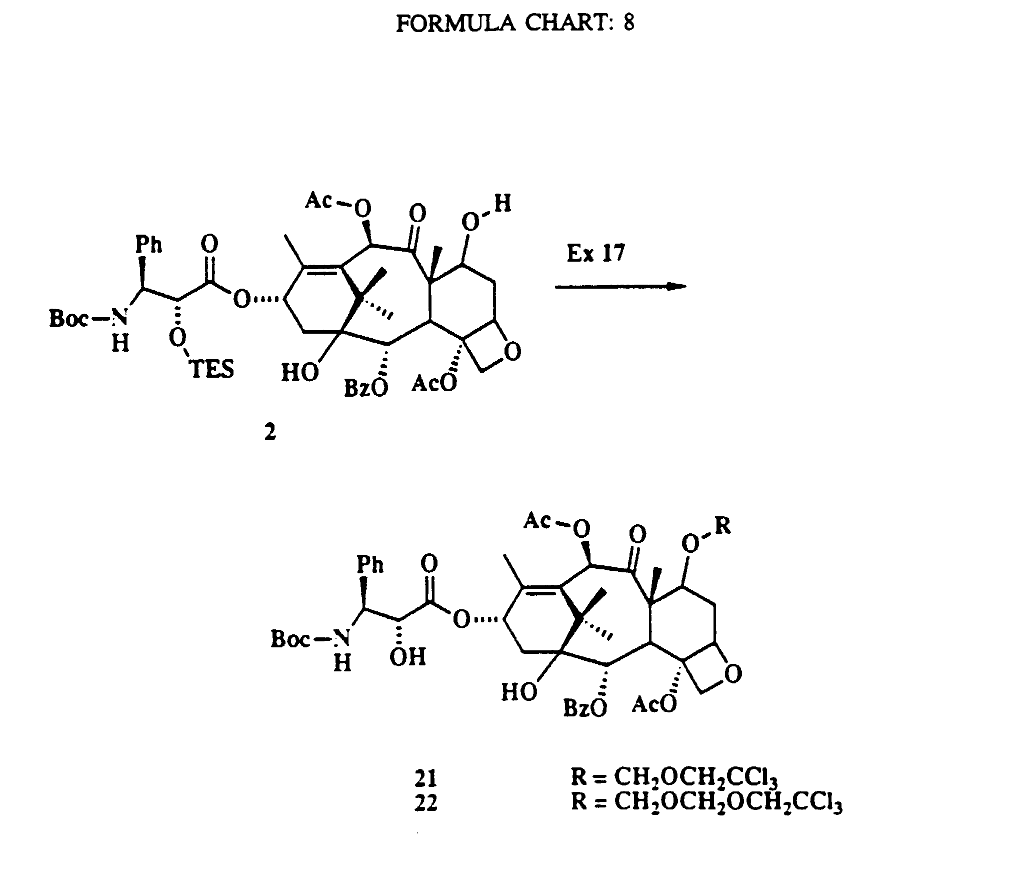

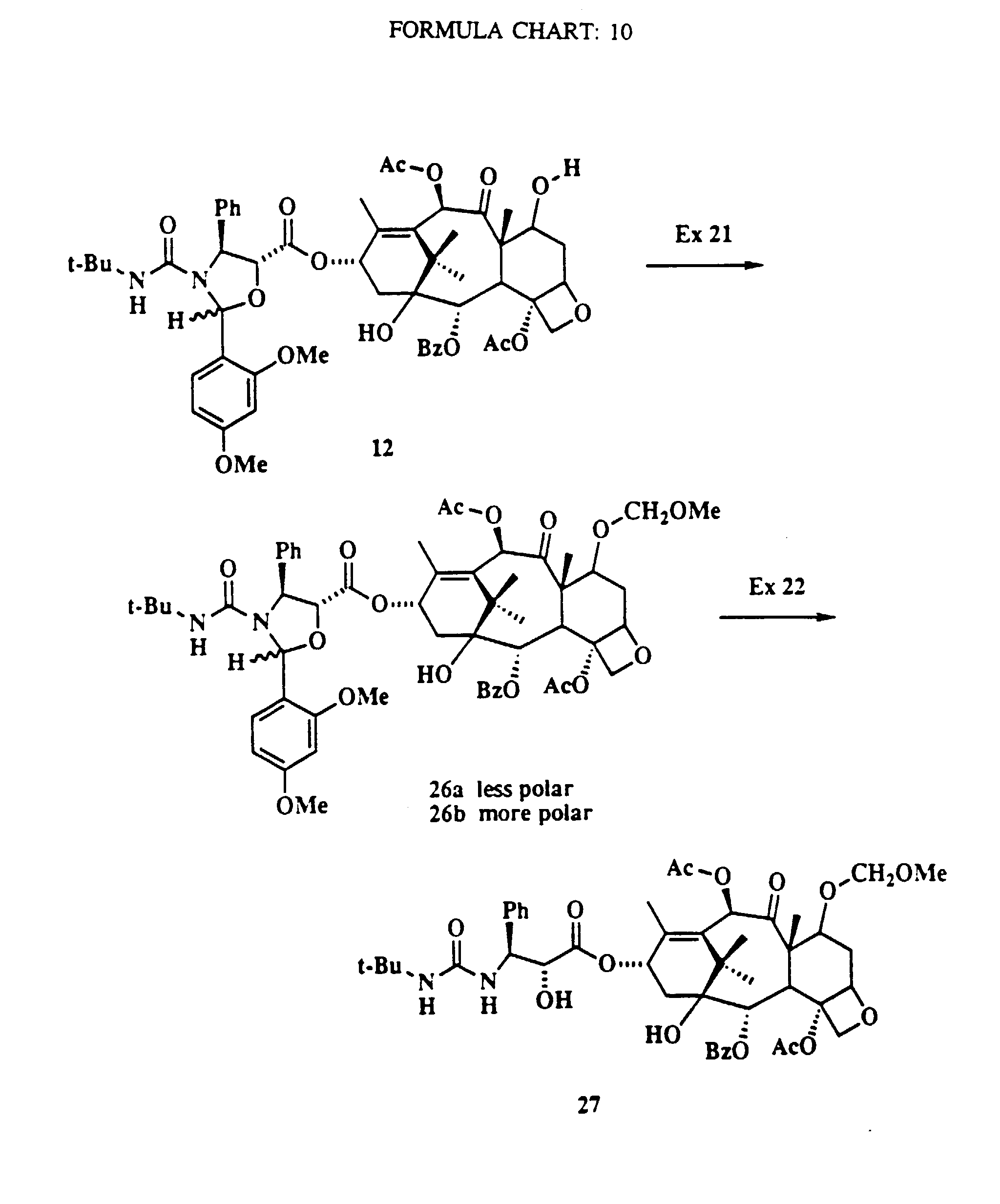

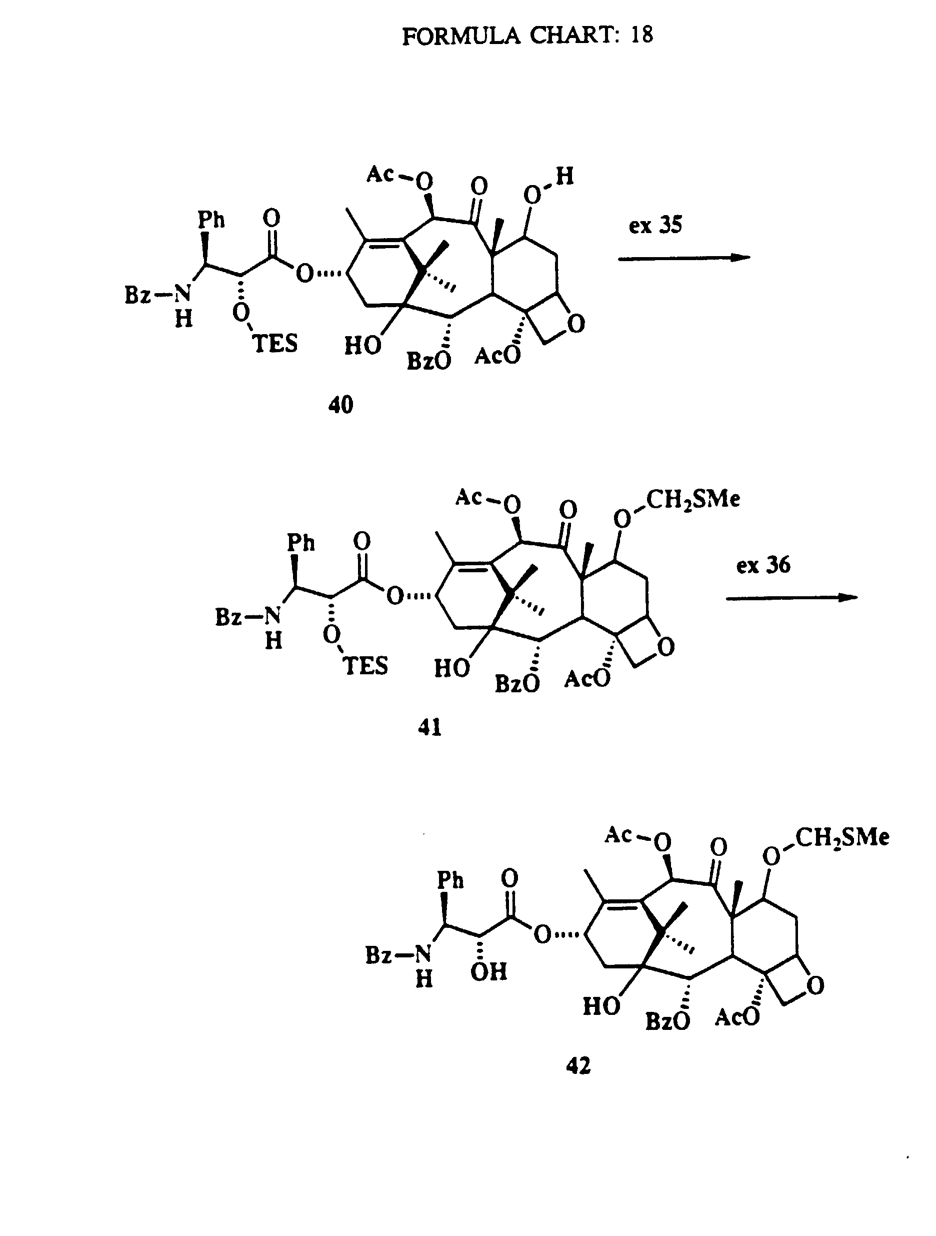

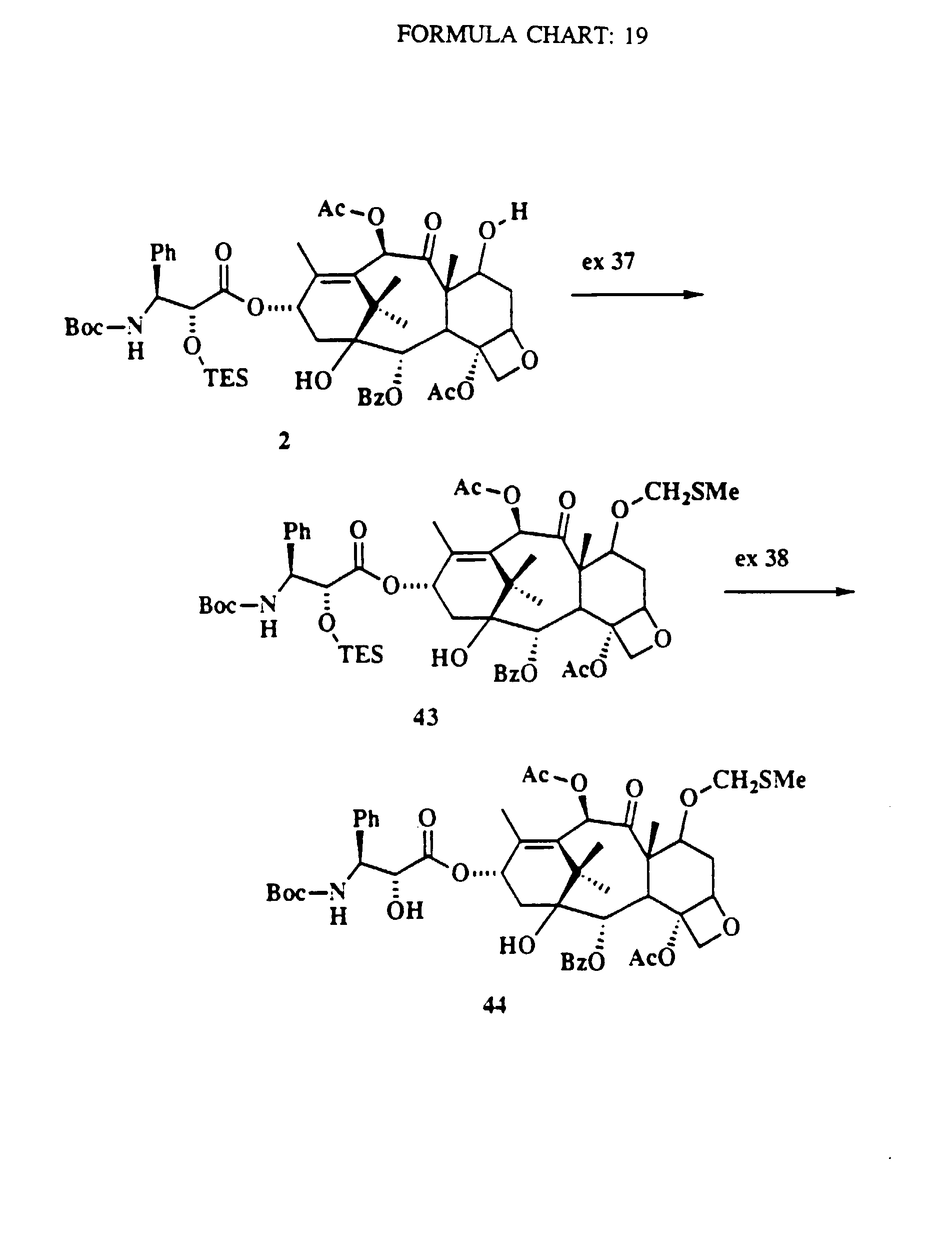

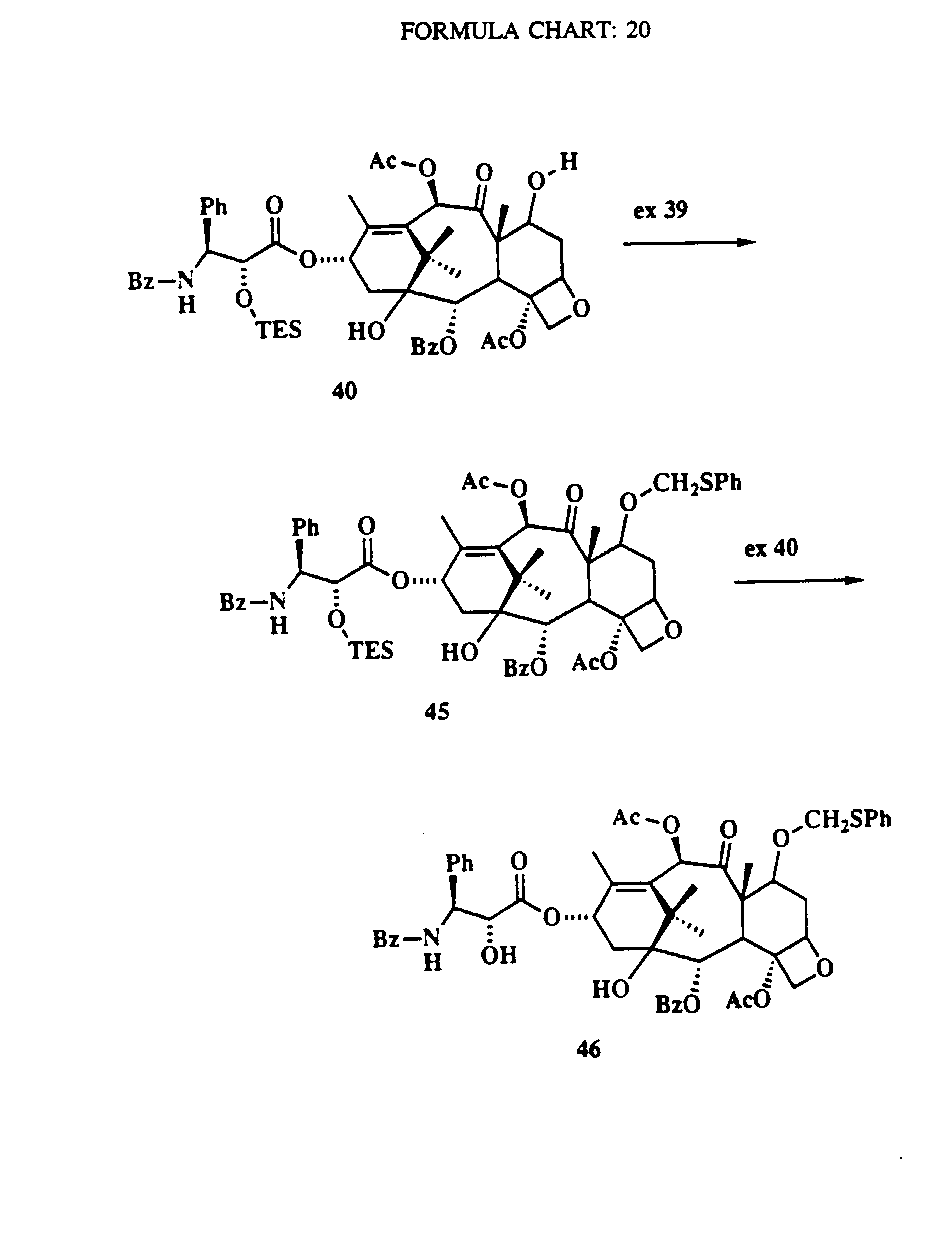

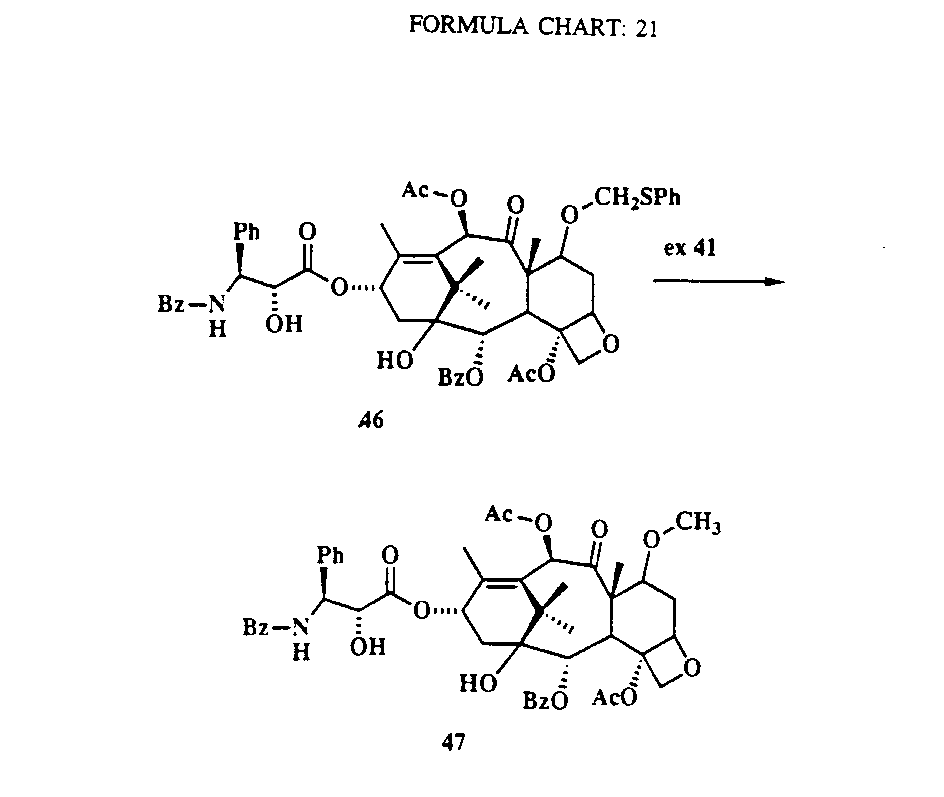

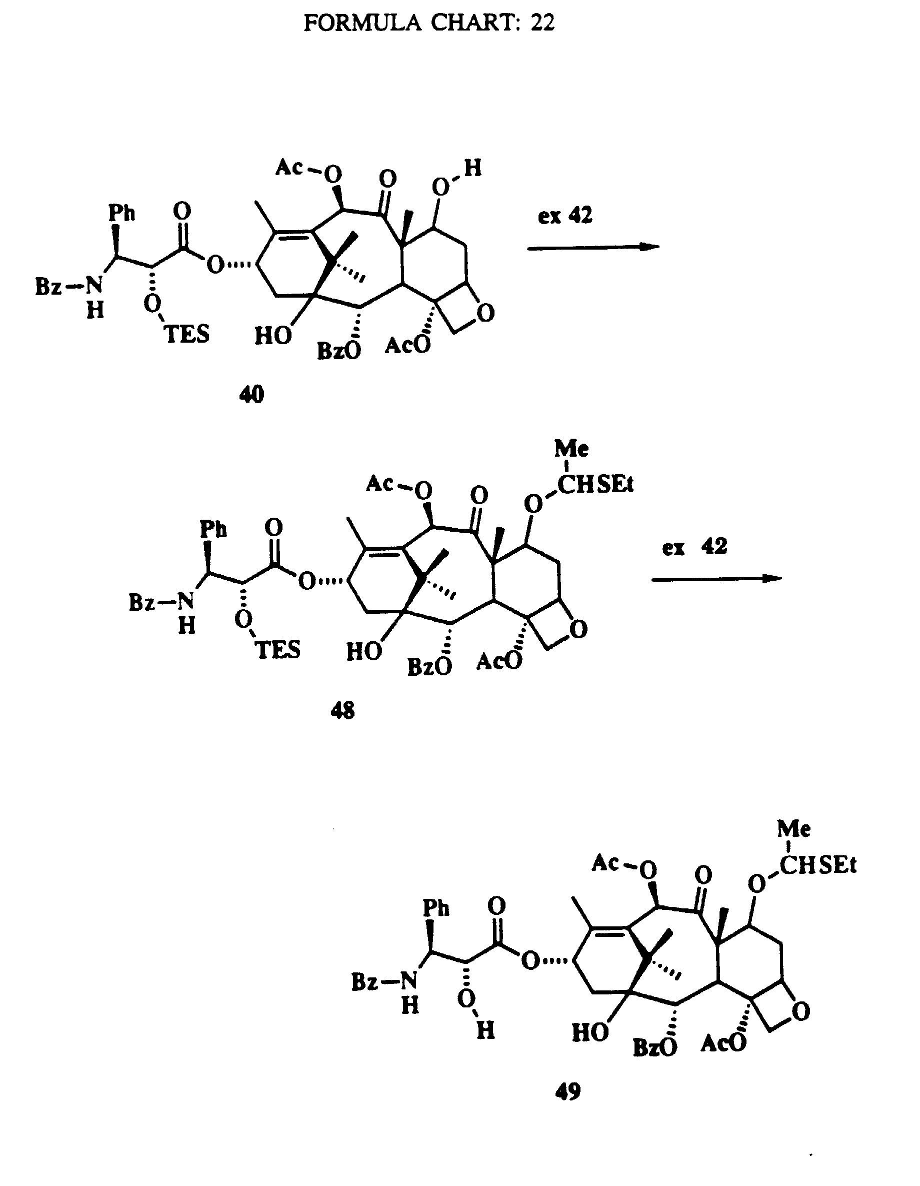

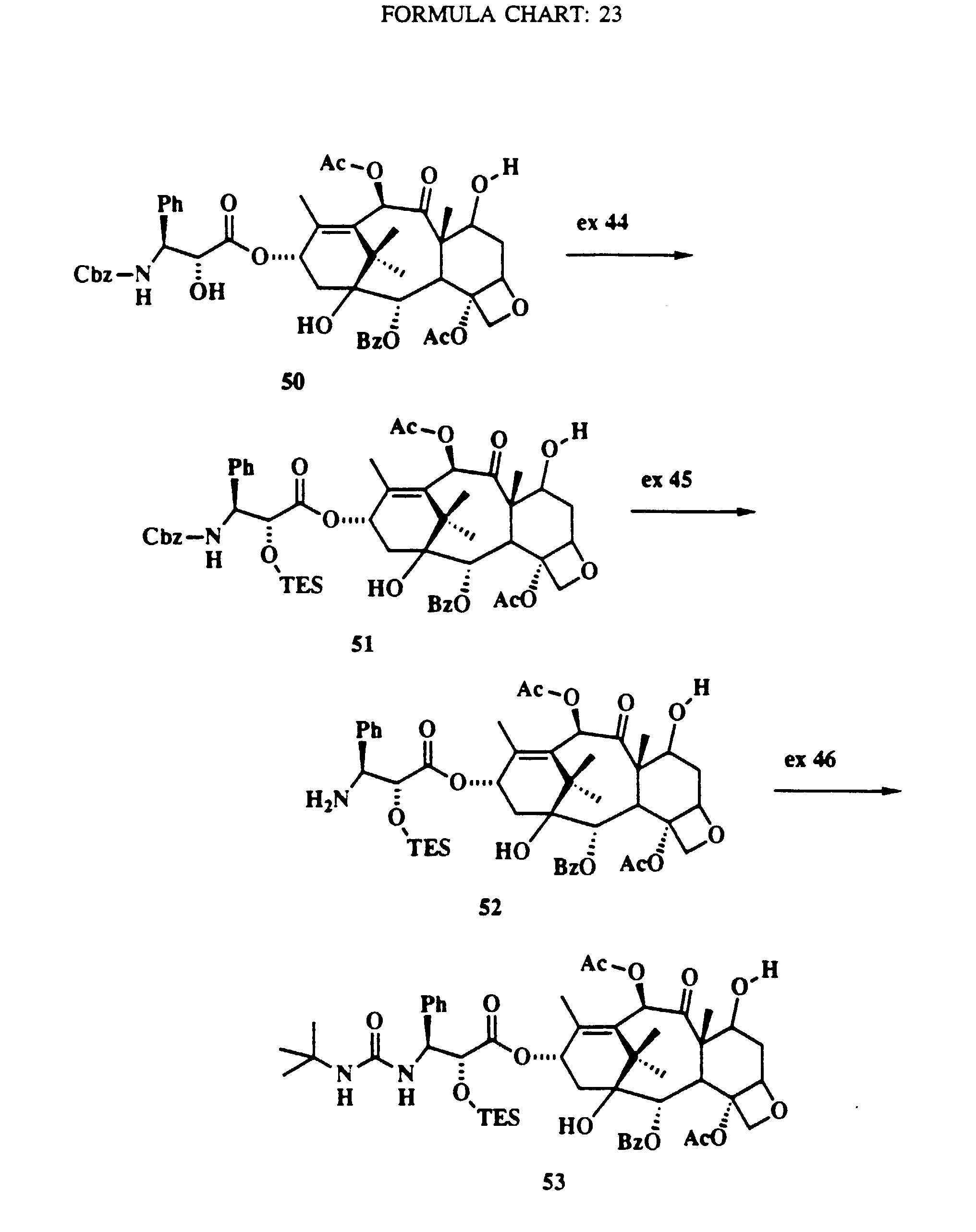

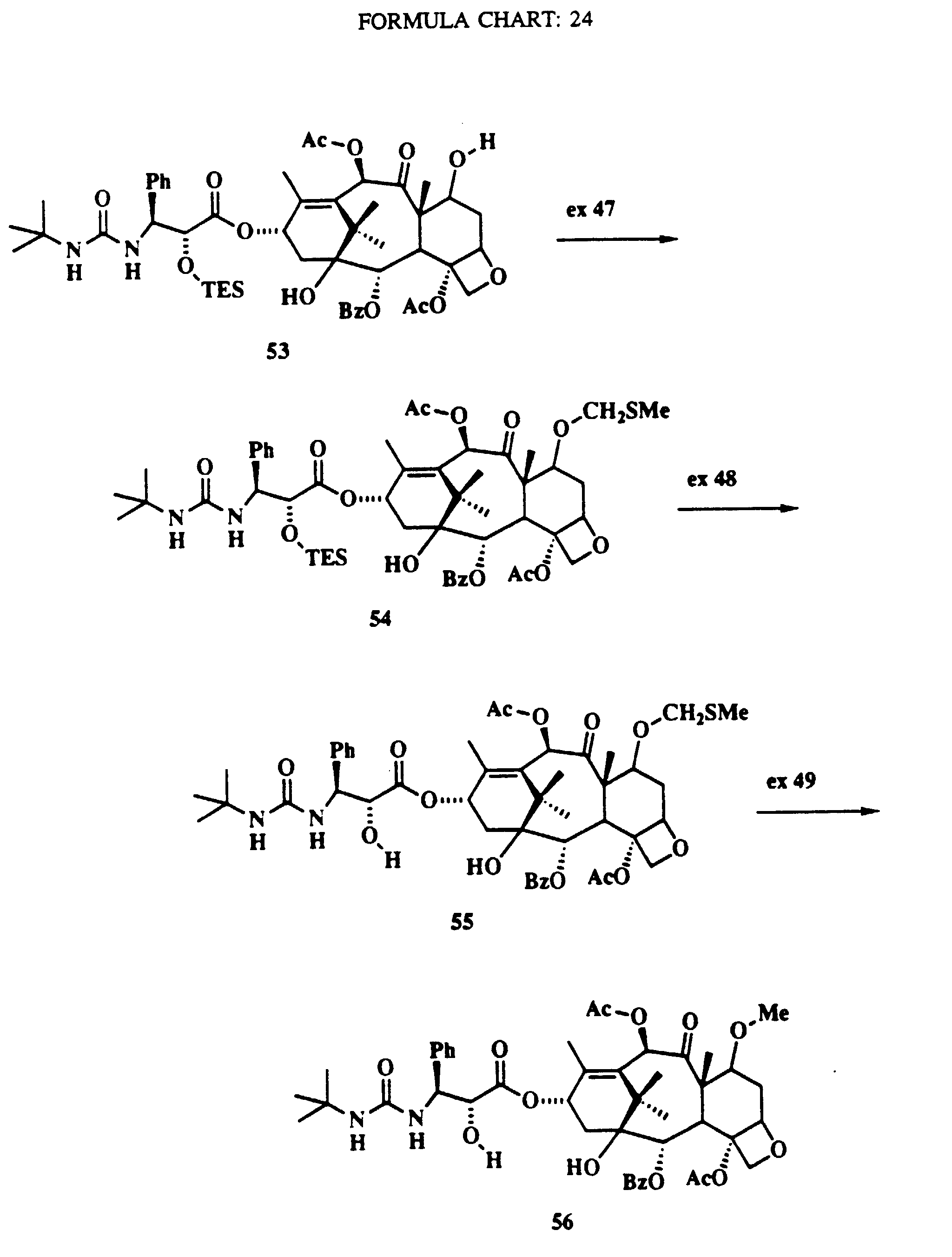

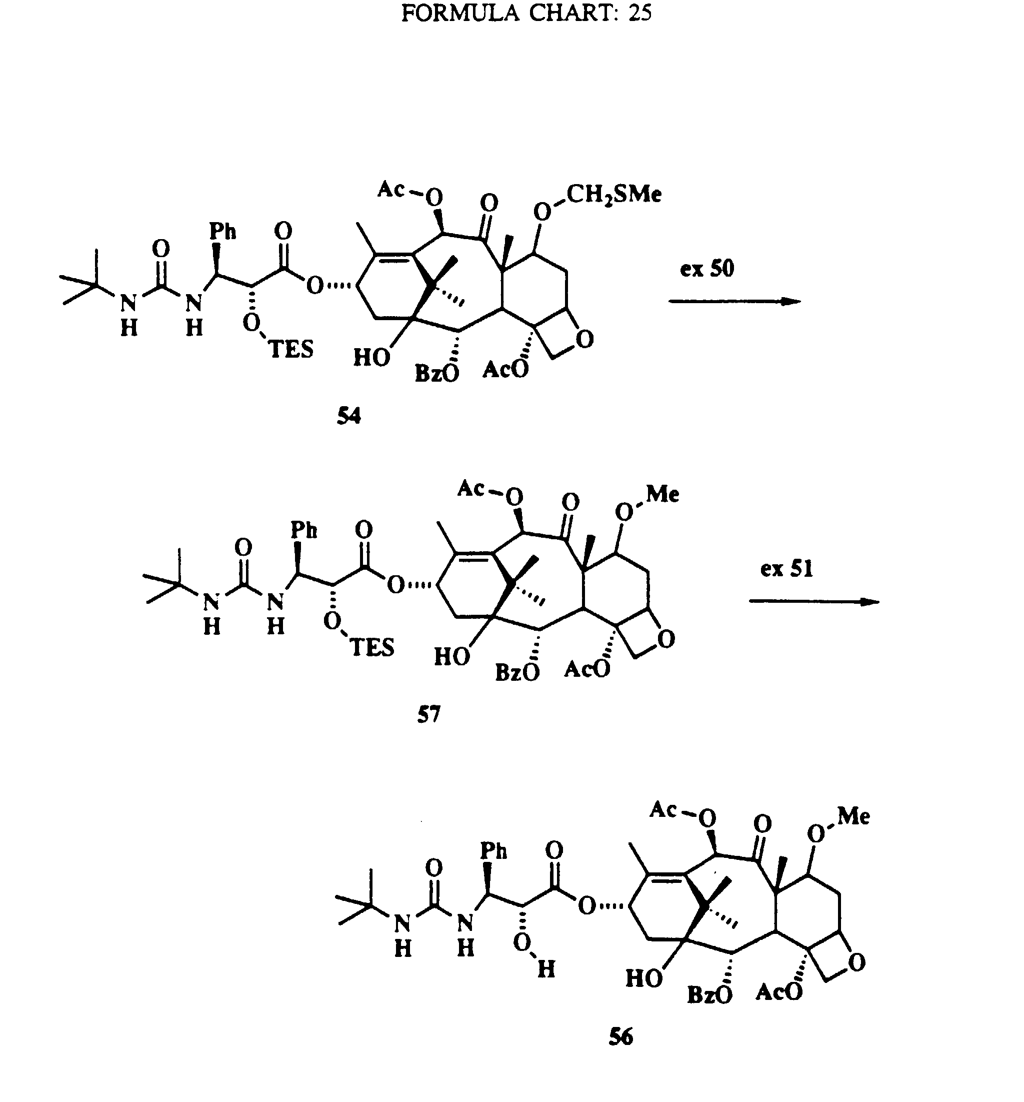

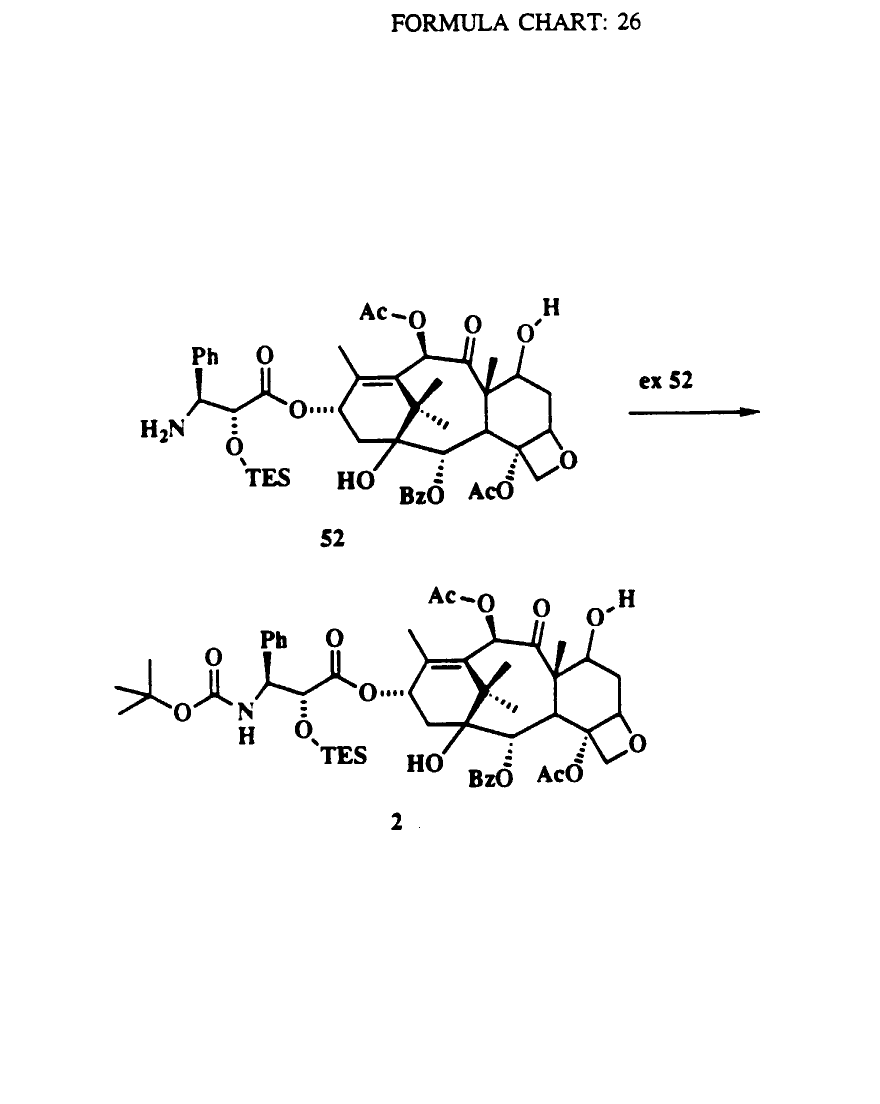

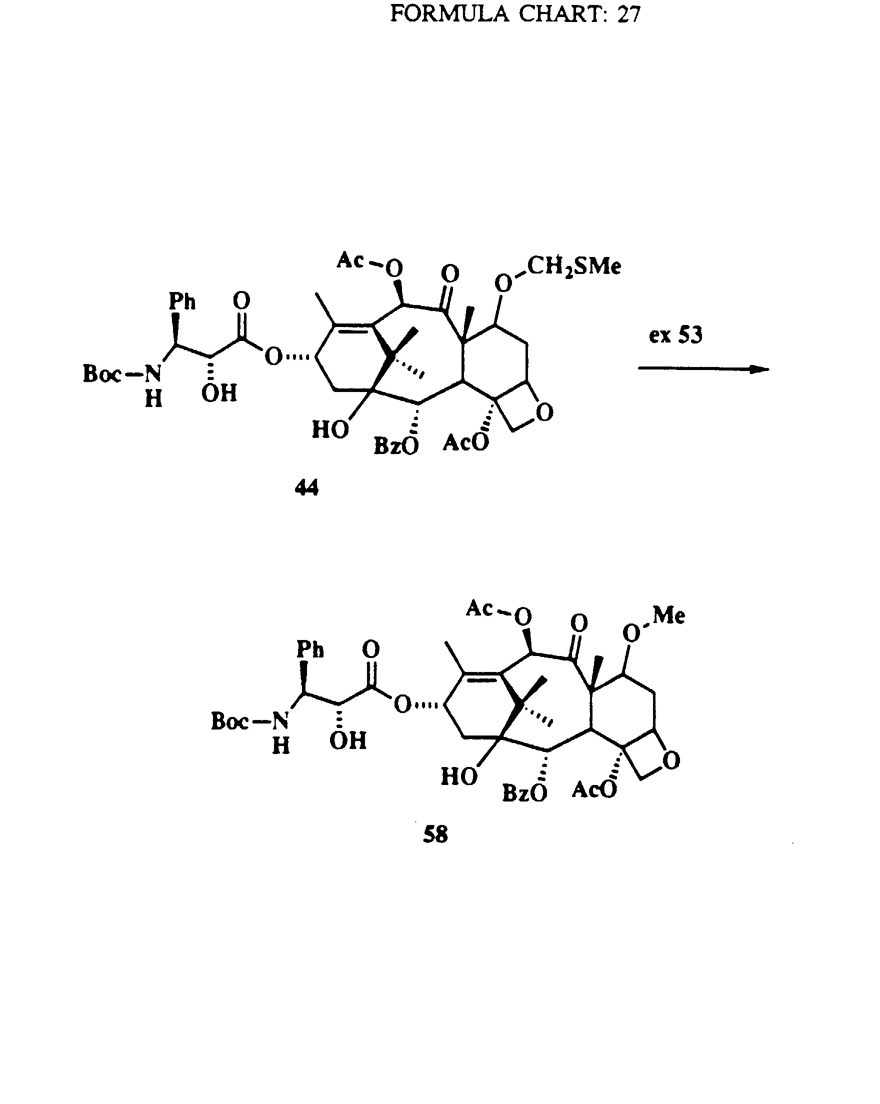

## Claims

1. A compound of the Formula I: 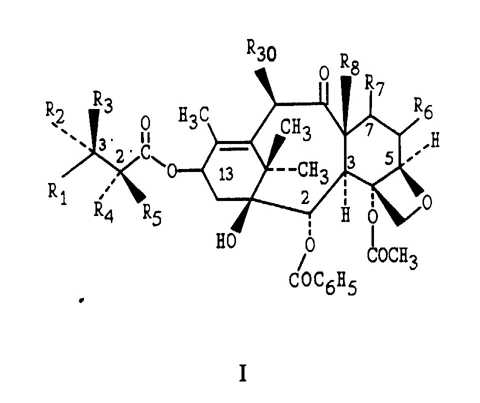 wherein:
R₁ is selected from the group consisting of
-CH₃,
-C₆H₅ or phenyl substituted with one, 2 or 3 C₁-C₄ alkyl, C₁-C₃ alkoxy, halo, C₁ -C₃ alkylthio, trifluoromethyl, C₂-C₆ dialkylamino, hydroxy or nitro,
-2-furyl, 2-thienyl, 1-naphthyl, 2-naphthyl or 3,4-methylenedioxyphenyl;
R₂ is selected from the group consisting of -H, -NHC(O)H,-NHC(O)C₁-C₁₀alkyl, -NHC(O)phenyl, -NHC(O)phenyl substituted with one, 2 or 3 C₁ -C₄ alkyl, C₁-C₃ alkoxy, halo, C₁-C₃ alkylthio, trifluoromethyl, C₂-C₆ dialkylamino, hydroxy or nitro, -NHC(O)C(CH₃)=CHCH₃, -NHC(O)OC(CH₃)₃, -NHC(O)OCH₂phenyl, -NH₂, -NHSO₂-4-methylphenyl, -NHC(O)(CH₂)₃COOH, -NHC(O)-4-(SO₃H)phenyl, -OH, -NHC(O)-1-adamantyl, NHC(O)O-3-tetrahydrofuranyl, -NHC(O)O-4-tetrahydropyranyl, -NHC(O)CH₂C(CH₃)₃, -NHC(O)C(CH₃)₃, -NHC(O)OC₁-C₁₀alkyl, -NHC(O)NHC₁-C₁₀alkyl, -NHC(O)NHPh, -NHC(O)NHPh substituted with one, 2 or 3 C₁-C₄ alkyl, C₁-C₃ alkoxy, halo, C₁-C₃ alkylthio, trifluoromethyl, C₂-C₆ dialkylamino, or nitro, -NHC(O)C₃-C₈cycloalkyl, -NHC(O)OC(CH₂CH₃)₂CH₃, -NHC(O)OC(CH₃)₂CH₂Cl, -NHC(O)OC(CH₃)₂CH₂CH₃, -NHC(O)-1-phenyl-1-cyclopentyl, -NHC(O)-1-methyl-1-cyclohexyl, -NHC(S)NHC(CH₃)₃ or -NHC(O)NHC(CH₃)₃;
R₃ is selected from the group consisting of -H, -NHC(O)phenyl or -NHC(O)OC(CH₃)₃, with the overall proviso that one of R₂ and R₃ is -H but R₂ and R₃ are not both -H;
R₄ is -H or selected from the group consisting of -OH, -OAc (-OC(O)CH₃), -OC(O)OCH₂C(Cl)₃, -OCOCH₂CH₂NH₃⁺ HCOO⁻, -NHC(O)phenyl, -NHC(O)OC(CH₃)₃, -OCOCH₂CH₂COOH and pharmaceutically acceptable salts thereof, -OCO(CH₂)₃COOH and pharmaceutically acceptable salts thereof, and -OC(O)-Z-C(O)-R' [where Z is ethylene (-CH₂CH₂-), propylene (-CH₂CH₂CH₂-), -CH=CH-, 1,2-cyclohexane or 1,2-phenylene, R' is -OH, -OH base, -NR'₂R'₃, -OR'3, -SR'₃, -OCH₂C(O)NR'₄R'₅ where R'₂ is -H or -CH₃, R'₃ is -(CH₂)ₙNR'₆R'₇ or (CH₂)ₙN+R'₆R'₇R'₈ X" where n is 1-3, R'₄ is -H or -C₁-C₄alkyl, R'₅ is -H, -C₁-C₄alkyl, benzyl, hydroxyethyl, -CH₂CO₂H or dimethylaminoethyl, R'₆ and R'₇ are -CH₃, -CH₂CH₃, benzyl or R'₆ and R'₇ together with the nitrogen of NR'₆R'₇ form a pyrrolidino, piperidino, morpholino, or N-methylpiperazino group; R'₈ is -CH₃, -CH₂CH₃ or benzyl , X⁻ is halide, and base is NH₃, (HOC₂H₄)₃N, N(CH₃)_{3,} CH₃N(C₂H₄)₂NH, NH₂(CH₂)₆NH₂, N-methylglucarnine, NaOH or KOH], -OC(O)(CH₂)ₙNR²R³ [where n is 1-3, R² is -H or -C₁-C₃alkyl and R³ -H or -C₁-C₃alkyl], -OC(O)CH(R")NH₂ [where R" is selected from the group consisting of -H, -CH₃, -CH₂CH(CH₃)₂, -CH(CH₃)CH₂CH₃, -CH(CH₃)₂, -CH₂phenyl, -(CH₂)₄NH₂, -CH₂CH₂COOH, -(CH₂)₃NHC(=NH)NH₂], the residue of the amino acid proline, -OC(O)CH=CH₂, -C(O)CH₂CH₂C(O)NHCH₂CH₂SO₃⁻ Y⁺, -OC(O)CH₂CH₂C(O)NHCH₂CH₂CH₂SO₃⁻Y⁺ wherein Y⁺ is Na⁺ or N⁺(Bu)₄, -OC(O)CH₂CH₂C(O)OCH₂ CH₂OH;
R₅ is -H or -OH, with the overall proviso that when R₅ is -OH, R₄ is -H and with the further proviso that when R₅-H, R₄ is other than -H;
R₆ is -H:-H;
R₇ is α-R₉₁:β-R₉₂ where one of R₉₁and R₉₂ is -H and the other of R₉₁ and R₉₂ is -W where W is selected from the group consisting of
-O-C₁-C₁₀alkyl,
-O-C₃-C₁₀ unsaturated alkyl,
-O-C₅-C₁₅ heteroalkyl,
-O-CH(R²¹)OR²² where
R²¹ is -H or -C₁-C₆ alkyl, and
R²² is -C₁-C₁₀alkyl, C₃-C₁₀ unsaturated alkyl or -C₅-C₁₅ heteroalkyl;
or when R²¹ and R²² are taken together to form a ring with 4 to 6 carbon atoms;
-CH(R²⁸)S(O)ₘAr where Ar is phenyl or phenyl substituted with one, 2 or 3 C₁-C₄ alkyl, C₁-C₃ alkoxy, halo, C₁-C₃ alkylthio, trifluoromethyl, C₂-C₆ dialkylamino, or nitro;
or -CH(R²⁸)S(O)ₘCH₂R²⁸
where R²⁸ is
-C₁-C₆ alkyl,
-C₃-C₁₀ unsaturated alkyl,
-(CH₂)_{q}phenyl where q is 0-6,
-(CH₂)_{q}phenyl where q is 0-6 and substituted with one, 2 or 3 C₁-C₄ alkyl, C₁-C₃ alkoxy, halo, C₁-C₃ alkylthio, trifluoromethyl, C₂-C₆ dialkylamino, or nitro,
-naphthyl,
-naphthyl substituted with one, 2 or 3 C₁-C₄ alkyl, C₁-C₃ alkoxy, halo, C₁-C₃ alkylthio, trifluoromethyl, C₂-C₆ dialkylamino, or nitro, -C₅-Cₗ₅ heteroalkyl,
or when R²⁸ and R²⁸ are taken together to form a ring with 4 to 6 carbon atoms;
m is 0 to 2;
R₈ is -CH₃;
R₃₀ is -H, OH, or -OC(O)CH₃; and
pharmaceutically acceptable salts thereof when the compound contains either an acidic or basic functional group.

2. A compound according to Claim 1 wherein R₂ is -NHC(O)C₆H₅, R₄ is hydroxy, R₃ and R₅ are -H, and R₁ is phenyl or substituted phenyl.

3. A compound according to Claim 1 wherein R₂ is -NHC(O)OC(CH₃)₃, R₁ is phenyl or substituted phenyl, R₄ is hydroxy, and R₃ and R₅ are -H.

4. A compound according to Claim 1 wherein R₂ is -NHC(O)NHC(CH₃)₃, R₁ is phenyl or substituted phenyl, R₄ is hydroxy, and R₃ and R₅ are -H.

5. A compound according to Claim 1 wherein W is -O-C₁-C₁₀alkyl, -O-C₃-C₁₀ unsaturated alkyl or -O-C₅-C₁₅ heteroalkyl.

6. A compound according to Claim 1 wherein -W is -O-CH(R²¹)OR²² where
R²¹ is -H or -C₁-C₆ alkyl, and
R²² is -C₁-C₁₀alkyl or -C₃-C₁₀ unsaturated alkyl.

7. A compound according to Claim 1 wherein -W is -O-CH(R²¹)OR²² and R²² is selected from the group consisting of CH₂-(2- or 3-furyl), CH₂(2- or 3-pyrrolyl), CH₂(2-, 3, or 4-pyridyl), CH₂(2-, 4- or 5-imidazoyl) or CH₂(3-, 4- or 5-isoxazolyl).

8. A compound according to Claim 1 wherein -W is -CH(R²⁸)S(O)ₘCH₂R²⁸ and R²⁸ is -C₁-C₆ alkyl, -C₃-C₁₀ unsaturated alkyl or -(CH₂)_{q}phenyl where q is 0-6.

9. A compound according to Claim 1 wherein -W is -CH(R²⁸)S(O)ₘCH₂R²⁸ and R²⁸ is selected from the group consisting of CH₂-(2- or 3-furyl), CH₂(2- or 3-pyrrolyl), CH₂(2-, 3, or 4-pyridyl), CH₂(2-, 4- or 5-imidazoyl) or CH₂(3-, 4- or 5-isoxazolyl).

10. A compound according to Claim 1 wherein -W is -CH(R²⁸)S(O)ₘAr where R²⁸ is -C₁-C₆ alkyl, -C₃-C₁₀ unsaturated alkyl or -(CH₂)_{q}phenyl where q is 0-6.

11. A compound according to Claim 1 wherein R₂ is -NHC(O)C₆H₅, R₄ is hydroxy, R₃ and R₅ are -H, R₁ is phenyl or substituted phenyl, and -W is selected from the group consisting of:
-O-C₁-C₁₀alkyl;
-O-C₃-C₁₀ unsaturated alkyl;
-O-CH(R²¹ )OR²² where
R²¹ is H or C₁-C₆ alkyl, and
R²² is -C₁-C₆alkyl or -C₃-C₁₀ unsaturated alkyl;
-CH(R²⁸)S(O)ₘAr where Ar is phenyl or phenyl substituted with one, 2 or 3
C₁-C₄ alkyl, C₁-C₃ alkoxy, halo, C₁-C₃ alkylthio, trifluoromethyl, C₂-C₆ dialkylamino, or nitro; or
-CH(R²⁸)S(O)ₘCH₂R²⁸
where R²⁸ is
-C₁-C₆ alkyl,
-C₃-C₁₀ unsaturated alkyl, or
-(CH₂)_{q}phenyl where q is 0-3; and
m is 0.

12. A compound according to Claim 1 wherein R₂ is -NHC(O)OC(CH₃)₃, R₁ is phenyl or substituted phenyl, R₄ is hydroxy, R₃ and R₅ are -H, and -W is selected from the group consisting of:
-O-C₁-C₁alkyl; .
-O-C₃-C₁₀ unsaturated alkyl;
-O-CH(R²¹)OR²² where
R²¹ is H or C₁-C₆ alkyl, and
R²² is C₁-C₆alkyl or -C₃-C₁₀ unsaturated alkyl;
-CH(R²⁸)S(O)ₘAr where Ar is phenyl or phenyl substituted with one, 2 or 3 C₁-C₄ alkyl, C₁-C₃ alkoxy, halo, C₁-C₃ alkylthio, trifluoromethyl, C₂-C₆ dialkylamino, or nitro; or
-CH(R²⁸)S(O)ₘCH₂R²⁸
where R²⁸ is
-C₁-C₆ alkyl,
-C₃-C₁₀ unsaturated alkyl, or
-(CH₂)_{q}phenyl where q is 0-3; and
m is 0.

13. A compound according to Claim 1 wherein R₂ is -NHC(O)NHC(CH₃)₃.

14. A compound according to Claim 13 wherein -W is selected from the group consisting of:
O-methyl;
O-propyl;
O-allyl;
O-methoxymethyl;
O-ethoxymethyl;
O-methoxyethoxymethyl;
O-benzyloxymethyl;
O-(2,2,2-trichloroethoxy)methyl;
O-(2,2,2-trichloroethoxy)methoxymethyl;
O-methylthiomethyl; and
O-phenylthiomethyl.

15. A compound according to Claim 13 wherein -W is selected from the group consisting of:
O-methoxymethyl;
O-ethoxymethyl;
O-methoxyethoxymethyl;
O-benzyloxymethyl;
O-(2,2,2-trichloroethoxy)methyl;
O-(2,2,2-trichloroethoxy)methoxymethyl;
O-methylthiomethyl; and
O-phenylthiomethyl.

16. A compound according to Claim 12 wherein -W is selected from the group consisting of:
O-methyl;
O-propyl;
O-allyl;
O-methoxymethyl;
O-ethoxymethyl;
O-methoxyethoxymethyl;
O-benzyloxymethyl;
O-(2,2,2-trichloroethoxy)methyl;
O-(2,2,2-trichloroethoxy)methoxymethyl;
O-methylthiomethyl; and
O-phenylthiomethyl.

17. A compound according to Claim 12 wherein -W is selected from the group consisting of:
O-methoxymethyl;
O-ethoxymethyl;
O-methoxyethoxymethyl;
O-benzyloxymethyl;
O-(2,2,2-trichloroethoxy)methyl;
O-(2,2,2-trichloroethoxy)methoxymethyl;
O-methylthiomethyl; and
O-phenylthiomethyl.

18. A compound according to Claim 1 wherein -W is selected from the group consisting of:
O-methyl;
O-propyl;
O-allyl;
O-methoxymethyl;
O-ethoxymethyl;
O-methoxyethoxymethyl;
O-benzyloxymethyl;
O-(2,2,2-trichloroethoxy)methyl;
O-(2,2,2-trichloroethoxy)methoxymethyl;
O-methylthiomethyl; and
O-phenylthiomethyl.

19. A compound according to Claim 1 wherein -W is selected from the group consisting of:
O-methoxymethyl;
O-ethoxymethyl;
O-methoxyethoxymethyl;
O-benzyloxymethyl;
O-(2,2,2-trichloroethoxy)methyl;
O-(2,2,2-trichloroethoxy)methoxymethyl;
O-methylthiomethyl; and
O-phenylthiomethyl.

20. A compound according to Claim 1 selected from the group consisting of:
7-(O-ethoxymethyl)-13-(N-Boc-β-phenyl isoserinyl)-baccatin III (4),
7-(O-methoxyethoxymethyl)-13-(N-Boc-β-phenyl isoserinyl)-baccatin III (6),
7-(O-methoxymethyl)-13-(N-Boc-2'-β-phenyl isoserinyl)-baccatin III (8),
7-(O-benzyloxymethyl)-13-(N-Boc-β-phenyl isoserinyl)-baccatin III (10),
7-(O-ethoxymethyl)-13-(N-(t-butylaminocarbonyl)-β-phenyl isoserinyl)-baccatin III (14),
7-[O-(2,2,2-trichloroethoxy)methyl]-13-(N-Boc-β-phenyl isoserinyl)-baccatin III (21),
7-[O-(2,2,2-trichloroethoxy)methoxymethyl]-13-(N-Boc-β-phenyl isoserinyl)-baccatin III (22),
7-(O-methoxymethyl)-13-(N-(t-butylaminocarbonyl)-β-phenyl isoserinyl)-baccatin III (27),
7-(O-methylthiomethyl) taxol (42),
7-(O-methylthiomethyl)-13-(N-Boc-β-phenyl isoserinyl)-baccatin III (44),
7-(O-phenylthiomethyl) taxol (46),
7-O-methyl Taxol (47),
7-[O-ethyl(1-thioethyl)] Taxol (49),
13-(N-(t-butylaminocarbonyl)-b-phenyl isoserinyl)-baccatin III 7-O-methylthiomethyl ether (55),
13-(N-(t-butylaminocarbonyl)-b-phenyl isoserinyl)-baccatin III 7-O-methyl ether (56), and
13-(N-Boc-2'-TES-b-phenyl isoserinyl)-baccatin III 7-O-methyl ether (58).

## Patentansprüche

1. Verbindung der Formel I: 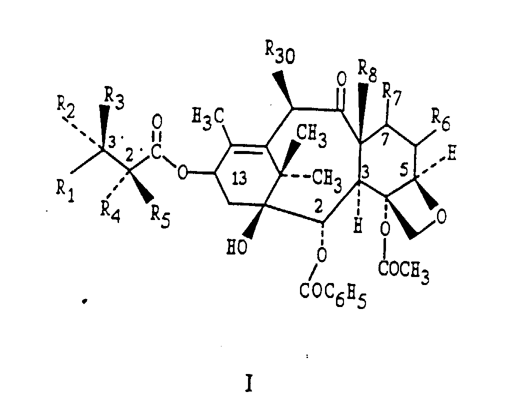 worin bedeuten:
**R**_{**1**} -CH₃, -CH₆H₅ oder ein-, zwei- oder dreifach C₁-C₄-alkyl-, C₁-C₃-alkoxy-, halogen-, C₁-C₃-alkylthio-, trifluormethyl-, C₂-C₆-dialkylamino-, hydroxy- oder nitrosubstituiertes Phenyl, 2-Furyl, 2-Thienyl, 1-Naphthyl, 2-Naphthyl oder 3,4-Methylendioxyphenyl;
**R**_{**2**} -H, -NHC(O)H, -NHC(O)C₁-C₁₀-Alkyl, -NHC(O)-Phenyl, -NHC(O)-Phenyl, ein- zwei- oder dreifach sub stituiert durch C₁-C₄-Alkyl, C₁-C₃-Alkoxy, Halogen, C₁-C₃-Alkylthio, Trifluormethyl, C₂-C₆-Dialkylamino, Hydroxy oder Nitro, NHC(O)C(CH₃)=CHCH₃, NHC(O)OC(CH₃)₃, -NHC(O)OCH₂-Phenyl, -NH₂, -NHSO₂-4-Methylphenyl, -NHC(O)(CH₂)₃COOH, -NHC(O)-4-(SO₃H)-Phenyl, -OH, -NHC(O)-1-Adamantyl, -NHC(O)O-3-Tetrahydrofuranyl, -NHC(O)O-4-Tetrahydropyranyl, -NHC(O)CH₂C(CH₃)₃, -NHC(O)C(CH₃)₃, -NHC(O)OC₁-C₁₀-Alkyl, -NHC(O)NHC₁-C₁₀-Alkyl, -NHC(O)NHPh, -NHC(O)NHPh, ein-, zwei- oder dreifach substituiert durch C₁-C₄-Alkyl, C₁-C₃-Alkoxy, Halogen, C₁-C₃-Alkylthio, Trifluormethyl, C₂-C₆-Dialkylamino oder Nitro, -NHC(O)C₃-C₈-Cycloalkyl, -NHC(O)OC(CH₂CH₃)₂CH₃, -NHC(O)OC(CH₃)₂CH₂Cl, -NHC(O)OC(CH₃)₂CH₂CH₃, -NHC(O)-1-Phenyl-1-cyclo-pentyl, -NHC(O)-1-Methyl-1-cyclohexyl, -NHC(S)NHC(CH₃)₃ oder -NHC(O)NHC(CH₃)₃;
**R**_{**3**} -H, -NHC(O)-Phenyl oder -NHC(O)OC(CH₃)₃, wobei gilt, daß einer der Reste R₂ und R₃ für -H, jedoch nicht beide Reste R₂ und R₃ für -H stehen;
**R**_{**4**} -H oder -OH, -OAc(-OC(O)CH₃), -OC(O)OCH₂C(Cl)₃, -OCOCH₂CH₂NH₃⁺HCOO⁻, -NHC(O)-Phenyl, -NHC(O)OC(CH₃)₃, -OCOCH₂CH₂COOH und deren pharmazeutisch akzeptable Salze, -OCO(CH₂)₃COOH und deren pharmazeutisch akzeptablen Salze und -OC(O)-Z-C(O)-R' [mit Z gleich Ethylen-(-CH₂CH₂-), Propylen-(-CH₂CH₂-CH₂-), -CH=CH-, 1,2-Cyclohexan oder 1,2-Phenylen und R' gleich -OH, -OH-Base, -NR'₂R'₃,-OR'₃, -SR'₃, -OCH₂C(O)NR'₄R'₅, worin R'₂ für -H oder -CH₃ steht und R'₃ = -(CH₂)ₙNR'₆R'₇ oder (CH₂)ₙN+R'₆R'₇R'₈X⁻ mit n = 1-3, R'₄ = -H oder -C₁-C₄-Alkyl, R'₅ = -H, -C₁-C₄-Alkyl, Benzyl, Hydroxyethyl, -CH₂CO₂H oder Dimethylaminoethyl, R'₆ und R'₇ = -CH₃, -CH₂CH₃, Benzyl oder R'₆ und R'₇ zusammen mit dem Stickstoff von NR'₆R'₇ eine Pyrrolidino-, Piperidino-, Morpholino- oder N-Methylpiperazinogruppe; R'₈ = -CH₃, -CH₂CH₃ oder Benzyl, X⁻ = Halogenid und Base = NH₃, (HOC₂H₄)₃N, N(CH₃)₃, CH₃N(C₂H₄)₂NH, NH₂(CH₂)₆NH₂, N-Methyl-glucamin, NaOH oder KOH], -OC(O) (CH₂)ₙNR²R³ [mit n = 1-3, R² = -H oder -C₁-C₃-Alkyl und R³ = -H oder -C₁-C₃ -Alkyl] , -OC(O)CH(R")NH₂ [wobei R" aus der Gruppe -H, -CH₃, -CH₂CH(CH₃)₂, -CH(CH₃)CH₂CH₃, -CH(CH₃)₂, -CH₂-Phenyl, -(CH₂)₄NH₂, -CH₂CH₂COOH, -(CH₂)₃NHC(=NH)NH₂ ausgewählt ist, den Rest der Aminosäure Prolin, -OC(O)CH=CH₂, -C(O)CH₂CH₂C(O) NHCH₂CH₂SO₃⁻Y⁺, -OC(O)CH₂CH₂C(O)NHCH₂CH₂CH₂SO₃⁻Y⁺, worin Y⁺ = Na⁺ oder N⁺ (Bu)₄, -OC(O)CH₂CH₂C(O)OCH₂CH₂OH;
**R**_{**5**} -H oder -OH, wobei gilt, daß im Falle, daß R₅ -OH darstellt, R₄ -H bedeutet und wobei ferner gilt, daß im Falle, daß R₅ für -H steht, R₄ von -H verschieden ist;
**R**_{**6**} -H:-H;
**R**_{**7**} α-R₉₁:β-R₉₂, wobei einer der Reste R₉₁ und R₉₂ für -H und der andere der Reste R₉₁ und R₉₂ für -W stehen, wobei W aus der Gruppe:
-O-C₁-C₁₀-Alkyl,
-O-C₃-C₁₀ ungesättigtes Alkyl,
-O-C₅-C₁₅-Heteroalkyl,
-O-CH(R²¹)OR²² mit
R²¹ gleich -H oder -C₁-C₆-Alkyl und
R²² gleich -C₁-C₁₀-Alkyl, -C₃-C₁₀ ungesättigtes Alkyl oder C₅-C₁₅-Heteroalkyl oder
R²¹ und R²² zusammen gleich einem Ring mit 4 bis 6 Kohlenstoffatomen;
-CH(R²⁸)S(O)ₘAr mit Ar gleich Phenyl oder ein-, zwei- oder dreifach C₁-C₄-alkyl-, C₁-C₃-alkoxy-, halogen-, C₁-C₃-alkylthio-, trifluormethyl-, C₂-C₆-dialkylamino- oder nitrosubstituiertem Phenyl;
oder -CH(R²⁸)S(O)ₘCH₂R²⁸ mit R²⁸ gleich -C₁-C₆-Alkyl, -C₃-C₁₀ ungesättigtem Alkyl, -(CH₂)_{q}-Phenyl mit q = 0-6, -(CH₂)_{q}-Phenyl mit q = 0-6 und ein-, zwei- oder dreifach C₁-C₄-alkyl-, C₁-C₃-alkoxy-, halogen-, C₁-C₃-alkylthio-, trifluormethyl-, C₂-C₆-dialkylamino-oder nitrosubstituiert,
-Naphthyl,
-Naphthyl, ein-, zwei- oder dreifach C₁-C₄ -alkyl-, C₁-C₃-alkoxy-, halogen-, C₁-C₃-alkylthio-, trifluormethyl-, C₂-C₆-dialkylamino- oder nitrosubstituiert, -C₅-C₁₅-Heteroalkyl,
oder beide Reste R²⁸ zusammen gleich einem Ring mit 4 bis 6 Kohlenstoffatomen;
m = 0-2;
**R**_{**8**} -CH₃;
**R**_{**30**} -H, OH oder -OC(O)CH₃; und deren pharmazeutisch akzeptable Salze im Falle, daß die Verbindung entweder eine saure oder basische funktionelle Gruppe aufweist.

2. Verbindung nach Anspruch 1, worin bedeuten:
R₂ -NHC(O)C₆H₅;
R₄ Hydroxy;
R₃ und R₅ -H und
R₁ Phenyl oder substituiertes Phenyl.

3. Verbindung nach Anspruch 1, worin bedeuten:
R₂ -NHC(O)OC(CH₃)₃;
R₁ Phenyl oder substituiertes Phenyl;
R₄ Hydroxy und
R₃ und R₅ -H.

4. Verbindung nach Anspruch 1, worin bedeuten:
R₂ -NHC(O)NHC(CH₃)₃;
R₁ Phenyl oder substituiertes Phenyl;
R₄ Hydroxy und
R₃ und R₅ -H.

5. Verbindung nach Anspruch 1, worin W für -O-C₁-C₁₀-Alkyl, -O-C₃-C₁₀ ungesättigtes Alkyl oder -O-C₅-C₁₅-Heteroalkyl steht.

6. Verbindung nach Anspruch 1, worin -W für -O-CH(R²¹)OR²² mit R²¹ gleich -H oder -C₁-C₆-Alkyl und R²² gleich -C₁-C₁₀-Alkyl -C₃-C₁₀ ungesättigtem Alkyl steht.

7. Verbindung nach Anspruch 1, worin -W für -O-CH (R²¹) OR²² steht und R²² aus der Gruppe CH₂-(2- oder 3-Furyl), CH₂(2- oder 3-Pyrrolyl), CH₂(2-, 3- oder 4-Pyridyl), CH₂(2-, 4- oder 5-Imidazoyl) oder CH₂(3-, 4- oder 5-Isoxazolyl) ausgewählt ist.

8. Verbindung nach Anspruch 1, worin -W für -CH(R²⁸)S(O)ₘCH₂R²⁸ mit R²⁸ gleich -C₁-C₆-Alkyl, -C₃-C₁₀ ungesättigtem Alkyl oder -(CH₂)_{q}-Phenyl, worin q = 0 bis 6, steht.

9. Verbindung nach Anspruch 1, worin -W für -CH(R²⁸)S(O)ₘCH₂R²⁸ steht und R²⁸ aus der Gruppe CH₂-(2- oder 3-Furyl), CH₂(2- oder 3-Pyrrolyl), CH₂(2-, 3-oder 4-Pyridyl), CH₂(2-, 4- oder 5-Imidazoyl) oder CH₂(3-, 4- oder 5-Isoxazolyl) ausgewählt ist.

10. Verbindung nach Anspruch 1, worin -W für -CH(R²⁸)S(O)ₘAr mit R²⁸ gleich -C₁-C₆-Alkyl, -C₃-C₁₀ ungesättigtem Alkyl oder -(CH₂)_{q}-Phenyl, worin q = 0 bis 6, steht.

11. Verbindung nach Anspruch 1, worin bedeuten:
R₂ -NHC(O)C₆H₅;
R₄ Hydroxy;
R₃ und R₅ -H;
R₁ Phenyl oder substituiertes Phenyl und -W
-O-C₁-C₁₀-Alkyl;
-O-C₃-C₁₀ ungesättigtes Alkyl;
-O-CH(R²¹)OR²² mit
R²¹ gleich H oder C₁-C₆-Alkyl und
R²² gleich -C₁-C₆-Alkyl oder -C₃-C₁₀ ungesättigtem Alkyl;
-CH(R²⁸)S(O)ₘAr mit Ar gleich Phenyl oder Phenyl, ein-, zwei- oder dreifach substituiert durch C₁-C₄-Alkyl, C₁-C₃-Alkoxy, Halogen, C₁-C₃-Alkylthio, Trifluormethyl, C₂-C₆-Dialkylamino oder Nitro, oder
-CH(R²⁸)S(O)mCH₂R²⁸
mit R²⁸ gleich -C₁-C₆-Alkyl, -C₃-C₁₀ ungesättigtem
Alkyl oder -(CH₂)_{q}-Phenyl, worin q = 0-3, und
m = 0.

12. Verbindung nach Anspruch 1, worin bedeuten:
R₂ -NHC(O)OC(CH₃)₃;
R₁ Phenyl oder substituiertes Phenyl;
R₄ Hydroxy;
R₃ und R₅ -H und
-W
-O-C₁-C₁₀-Alkyl,
-O-C₃-C₁₀ ungesättigtes Alkyl,
-O-CH(R²¹)OR²² mit R²¹ gleich H oder C₁-C₆-Alkyl und R²² gleich -C₁-C₆-Alkyl oder -C₃-C₁₀ ungesättigtem Alkyl,
-C(R²⁸)S(O)ₘAr mit Ar gleich Phenyl oder Phenyl, ein-, zwei- oder dreifach substituiert durch C₁-C₄-Alkyl, C₁-C₃-Alkoxy, Halogen, C₁-C₃-Alkylthio, Trifluormethyl, C₂-C₆-Dialkylamino oder Nitro, oder
-CH(R²⁸)S(O)ₘCH₂R²⁸ mit R²⁸ gleich -C₁-C₆-Alkyl,
-C₃-C₁₀ ungesättigtem Alkyl oder -(CH₂)_{q}-Phenyl,
worin q = 0-3 und
m = 0.

13. Verbindung nach Anspruch 1, worin R² für -NHC(O)NHC(CH₃)₃ steht.

14. Verbindung nach Anspruch 13, worin -W aus
O-Methyl;
O-Propyl;
O-Allyl;
O-Methoxymethyl;
O-Ethoxymethyl;
O-Methoxyethoxymethyl;
O-Benzyloxymethyl;
O-(2,2,2-Trichlorethoxy)methyl;
O-(2,2,2-Trichlorethoxy)methoxymethyl;
O-Methylthiomethyl und
O-Phenylthiomethyl
ausgewählt ist.

15. Verbindung nach Anspruch 13, worin -W aus
O-Methoxymethyl;
O-Ethoxymethyl;
O-Methoxyethoxymethyl;
O-Benzyloxymethyl;
O-(2,2,2-Trichlorethoxy)methyl;
O-(2,2,2-Trichlorethoxy)methoxymethyl;
O-Methylthiomethyl und
O-Phenylthiomethyl
ausgewählt ist.

16. Verbindung nach Anspruch 12, worin -W aus
O-Methyl;
O-Propyl;
O-Allyl;
O-Methoxymethyl;
O-Ethoxymethyl;
O-Methoxyethoxymethyl;
O-Benzyloxymethyl;
O-(2,2,2-Trichlorethoxy)methyl;
O-(2,2,2-Trichlorethoxy)methoxymethyl;
O-Methylthiomethyl und
O-Phenylthiomethyl
ausgewählt ist.

17. Verbindung nach Anspruch 12, worin -W aus
O-Methoxymethyl;
O-Ethoxymethyl;
O-Methoxyethoxymethyl;
O-Benzyloxymethyl;
O-(2,2,2 -Trichlorethoxy)methyl;
O-(2,2,2-Trichlorethoxy)methoxymethyl;
O-Methylthiomethyl und
O-Phenylthiomethyl
ausgewählt ist.

18. Verbindung nach Anspruch 1, worin -W aus
O-Methyl;
O-Propyl;
O-Allyl;
O-Methoxymethyl;
O-Ethoxymethyl;
O-Methoxyethoxymethyl;
O-Benzyloxymethyl;
O-(2,2,2-Trichlorethoxy)methyl;
O-(2,2,2-Trichlorethoxy)methoxymethyl;
O-Methylthiomethyl und
O-Phenylthiomethyl
ausgewählt ist.

19. Verbindung nach Anspruch 1, worin -W aus
O-Methoxymethyl;
O-Ethoxymethyl;
O-Methoxyethoxymethyl;
O-Benzyloxymethyl;
O-(2,2,2-Trichlorethoxy)methyl;
O-(2,2,2-Trichlorethoxy)methoxymethyl;
O-Methylthiomethyl und
O-Phenylthiomethyl
ausgewählt ist.

20. Verbindung nach Anspruch 1, ausgewählt aus der Gruppe
7-(O-Ethoxymethyl)-13-(N-Boc-β-phenylisoserinyl)-baccatin III (4),
7-(O-Methoxyethoxymethyl)-13-(N-Boc-β-phenyliso-serinyl)-baccatin III (6),
7-(O-Methoxymethyl)-13-(N-Boc-2'-β-phenylisoserinyl)-baccatin III (8),
7-(O-Benzyloxymethyl)-13-(N-Boc-β-phenylisoserinyl)-baccatin III (10),
7-(O-Ethoxymethyl)-13-(N-(tert.-butylaminocarbonyl)-β-phenylisoserinyl)-baccatin III (14),
7-[O-(2,2,2-Trichlorethoxy)methyl]-13-(N-Boc-β-phenyl-isoserinyl)-baccatin III (21),
7-[O-(2,2,2-Trichlorethoxy)methoxymethyl]-13-(N-Boc-β-phenylisoserinyl)-baccatin III (22),
7-(O-Methoxymethyl)-13-(N-(tert.-butylaminocarbonyl)-β-phenylisoserinyl)-baccatin III (27),
7-(O-Methylthiomethyl)taxol (42),
7-(O-Methylthiomethyl)-13-(N-Boc-β-phenylisoserinyl)-baccatin III (44),
7-(O-Phenylthiomethyl)taxol (46),
7-O-Methyltaxol (47),
7-[O-Ethyl(1-thioethyl)]taxol (49),
13-(N-(tert.-Butylaminocarbonyl)-b-phenylisoserinyl)-baccatin III 7-O-methylthiomethylether (55)
13-(N-(tert.-Butylaminocarbonyl)-b-phenylisoserinyl)-baccatin III 7-O-methylether (56) und
13-(N-Boc-2'-TES-b-Phenylisoserinyl)-baccatin III 7-O-methylether (58).

## Revendications

1. Composé de formule I : 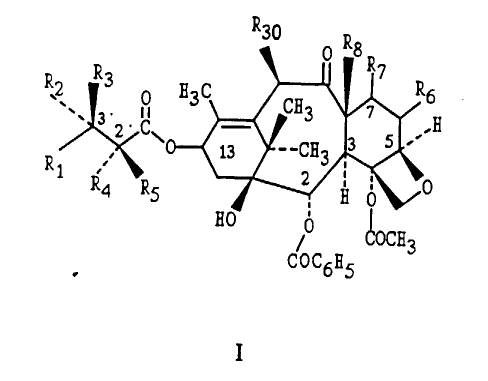 dans laquelle :
R₁ est choisi dans le groupe consistant en
un groupe -CH₃,
un groupe -C₆H₅ ou phényle substitué avec un, 2 ou 3 groupes alkyle en C₁ à C₄, alkoxy en C₁ à C₃, halogéno, alkylthio en C₁ à C₃, trifluorométhyle, dialkylamino en C₂ à C₆, hydroxy ou nitro, -2-furyle, 2-thiényle, 1-naphtyle, 2-naphtyle ou 3,4-méthylènedioxyphényle ;
R₂ est choisi dans le groupe consistant en -H, des groupes -NHC(O)H, -NHC(O)-(alkyle en C₁ à C₁₀), -NHC(O)-phényle, -NHC(O)phényle substitué avec un, 2 ou 3 groupes alkyle en C₁ à C₄, alkoxy en C₁ à C₃, halogéno, alkylthio en C₁ à C₃, trifluorométhyle, dialkylamino en C₂ à C₆, hydroxy ou nitro, -NHC(O)C(CH₃)=CHCH₃, -NHC(O)OC(CH₃)_{3,} -NHC(O)OCH₂-phényle, -NH₂, -NHSO₂-4-méthylphényle, -NHC(O)(CH₂)₃COOH, -NHC(O)-4-(SO₃H)phényle, -OH, -NHC(O)-1-adamantyle, -NHC(O)O-3-tétrahydrofurannyle, -NHC(O)O-4-tétrahydropyrannyle, -NHC(O)CH₂C(CH₃)₃, -NHC(O)C(CH₃)₃, -NHC(O)O-(alkyle en C₁ à C₁₀), -NHC(O)NH-(alkyle en C₁ à C₁₀), -NHC(O)NHPh, -NHC(O)NHPh substitué avec un, 2 ou 3 groupes alkyle en C₁ à C₄, alkoxy en C₁ à C₃, halogéno, alkylthio en C₁ à C₃, trifluorométhyle, dialkylamino en C₂ à C₆ ou nitro, -NHC(O)-(cycloalkyle en C₃ à C₈), -NHC(O)OC(CH₂CH₃)₂CH₃, -NHC(O)OC(CH₃)₂CH₂Cl, -NHC(O)OC(CH₃)₂CH₂CH₃, -NHC(O)-1-phényl-1-cyclopentyle, -NHC(O)-1-méthyl-l-cyclohexyle, -NHC(S)NHC(CH₃)₃ ou -NHC(O)NHC(CH₃)₃ ;
R₃ est choisi dans le groupe consistant en -H, un groupe -NHC(O)phényle et un groupe -NHC(O)OC(CH₃)_{3,} avec la restriction générale qu'un des groupes R₂ et R₃ représente -H mais que R₂ et R₃ ne représentent pas l'un et l'autre -H ;
R₄ représente -H ou est choisi dans le groupe consistant en des groupes -OH, -OAc (-OC(O)CH₃), -OC(O)OCH₂C(Cl)₃, -OCOCH₂CH₂NH₃⁺HCOO⁻, -NHC(O)phényle, -NHC(O)OC(CH₃)₃, -OCOCH₂CH₂COOH et ses sels pharmaceutiquement acceptables, -OCO(CH₂)₃COOH et ses sels pharmaceutiquement acceptables, et -OC(O)-Z-C(O)-R' [dans lequel Z représente un groupe éthylène (-CH₂CH₂-), propylène (-CH₂CH₂CH₂-), -CH=CH-, 1,2-cyclohexane ou 1,2-phénylène, R' représente un groupe -OH, -OH base, -NR'₂R'₃, -OR'₃, -SR^{'}₃, -OCH₂C(O)NR'₄R'₅ dans lesquels R'₂ représente -H ou un groupe -CH₃, R'3 représente un groupe -(CH₂) ₙNR'₆R,₇ ou (CH₂)ₙN⁺R'₆R'₇R'₈X⁻ dans lequel n a une valeur de 1 à 3, R'₄ représente -H ou un groupe alkyle en C₁ à C₄, R'₅ représente -H, un groupe alkyle en C₁ à C₄, benzyle, hydroxyéthyle, -CH₂CO₂H ou diméthylaminoéthyle, R'₆ et R'₇ représentent un groupe -CH₃, -CH₂CH₃, benzyle, ou bien R'₆ et R'₇, conjointement avec l'atome d'azote de NR'₆R'₇, forment un groupe pyrrolidino, pipéridino, morpholino ou N-méthylpipérazino ; R'₈ représente un groupe -CH₃, -CH₂CH₃ ou benzyle, X⁻ représente un halogénure, et la base consiste en NH₃, (HOC₂H₄)₃N, N(CH₃)_{3,} CH₃N(C₂H₄)₂NH, NH₂(CH₂)₆NH_{2,} la N-méthylglucamine, NaOH ou KOH] , -OC(O) (CH₂)ₙNR²R³ [dans lequel n a une valeur de 1 à 3, R² représente -H ou un groupe alkyle en C₁ à C₃ et R³ représente -H ou un groupe alkyle en C₁ à C₃], -OC(O)CH(R")NH₂ [dans lequel R" est choisi dans le groupe consistant en -H, des groupes -CH₃, -CH₂CH(CH₃)₂, -CH(CH₃)CH₂CH₃, -CH(CH₃)₂, -CH₂-phényle, (CH₂)₄NH_{2,} -CH₂CH₂COOH, -(CH₂)₃NHC(=NH)NH₂], le résidu de l'amino-acide consistant en proline, -OC(O)CH=CH₂, -C(O)CH₂CH₂C(O)-NHCH₂CH₂SO₃⁻Y⁺, -OC(O)CH₂CH₂C(O)NHCH₂CH₂SO₃⁻Y⁺ dans lesquels Y⁺ représente Na⁺ ou N⁺(Bu)₄, -OC(O)CH₂CH₂C(O)OCH₂CH₂OH;
R₅ représente -H ou un groupe -OH, avec la restriction générale que, lorsque R₅ représente un groupe -OH, R₄ représente -H, et avec la restriction supplémentaire que, lorsque R₅ représente -H, R₄ ait une autre valeur que -H ;
R₆ représente -H:-H ;
R₇ représente un groupe α-R₉₁:β-R₉₂ dans lequel un des groupes R₉₁ et R₉₂ représente -H et l'autre des groupes R₉₁ et R₉₂ représente -W, le groupe W étant choisi dans le groupe consistant en
un groupe -O-(alkyle en C₁ à C₁₀),
un groupe -O-(alkyle en C₃ à C₁₀) insaturé,
un groupe -O-(hétéroalkyle en C₅ à C₁₅),
un groupe -O-CH(R²¹)OR²² dans lequel
R²¹ représente -H ou un groupe alkyle en C₁ à C₆, et
R²² représente un groupe alkyle en C₁ à C₁₀, alkyle en C₃ à C₁₀ insaturé ou hétéroalkyle en C₅ à C₁₅ ,
ou R²¹ et R²² sont pris conjointement en formant un noyau ayant 4 à 6 atomes de carbone ;
un groupe -CH(R²⁸)S(O)ₘAr dans lequel Ar représente un groupe phényle ou phényle substitué avec un, 2 ou 3 groupes alkyle en C₁ à C₄, alkoxy en C₁ à C₃, halogéno, alkylthio en C₁ à C₃, trifluorométhyle, dialkylamino en C₂ à C₆ ou nitro ;
ou un groupe -CH(R²⁸)S(O)ₘCH₂R²⁸
dans lequel R²⁸ représente
un groupe alkyle en C₁ à C₆,
un groupe alkyle en C₃ à C₁₀ insaturé,
un groupe -(CH₂)_{q}phényle dans lequel q a une valeur de 0 à 6,
un groupe -(CH₂)_{q}phényle dans lequel q a une valeur de 0 à 6 et qui est substitué avec un, 2 ou 3 groupes alkyle en C₁ à C₄, alkoxy en C₁ à C₃, halogéno, alkylthio en C₁ à C₃, trifluorométhyle, dialkylamino en C₂ à C₆ ou nitro,
un groupe naphtyle,
un groupe naphtyle substitué avec un, 2 ou 3 groupes alkyle en C₁ à C₄, alkoxy en C₁ à C₃, halogéno, alkylthio en C₁ à C₃, trifluoro-méthyle, dialkylamino en C₂ à C₆ ou nitro, hétéroalkyle en C₅ à C₁₅,
ou R²⁸ et R²⁸ sont pris conjointement en formant un noyau ayant 4 à 6 atomes de carbone ;
m a une valeur de 0 à 2 ;
R₈ représente un groupe -CH₃ ;
R₃₀ représente -H, un groupe OH ou -OC(O)CH₃ ; et ses sels pharmaceutiquement acceptables lorsque le composé contient un groupe fonctionnel acide ou basique.

2. Composé suivant la revendication 1, dans lequel R₂ représente un groupe -NHC(O)C₆H₅, R₄ représente un groupe hydroxy, R₃ et R₅ représentent -H et R₁ représente un groupe phényle ou phényle substitué.

3. Composé suivant la revendication 1, dans lequel R₂ représente un groupe -NHC(O)OC(CH₃)₃, R₁ représente un groupe phényle ou phényle substitué, R₄ représente un groupe hydroxy et R₃ et R₅ représentent -H.

4. Composé suivant la revendication 1, dans lequel R₂ représente un groupe -NHC(O)NHC(CH₃)₃, R₁ représente un groupe phényle ou phényle substitué, R₄ représente un groupe hydroxy et R₃ et R₅ représentent -H.

5. Composé suivant la revendication 1, dans lequel W représente un groupe -O-(alkyle en C₁ à C₁₀), -O-(alkyle en C₃ à C₁₀) insaturé ou -O-(hétéroalkyle en C₅ à C₁₅).

6. Composé suivant la revendication 1, dans lequel -W représente un groupe -O-CH(R²¹)OR²² dans lequel
R²¹ représente -H ou un groupe alkyle en C₁ à C₆, et
R²² représente un groupe alkyle en C₁ à C₁₀ ou alkyle en C₃ à C₁₀ insaturé.

7. Composé suivant la revendication 1, dans lequel -W représente un groupe -O-CH(R²¹)OR²² et R²² est choisi dans le groupe consistant en des groupes CH₂-(2- ou-3-furyle), CH₂(2- ou 3-pyrrolyle), CH₂(2-, 3- ou 4-pyridyle), CH₂(2-, 4- ou 5-imidazoyle) et CH₂ (3-, 4- ou 5-isoxazolyle).

8. Composé suivant la revendication 1, dans lequel -W représente un groupe -CH(R²⁸)S(O)ₘCH₂R²⁸ et R²⁸ représente un groupe alkyle en C₁ à C₆, alkyle en C₃ à C₁₀ insaturé ou - (CH₂)_{q}phényle dans lequel q a une valeur de 0 à 6.

9. Composé suivant la revendication 1, dans lequel -W représente un groupe -CH(R²⁸)S(O)ₘCH₂R²⁸ et R²⁸ est choisi dans le groupe consistant en des groupes CH₂-(2- ou 3-furyle), CH₂(2- ou 3-pyrrolyle), CH₂(2-, 3- ou 4-pyridyle), CH₂(2-, 4- ou 5-imidazoyle) et CH₂ (3-, 4- ou 5-isoxazolyle).

10. Composé suivant la revendication 1, dans lequel -W représente un groupe -CH(R²⁸)S(O)ₘAr dans lequel R²⁸ représente un groupe alkyle en C₁ à C₆, alkyle en C₃ à C₁₀ insaturé ou -(CH₂)_{q}phényle dans lequel q a une valeur de 0 à 6.

11. Composé suivant la revendication 1, dans lequel R₂ représente un groupe -NHC(O)C₆H₅, R₄ représente un groupe hydroxy, R₃ et R₅ représentent -H, R₁ représente un groupe phényle ou phényle substitué et -W est choisi dans le groupe consistant en ;
un groupe -O-(alkyle en C₁ à C₁₀),
un groupe -O-(alkyle en C₃ à C₁₀) insaturé,
un groupe -O-CH(R²¹)OR²² dans lequel
R²¹ représente -H ou un groupe alkyle en C₁ à C₆, et
R²² représente un groupe alkyle en C₁ à C₆, alkyle en C₃ à C₁₀ insaturé ;
un groupe -CH(R²⁸)S(O)ₘAr dans lequel Ar représente un groupe phényle ou phényle substitué avec un, 2 ou 3 groupes alkyle en C₁ à C₄, alkoxy en C₁ à C₃, halogéno, alkylthio en C₁ à C₃, trifluorométhyle, dialkylamino en C₂ à C₆ ou nitro ; ou
un groupe -CH(R²⁸)S(O) ₘCH₂R²⁸
dans lequel R²⁸ représente
un groupe alkyle en C₁ à C₆,
un groupe alkyle en C₃ à C₁₀ insaturé, ou
un groupe (CH₂)_{q}phényle dans lequel q a une valeur de 0 à 3, et
m est égal à 0.

12. Composé suivant la revendication 1, dans lequel R₂ représente un groupe -NHC(O)OC(CH₃)₃, R₁ représente un groupe phényle ou phényle substitué, R₄ représente un groupe hydroxy, R₃ et R₅ représentent -H, et -W est choisi dans le groupe consistant en :
un groupe -O-(alkyle en C₁ à C₁₀),
un groupe -O-(alkyle en C₃ à C₁₀) insaturé,
un groupe -O-CH(R²¹)OR²² dans lequel
R²¹ représente -H ou un groupe alkyle en C₁ à C₆, et
R²² représente un groupe alkyle en C₁ à C₆ ou alkyle en C₃ à C₁₀ insaturé ;
un groupe -CH(R²⁸)S(O)ₘAr dans lequel Ar représente un groupe phényle ou phényle substitué avec un, 2 ou 3 groupes alkyle en C₁ à C₄, alkoxy en C₁ à C₃, halogéno, alkylthio en C₁ à C₃, trifluorométhyle, dialkylamino en C₂ à C₆ ou nitro ; ou
un groupe -CH(R²⁸)S(O)ₘCH₂R²⁸
dans lequel R²⁸ représente
un groupe alkyle en C₁ à C₆,
un groupe alkyle en C₃ à C₁₀ insaturé, ou
un groupe -(CH₂)_{q}phényle dans lequel q a une valeur de 0 à 3, et
m est égal à 0.

13. Composé suivant la revendication 1, dans lequel R₂ représente un groupe -NHC(O)NHC(CH₃)₃.

14. Composé suivant la revendication 13, dans lequel -W est choisi dasns le groupe consistant en les composés suivants :
O-méthyle ;
O-propyle ;
O-allyle;
O-méthoxyméthyle ;
O-éthoxyméthyle ;
O-méthoxyéthoxyméthyle ;
O-benzyloxyméthyle ;
O-(2,2,2-trichloréthoxy) méthyle ;
O-(2,2,2-trichloréthoxy)méthoxyméthyle ;
O-méthylthiométhyle ; et
O-phénylthiométhyle.

15. Composé suivant la revendication 13, dans lequel -W est choisi dans le groupe consistant en les groupes suivants :
O-méthoxyméthyle ;
O-éthoxyméthyle ;
O-méthoxyéthoxyméthyle ;
O-benzyloxyméthyle ;
O-(2,2,2-trichloréthoxy) méthyle ;
O-(2,2,2-trichloréthoxy)méthoxyméthyle ;
O-méthylthiométhyle ; et
O-phénylthiométhyle.

16. Composé suivant la revendication 12, dans lequel -W est choisi dans le groupe consistant en les groupes suivants :
O-méthyle ;
O-propyle ;
O-allyle ;
O-méthoxyméthyle ;
O-éthoxyméthyle ;
O-méthoxyéthoxyméthyle ;
O-benzyloxyméthyle ;
O-(2,2,2-trichloréthoxy)méthyle ;
O-(2,2,2-trichloréthoxy)méthoxyméthyle ;
O-méthylthiométhyle ; et
O-phénylthiométhyle.

17. Composé suivant la revendication 12, dans lequel -W est choisi dans le groupe consistant en les groupes suivants:
O-méthoxyméthyle ;
O-éthoxyméthyle ;
O-méthoxyéthoxyméthyle ;
O-benzyloxyméthyle ;
O-(2,2,2-trichloréthoxy) méthyle ;
O-(2,2,2-trichloréthoxy)méthoxyméthyle ;
O-méthylthiométhyle ; et
O-phénylthiométhyle.

18. Composé suivant la revendication 1, dans lequel -W est choisi dans le groupe consistant en les groupes suivants :
O-méthyle ;
O-propyle ;
O-allyle;
O-méthoxyméthyle ;
O-éthoxyméthyle ;
O-méthoxyéthoxyméthyle ;
O-benzyloxyméthyle ;
O-(2,2,2-trichloréthoxy) méthyle ;
O-(2,2,2-trichloréthoxy)méthoxyméthyle ;
O-méthylthiométhyle ; et
O-phénylthiométhyle.

19. Composé suivant la revendication 1, dans lequel -W est choisi dans le groupe consistant en les groupes suivants :
O-méthoxyméthyle ;
O-éthoxyméthyle ;
O-méthoxyéthoxyméthyle ;
O-benzyloxyméthyle ;
O-(2,2,2-trichloréthoxy)méthyle ;
O-(2,2,2-trichloréthoxy)méthoxyméthyle ;
O-méthylthiométhyle ; et
O-phénylthiométhyle.

20. Composé suivant la revendication 1, choisi dans le groupe consistant en les composés suivants :
7-(O-éthoxyméthyl)-13-(N-Boc-β-phénylisosérinyl)-baccatine III (4),
7-(O-méthoxyéthoxyméthyl)-13-(N-Boc-β-phényliso-sérinyl)-baccatine III (6),
7-(O-méthoxyméthyl)-13-(N-Boc-2'-β-phényliso-sérinyl)-baccatine III (8),
7-(O-benzyloxyméthyl)-13-(N-Boc-β-phényliso-sérinyl)-baccatine III (10),
7-(O-éthoxyméthyl)-13-(N-(t-butylaminocarbonyl)-β-phénylisosérinyl)-baccatine III (14),
7-[O-(2,2,2-trichloréthoxy)méthyl] -13-(N-Boc-β-phénylisosérinyl)-baccatine III (21),
7-[O-(2,2,2-trichloréthoxy)méthoxyméthyl]-13-(N-Boc-β-phénylisosérinyl)-baccatine III (22),
7-(O-méthoxyméthyl)-13-(N-(t-butylaminocarbonyl)-β-phénylisosérinyl)-baccatine III (27),
7-(O-méthylthiométhyl) taxol (42),
7-(O-méthylthiométhyl)-13-(N-Boc-β-phényliso-sérinyl)-baccatine III (44),
7-(O-phénylthiométhyl)taxol (46) ,
7-O-méthyltaxol (47),
7-[O-éthyl-(1-thioéthyl)]taxol (49),
éther 7-O-méthylthiométhylique de 13-(N-(t-butylaminocarbonyl)-β-phénylisosérinyl)-baccatine III (55),
éther 7-O-méthylique de 13-(N-(t-butylamino-carbonyl)-β-phénylisosérinyl)-baccatine III (56), et
éther 7-O-méthylique de 13-(N-Boc-2'-TES-β-phénylisosérinyl)-baccatine III (58).
